# EUROPEAN PATENT SPECIFICATION

(11) **EP 3 814 494 B1**
(45) Date of publication and mention of the grant of the patent: **01.11.2023**
(21) Application number: 18743691.0
(22) Date of filing: 29.06.2018
(51) Int. Cl.: C12N 15/10

(54) **HIGH THROUGHPUT ASSEMBLY OF NUCLEIC ACID MOLECULES**
HOCHDURCHSATZANORDNUNG VON NUKLEINSÄUREMOLEKÜLEN
ASSEMBLAGE À HAUT DÉBIT DE MOLÉCULES D'ACIDES NUCLÉIQUES

(43) Date of publication of application: 05.05.2021
(73) Proprietor: Thermo Fisher Scientific GENEART GmbH, 93059 Regensburg (DE)
(72) Inventor: LISS, Michael, 93059 Regensburg (DE)
(74) Representative: Ludwig, Christine Andrea
(86) International application number: PCT/EP2018/067575
(87) International publication number: WO 2020/001783

(56) References cited:
- WO-A1-2017/062343
- US-A1- 2006 194 214
- US-A1- 2009 148 849
- US-A1- 2016 060 671
- JIAYUAN QUAN ET AL: "Circular polymerase extension cloning of complex gene libraries and pathways", PLOS ONE, PUBLIC LIBRARY OF SCIENCE, US, vol. 4, no. 7, 30 July 2009 (2009-07-30), pages e6441-1, XP002729117, ISSN: 1932-6203, DOI: 10.1371/JOURNAL.PONE.0006441
- H. YE ET AL: "Experimental analysis of gene assembly with TopDown one-step real-time gene synthesis", NUCLEIC ACIDS RESEARCH, vol. 37, no. 7, 5 March 2009 (2009-03-05), pages e51-e51, XP055038670, ISSN: 0305-1048, DOI: 10.1093/nar/gkp118
- Bryan Sands ET AL: "Overview of Post Cohen-Boyer Methods for Single Segment Cloning and for Multisegment DNA Assembly : Post Cohen-Boyer Methods for Cloning and DNA Assembly" In: "Current Protocols in Molecular Biology", 4 January 2016 (2016-01-04), Wiley, New York, NY [u.a.], XP055420194, ISBN: 978-0-471-14272-0 pages 3.26.1-3.26.20, DOI: 10.1002/0471142727.mb0326s113, the whole document Fig. 3.26.8
- Jurate Bitinaite ET AL: "DNA Cloning and Engineering by Uracil Excision" In: "Current Protocols in Molecular Biology", 1 April 2009 (2009-04-01), John Wiley & Sons, Inc., Hoboken, NJ, USA, XP055170290, ISBN: 978-0-47-114272-0 DOI: 10.1002/0471142727.mb0321s86, the whole document Fig. 3.21.1

## Description

### FIELD OF THE INVENTION

The present disclosure generally relates to compositions and methods for the synthesis of nucleic acid molecules. Provided, as examples, are compositions and methods for high throughput synthesis and assembly of nucleic acid molecules, in many instances, with high sequence fidelity. Disclosed compositions and methods allow, in part, for miniaturization, parallelization, high throughput production, and cost reduction in gene synthesis workflows.

### BACKGROUND OF THE INVENTION

Over the years gene synthesis has become more cost effective and efforts to develop high throughput synthesis platforms and miniaturize certain workflows offers new applications and market opportunities

With progress in genetic engineering, a need for the generation of larger nucleic acid molecules has developed. In many instances, nucleic acid assembly methods start with the synthesis of relatively short nucleic acid molecules (*e.g*., chemically synthesized oligonucleotides), followed by the generation of double-stranded fragments or sub-assemblies (*e.g.,* by annealing and elongating multiple overlapping oligonucleotides), and often proceeds to build larger assemblies such as genes, operons or even functional biological pathways (*e.g.*, by ligation, enzymatic elongation, recombination or a combination thereof). JIAYUAN QUAN ET AL, PLOS ONE, PUBLIC LIBRARY OF SCIENCE, US, vol. 4, no. 7, 30 July 2009, pages e6441, relates to circular polymerase extension cloning of complex gene libraries and pathways. The present disclosure generally relates to compositions and methods for efficient assembly of nucleic acid molecules useful, in part, for (i) miniaturization, (ii) parallelization, (iii) high throughput production and (iv) cost reduction in nucleic acid synthesis.

### SUMMARY OF THE INVENTION

The present invention is defined by the appended claims. The present disclosure relates, in part, to compositions and methods for high throughput synthesis and assembly of nucleic acid molecules. Aspects of the disclosure that may be used in combination or separately include (i) methods and compositions for sorting and pooling of oligonucleotides and (ii) methods and compositions for assembling polynucleotides. The disclosure also includes compositions comprising mixtures of nucleic acid molecules, as well as methods employing such compositions.

The disclosure includes methods for assembling double-stranded target nucleic acids intended to have a predefined sequence. Such methods which do not define the claimed invention, include those a) providing a population of single-stranded oligonucleotides, each of the oligonucleotides having an assembly region representing a portion of the target nucleic acid, wherein each assembly region has a sequence region that is capable of hybridizing to at least one other oligonucleotide in the population, b) providing a linear double-stranded polynucleotide, each terminus of the linear double-stranded polynucleotide having a sequence region that is capable of hybridizing to at least one oligonucleotide of the population, c) combining the population of single-stranded oligonucleotides with the linear double-stranded polynucleotide and a polymerase and dNTPs in a buffer to generate a reaction mixture, and d) subjecting the reaction mixture to cycling conditions that allow for (i) at least partial denaturation of the linear double-stranded polynucleotide to obtain single-stranded termini, (ii) hybridization of the population of single-stranded oligonucleotides to one another and to the single-stranded termini of the polynucleotide in a pre-determined order, (iii) polymerase-mediated extension of the free 3' ends of the hybridized oligonucleotides and the polynucleotide strands to generate a double-stranded non-covalently closed circularized assembly product comprising a linear arrangement of assembly regions, and (iv) amplification of the double-stranded circularized assembly product to generate an amplified double-stranded target nucleic acid intended to have a predefined sequence.

The disclosure further comprises methods wherein the population of single-stranded oligonucleotides comprise: a first set of oligonucleotides, together comprising at least a portion of a first strand of the double-stranded target nucleic acid, and a second set of oligonucleotides together comprising at least a portion of a second strand of the double-stranded target nucleic acid, wherein each oligonucleotide within the first set of oligonucleotides has a sequence region that is capable of hybridizing to at least one other oligonucleotide in the second set of oligonucleotides.

In some instances, each oligonucleotide within the first set of oligonucleotides may be at least partially complementary to at least one oligonucleotide in the second set of oligonucleotides. Further, the first and second sets of oligonucleotides may be designed to hybridize with intervening nicks between the oligonucleotides. Also, the first and second sets of oligonucleotides may be designed to hybridize with one or more nucleotide gaps between the oligonucleotides. Additionally, the first/or second set of oligonucleotides may be designed to hybridize with intervening nicks between the oligonucleotides, whereas the other set of oligonucleotides is designed to hybridize with one or more nucleotide gaps between the oligonucleotides.

The disclosure includes methods wherein the oligonucleotides of the first set are between about 36 to about 120 nucleotides in length, and wherein the oligonucleotides of the second set are between about 36 and about 44 nucleotides in length, or vice versa. Further, the complementary sequence regions of the oligonucleotides hybridizing to at least one terminus of the linear polynucleotide is between about 20 and about 45 nucleotides in length.

Additionally, the plurality of single-stranded oligonucleotides may be obtained by chemical synthesis, and, optionally, wherein the chemical synthesis comprises a photochemical or electrochemical deprotection step and/or by microarray- or chip-based synthesis. The plurality of single-stranded oligonucleotides may be synthesized on beads in micro-cavities. Further, the plurality of single-stranded oligonucleotides may be obtained by using a method for selective retrieval of one or more nucleic acid molecules having a desired length as set out herein.

Further, methods may additionally comprise: subjecting the assembled double-stranded target nucleic acid to one or more error correction and/or error filtration steps and/or transforming a host cell with the assembled circularized molecule.

Further, any amplification step may be performed with a polymerase with proof-reading activity. Exemplary polymerases that may be used in methods of the disclosure include one or more polymerase selected from the group consisting of Pfu DNA polymerase, DEEP VENT^{®} DNA polymerase, Q5^{®} High-Fidelity DNA Polymerase, PHUSION^{®}, PHUSION^{®} HotStartFlex, PRIMESTAR^{®} HS, PRIMESTAR^{®} GXL, PRIMESTAR^{®} Max, ACCUPRIME^{™} Pfx, PLATINUM^{™} DNA Polymerase High Fidelity, PHUSION^{®} Flash II DNA Polymerase, PHUSION^{®} Hot Start II High-Fidelity DNA Polymerase, ACCURA^{®} High-Fidelity polymerase or IPROOF^{™} High-Fidelity polymerase.

Methods of the disclosure include those wherein the linear double-stranded polynucleotide (*e.g.*, linearized by restriction enzyme cleavage, linearized by PCR amplification using a closed circular polynucleotide as template, etc.) has a size of between about 0.5 kb and about 5 kb or between about 10 kb and about 30 kb. Further, the linear double-stranded polynucleotide used in methods disclosed herein may be a vector. Such vector may be a high copy number vector. The linear double-stranded polynucleotide or vector may also comprise one or more resistance and/or selectable marker.

The disclosure further includes kits, which are not part of the invention, such as kits for assembling a double-stranded target nucleic acid intended to have a predefined sequence wherein, for example, the kits comprise: a) a population of single-stranded oligonucleotides each of the oligonucleotides having an assembly region representing a portion of the target nucleic acid, wherein each assembly region has a sequence region that is capable of hybridizing to at least one other oligonucleotide in the population, b) a linear double-stranded polynucleotide, each terminus of the linear double-stranded polynucleotide having a sequence region that is capable of hybridizing to at least one oligonucleotide of the population, c) a polymerase, dNTPs and a buffer system, and d) optionally, competent host cells for transformation. The disclosure also includes kits, wherein for example, the kits comprise: a) a population of single-stranded oligonucleotides each of the oligonucleotides having an assembly region representing a portion of the target nucleic acid, wherein each assembly region has a sequence region that is capable of hybridizing to at least one other oligonucleotide in the population, b) a circular double-stranded polynucleotide having a first and a second sequence region capable of hybridizing to at least one oligonucleotide of the population, a polymerase, dNTPs and a buffer system, and d) optionally, competent host cells for transformation. Kits of the disclosure may further comprise at least one restriction enzyme. Such restriction enzyme may be a type IIS restriction enzyme. The invention also relates to the use of such kits in the methods of the invention defined by the appended claims.

The disclosure includes, but not as part of the invention, in part, populations of oligonucleotides. In some instances, wherein the individual oligonucleotides that make up the population comprise: (a) a tag region, (b) a spacer (*e.g.,* a spacer comprising a hexaethylene glycol group), and (c) an assembly region. In some instances, the assembly region may be capable of hybridizing to at least one other oligonucleotide in the population. In additional instances, the spacer may be located between the tag region and the region capable of hybridizing to at least one other oligonucleotide in the population. Further, the spacer may be cleavable or capable of terminating enzymatic mediated nucleic acid replication reactions. In some instances, the tag region may be represented by a plurality of tags having different nucleotide sequences. Further, subsets of the individual oligonucleotides may comprise assembly regions capable of forming hybridization complexes with each other. The population of oligonucleotides may be composed of individual oligonucleotides comprising assembly regions capable of forming hybridization complexes (*e.g*., at least twenty different hybridization complexes) with each other have tag regions comprising the same nucleotide sequence.

The disclosure also includes compositions but not as part of the invention, comprising such populations of oligonucleotides. For example, the disclosure includes compositions comprising a first population of single-stranded oligonucleotides, each of the oligonucleotides of the first population having an assembly region representing a portion of a first target nucleic acid, wherein each assembly region has a sequence region that is capable of hybridizing to at least one other oligonucleotide in the first population, wherein each oligonucleotide of the first population comprises a tag and a spacer, wherein the tag is located at the 5'-end of the assembly region of each oligonucleotide and the spacer is located between the tag and the assembly region, and wherein the spacer is either cleavable or capable of terminating enzymatic mediated nucleic acid replication reactions. Each oligonucleotide of the first population of oligonucleotides may comprise a tag with an identical nucleotide sequence. Compositions of the disclosure may further comprise a second population of single-stranded oligonucleotides, each of the oligonucleotides of the second population having an assembly region representing a portion of a second target nucleic acid, wherein each assembly region has a sequence region that is capable of hybridizing to at least one other oligonucleotide in the second population, wherein each oligonucleotide of the second population further comprises a tag and a spacer, wherein the tag is located at the 5'-end of the assembly region of each oligonucleotide and the spacer is located between the tag and the assembly region, and wherein the spacer is either cleavable or capable of terminating enzymatic mediated nucleic acid replication reactions, and wherein each oligonucleotide of the second population of oligonucleotides comprises a tag with an identical nucleotide sequence that is different from the tag nucleotide sequence of the oligonucleotides of the first population. Compositions of the disclosure may further comprise a linear double-stranded polynucleotide, each terminus of the linear double-stranded polynucleotide having a sequence region that is capable of hybridizing to at least one oligonucleotide of the first population and/or the second population. Exemplary tags used in compositions of the disclosure may be between about 12 and about 24 nt in length. Further, assembly regions of oligonucleotides used in compositions of the disclosure may be between about 15 and about 60 nucleotides in length. In examples, spacers used in compositions of the disclosure may comprise a hexaethylene glycol group.

In addition, the disclosure includes methods for generating sorted populations of oligonucleotides. Such methods not as part of the claimed invention, include those comprising: a) producing a mixed population of oligonucleotides that differ in nucleotide sequence, wherein oligonucleotides of the mixed population comprise (i) a tag, (ii) a spacer (*e.g.,* a spacer comprising a hexaethylene glycol group), and (iii) an assembly region, b) contacting the mixed population of oligonucleotides with a capture oligonucleotide bound to a solid support, under conditions that allow for hybridization between the capture oligonucleotide and one of the tag regions, wherein the capture oligonucleotide has sequence complementary to one of the tag regions in the mixed population of oligonucleotides and wherein two or more oligonucleotides comprising assembly regions that differ in nucleotide sequence have the tag regions with the same nucleotide sequence, c) removal of members of the mixed population of oligonucleotides that do not hybridize to the solid support to generate the sorted population of oligonucleotides.

Methods for generating sorted populations of oligonucleotides may further comprise d) releasing the sorted oligonucleotides from the solid support. In examples, the release of at least a portion of the sorted population of oligonucleotides may be achieved by a heat denaturation step. In related examples, the solid support may be a bead. Further, the bead may contain capture oligonucleotides intended to all having the same nucleotide sequence. Methods of the disclosure may further comprise assembly of the sorted population of oligonucleotides (*e.g.,* mediated by one or more polymerase chain reactions) into one or more larger nucleic acid molecules. In some aspects, the tags of the sorted population of oligonucleotides may not be incorporated into the one or more larger nucleic acid.

The disclosure also includes not as part of the claimed invention, compositions and methods for obtaining or selecting error-free or sequence-verified nucleic acid molecules and assembling two or more sequence-verified nucleic acid molecules. Further, mixtures of nucleic acid molecules, as set out above and elsewhere herein, may be obtained by synthesizing (*e.g.,* on one or more solid support, such on beads within wells of a microchip) the nucleic acid molecules and/or pooling the nucleic acid molecules and assembling the pooled nucleic acid molecules in one or more optionally multiplexed assembly reactions.

### BRIEF DESCRIPTION OF THE DRAWINGS

The novel features of the invention are set forth in the appended claims. A better understanding of the features and advantages of the present disclosure will be obtained by reference to the following detailed description that sets forth illustrative examples, in which the principles of the disclosure are utilized, and the accompanying drawings of which:

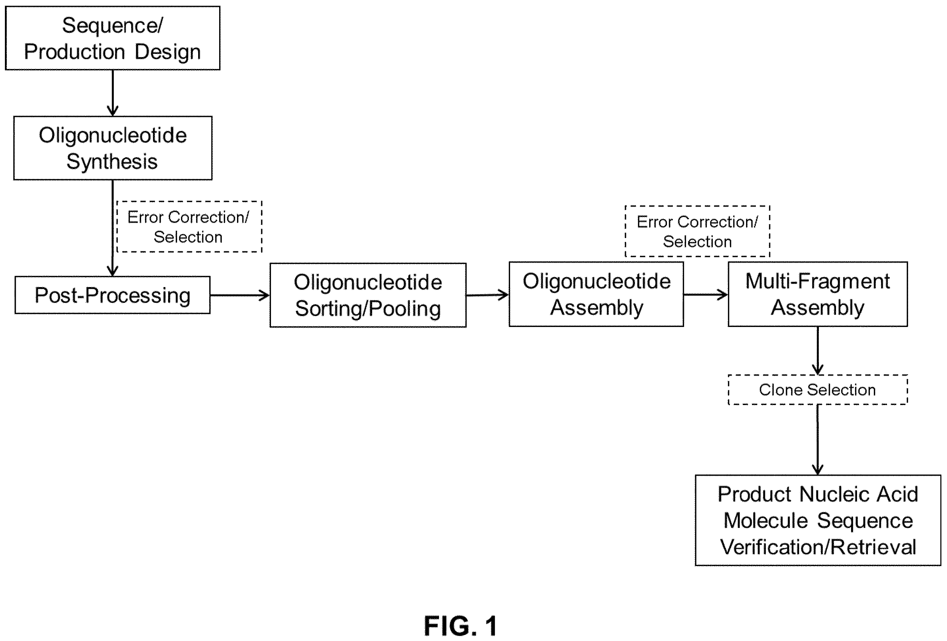

**FIG. 1** is a schematic of a workflow of various aspects of the disclosure. In this workflow, once a desired nucleic acid molecule is contemplated, a design path is set up for the production of this nucleic acid molecule. The first step will typically comprise the design of the sequence and how it is to be generated. Error correction and/or selection of oligonucleotides and nucleic acid fragments or subfragments may occur at one or more points along the workflow.

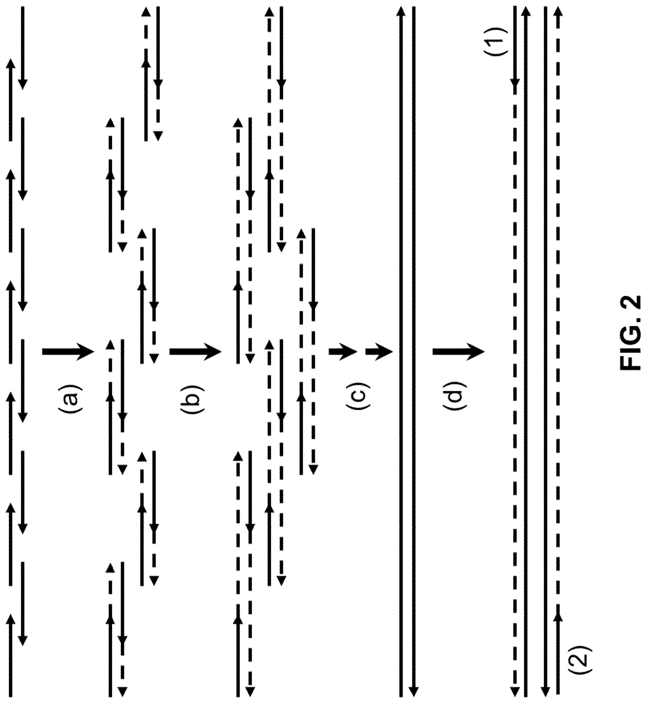

**FIG. 2** is a schematic of a PCR-based process for assembling nucleic acid molecules. (a) Overlapping forward and reverse oligonucleotides are extended in the first cycle round. (b) Extended assembly products anneal to each other and are further extended in the second cycle round. (c) Further extensions take place in subsequent rounds and full-length product accumulates. Two terminal oligonucleotides (1) and (2) can also be universal and applied in excess within the PCR mix to allow a controlled amplification of full-length molecules or can be provided by vector ends.

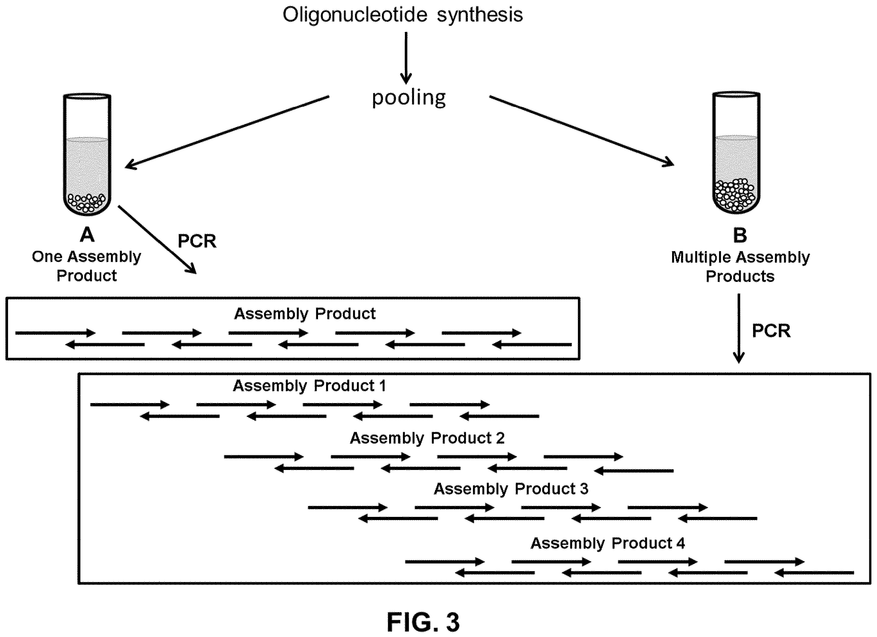

**FIG. 3** is a schematic representation of multiplex nucleic acid assembly workflows of the disclosure. (A) shows beads with oligonucleotides in a vessel, where oligonucleotides are released into solution and are then used for assembly of a single nucleic acid molecule. (B) shows beads in a vessel, where oligonucleotides from these beads are components of more than one nucleic acid molecule. These oligonucleotides are presented directly under (B)..

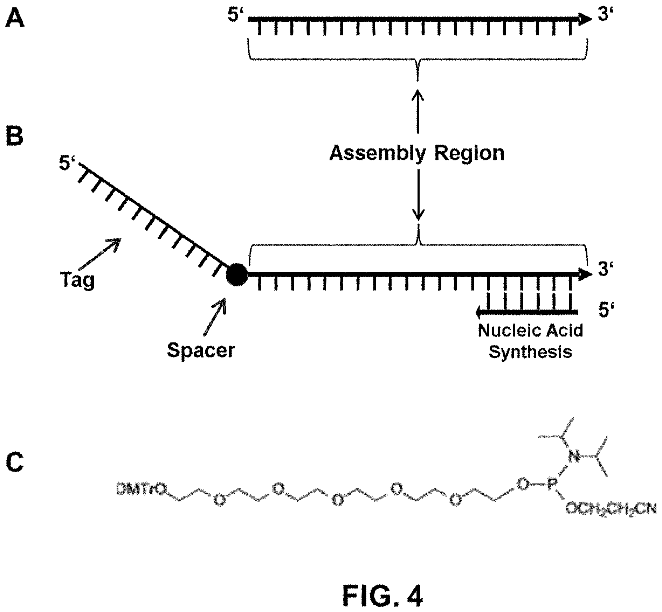

**FIG. 4** shows two oligonucleotides, labeled A and B. The oligonucleotide labeled A is a standard oligonucleotide used in assembly reactions to generate larger nucleic acid molecules. The oligonucleotide labeled B is similar in some respects to the oligonucleotide labeled A, but contains a tag region separated from the rest of the oligonucleotide by a spacer (represented by a solid circle). A primer may hybridize to the 3' end of the oligonucleotide to allow for PCR-mediated elongation. One exemplary spacer is labeled C. This molecule is hexaethylene glycol (HEG).

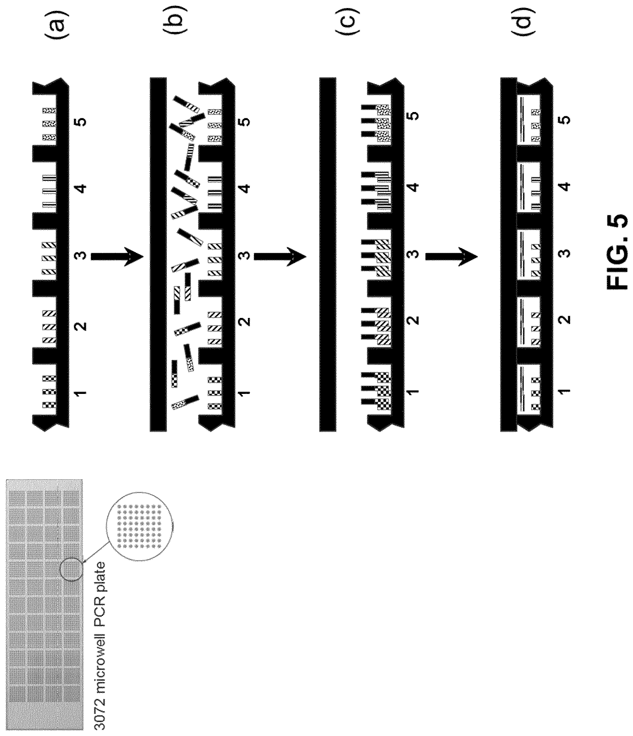

**FIG. 5** shows compositions and methods for the capture of oligonucleotides having particular tags. (a) A multiwell plate suitable for PCR is coated with individual "capture" oligonucleotides (represented by the short vertical lines in the bottom of each well numbered 1 through 5) that differ in each well. (b) The coated multiwell plate is incubated with complete eluate from a chip-based synthesis of tagged oligonucleotides. (c) Under regular hybridization conditions, tagged oligonucleotides belonging together anneal to their counterpart capture oligonucleotides, thus, are grouped and concentrated in their respective PCR well. (d) The multiwell plate with grouped oligonucleotides is washed, provided with assembly PCR or amplification PCR or Sequence Elongation and Ligation reaction mix and cycled accordingly. Each well then encloses a distinct linear or circular full-length nucleic acid product for further cloning or transformation.

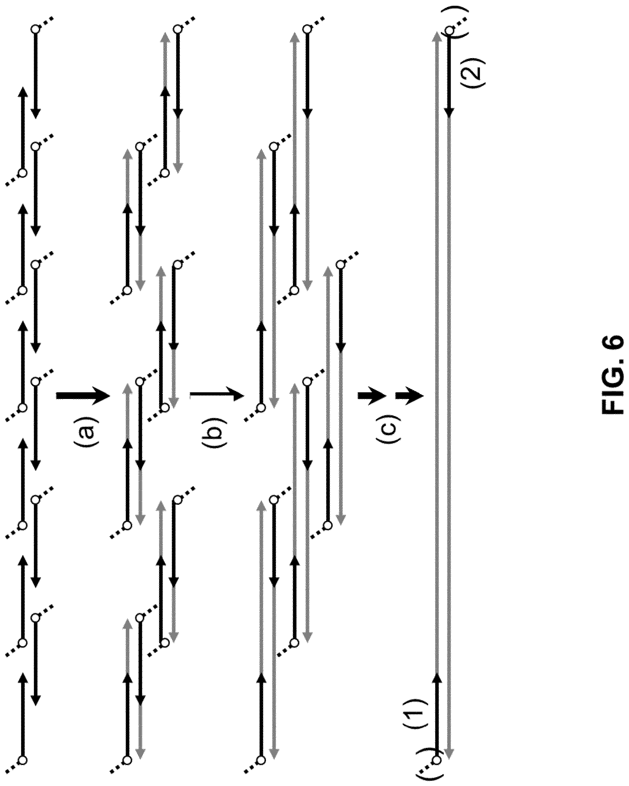

**FIG. 6** describes methods of the disclosure related to the assembly of tagged oligonucleotides. (a) overlapping forward and reverse oligonucleotides carrying uniform hybridization tags (indicated by dotted lines) separated by a spacer (indicated by a circle, *e.g.,* a HEG group) are extended in the first cycle round. (b) Extension has stopped at the spacer and extended molecules anneal to each other and are further extended in the second cycle round. (c) Further extensions take place in subsequent rounds and full-length product accumulates, thereby excluding tag incorporation. The terminal oligonucleotides (1) and (2) can also be universal, without tags, and applied in excess within the PCR mix to allow a controlled amplification of full-length molecules or can be provided by vector ends if Sequence Elongation and Ligation protocol is applied according to methods of the disclosure.

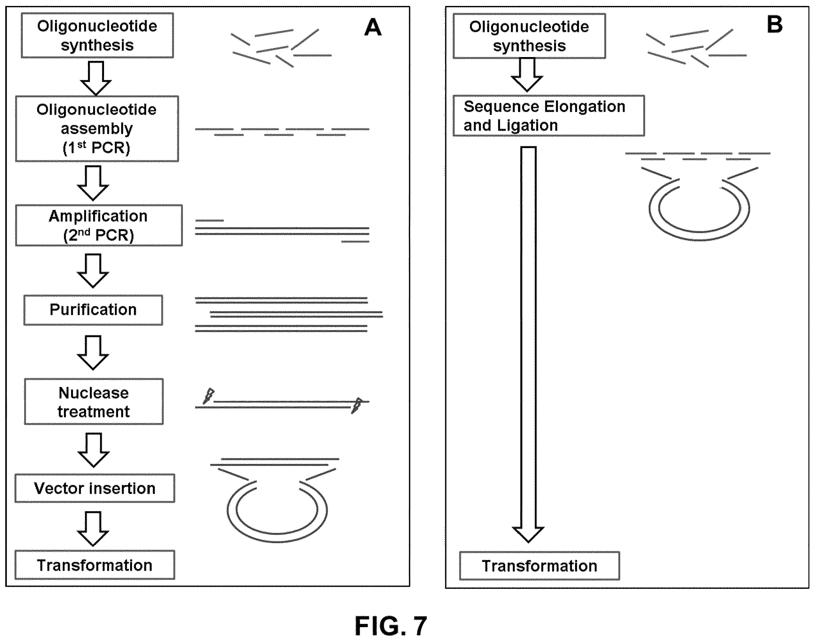

**FIG. 7** shows a side-by-side comparison of two assembly workflows. (A) is a schematic of a standard workflow for assembling a polynucleotide from single-stranded overlapping oligonucleotides comprising the steps of oligonucleotide synthesis, oligonucleotide assembly, amplification of the assembly product, purification of amplified product, nuclease treatment to generate complementary overhangs, vector insertion and transformation. (B) is a schematic of a Sequence Elongation and Ligation reaction according to methods of the disclosure.

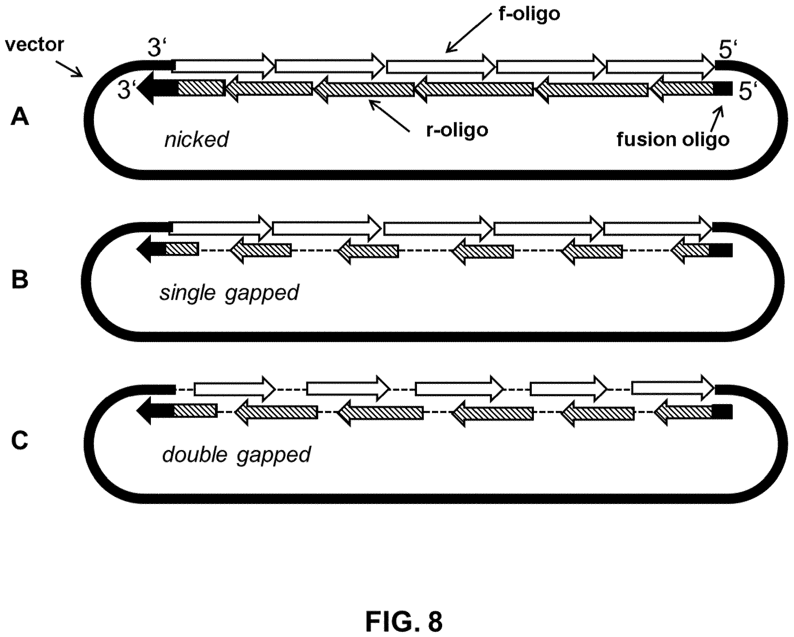

**FIG. 8** shows different oligonucleotide conformations of a Sequence Elongation and Ligation reaction with (A) oligonucleotides arranged in a "nicked" conformation; (B) oligonucleotides arranged in a "single gapped" confirmation and (C) oligonucleotides arranged in a "double gapped" confirmation, respectively.

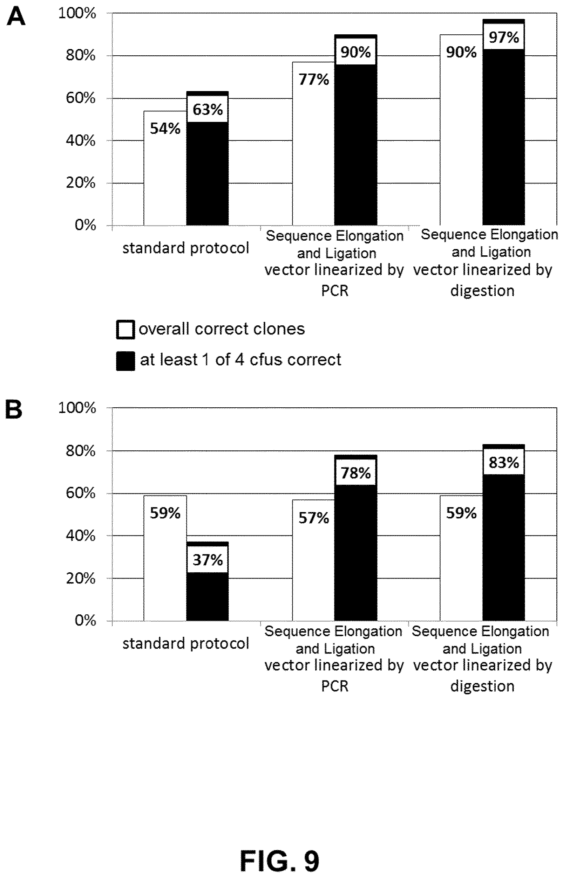

**FIG. 9** shows an analysis of (A) cloning efficiency and (B) sequence correctness of nucleic acid molecules assembled with a Sequence Elongation and Ligation reaction compared to a standard protocol.

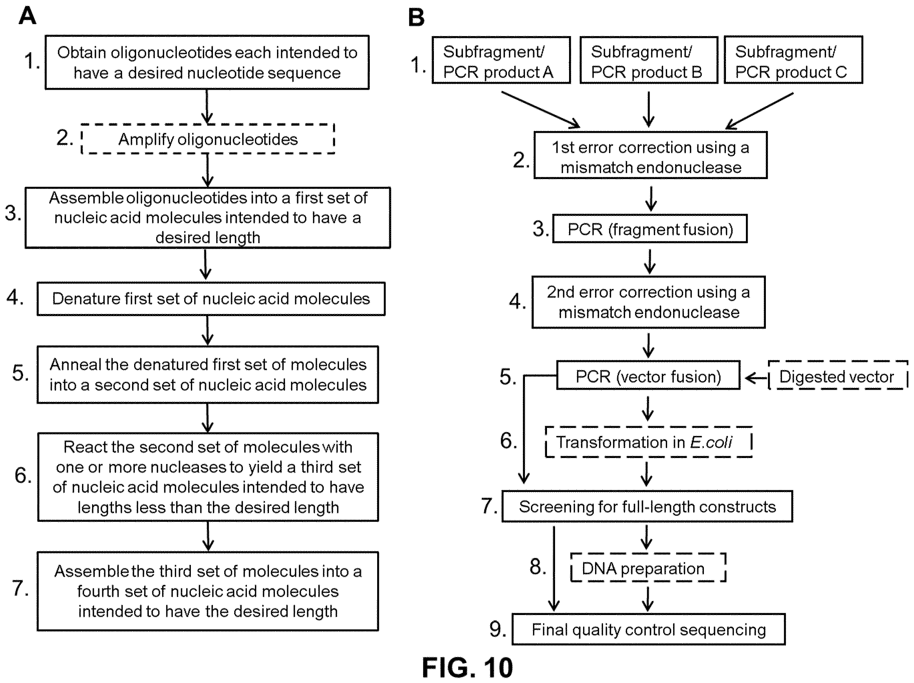

**FIG. 10** shows a workflow schematic for the assembly of oligonucleotides applying one or more error correction steps. A specific example of such workflow is shown in (B). Line numbers are labeled in the workflow for reference in the specification.

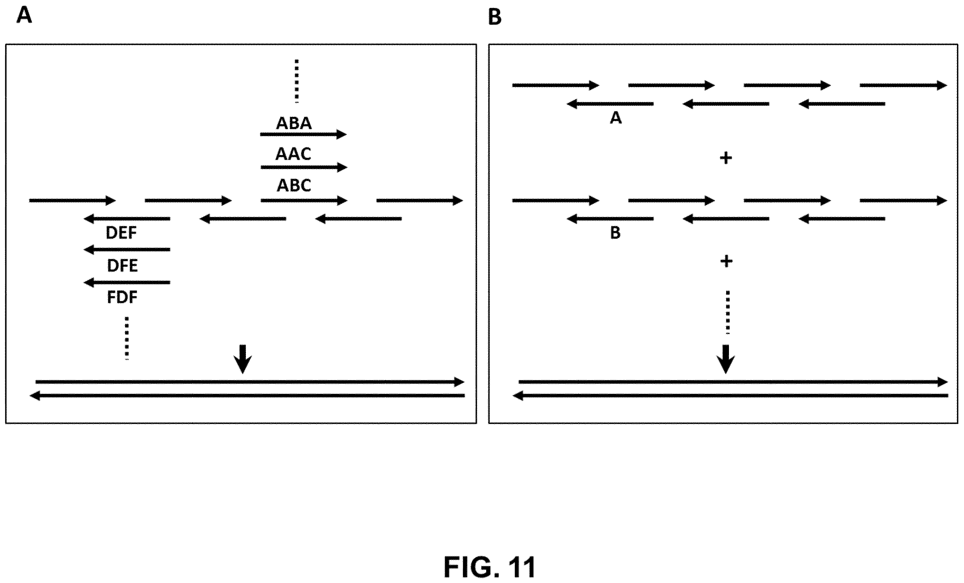

**FIG. 11** shows two exemplary strategies for library production based on fragment assembly from overlapping oligonucleotides. (A) shows a multiplex format where variations are introduced at different positions of a given fragment by mixed oligonucleotide pools. (B) shows another strategy where the same pool of overlapping oligonucleotides is used for different assembly reactions with only one or a few of the oligonucleotides replaced by a specific variant in each reaction.

### DETAILED DESCRIPTION

### Definitions

The term "nucleic acid molecule" as used herein refers to a covalently linked sequence of nucleotides or bases (*e.g.,* ribonucleotides for RNA and deoxyribonucleotides for DNA but also include DNA/RNA hybrids where the DNA is in separate strands or in the same strands) in which the 3' position of the pentose of one nucleotide is joined by a phosphodiester linkage to the 5' position of the pentose of the next nucleotide. Nucleic acid molecules may be single- or double-stranded or partially double-stranded. Nucleic acid molecule may appear in linear or circularized form in a supercoiled or relaxed formation with blunt or sticky ends and may contain "nicks". Nucleic acid molecules may be composed of completely complementary single strands or of partially complementary single strands forming at least one mismatch of bases. Nucleic acid molecules may further comprise two self-complementary sequences that may form a double-stranded stem region, optionally separated at one end by a loop sequence. The two regions of nucleic acid molecules which comprise the double-stranded stem region are substantially complementary to each other, resulting in self-hybridization. However, the stem can include one or more mismatches, insertions or deletions. Nucleic acid molecules can include polynucleotides, oligonucleotides, primers, probes, tags or adapters or portions or combinations thereof as described below.

Nucleic acid molecules may comprise chemically, enzymatically, or metabolically modified forms of nucleotides or combinations thereof. Chemically synthesized nucleic acid molecules may refer to nucleic acids typically less than or equal to 200 nucleotides long (*e.g.,* between 5 and 200, between 10 and 150, between 15 and 100, or between 20 and 50 nucleotides in length), whereas enzymatically synthesized nucleic acid molecules may encompass smaller as well as larger nucleic acid molecules as described elsewhere herein. Enzymatic synthesis of nucleic acid molecules may include stepwise processes using enzymes such as polymerases, ligases, exonucleases, endonucleases, recombinases or the like or a combination thereof. Thus, the disclosure provides, in part, compositions and combined methods relating to the enzymatic assembly of chemically synthesized nucleic acid molecules.

A nucleic acid molecule has a "5'-terminus" and a "3'-terminus" because nucleic acid molecule phosphodiester linkages occur between the 5' carbon and 3' carbon of the pentose ring of the substituent mononucleotides. The end of a nucleic acid molecule at which a new linkage would be to a 5' carbon is its 5' terminal nucleotide. The end of a nucleic acid molecule at which a new linkage would be to a 3' carbon is its 3' terminal nucleotide. A terminal nucleotide or base, as used herein, is the nucleotide at the end position of the 3'- or 5'-terminus. A nucleic acid molecule region, even if internal to a larger nucleic acid molecule (*e.g.,* a sequence region within a nucleic acid molecule), also can be said to have 5'- and 3'-ends. Nucleic acid molecule also refers to short nucleic acid molecules, often referred to as, for example, primers or probes. Also, the terms "5'-" and "3'-" refer to strands of nucleic acid molecules. Thus, a linear, single-stranded nucleic acid molecule will have a 5'-terminus and a 3'-terminus. However, a linear, double-stranded nucleic acid molecule will have a 5'-terminus and a 3'-terminus for each strand. Thus, for nucleic acid molecules that encode proteins, for example, the 3'-terminus of the sense strand may be referred to.

As used herein, a target nucleic acid may refer to a desired assembly product intended to have a predetermined or predefined sequence. A predetermined or predefined sequence may be obtained using sequence design strategies disclosed elsewhere herein.

In some instances an assembled target nucleic acid may have errors in a sense that the assembled target nucleic acid may deviate by one or more nucleotides from the predefined sequence. Such errors may be corrected, removed or filtered using methods described herein. In many instances target nucleic acids comprise double-stranded polynucleotides that may be obtained by assembly of single-stranded oligonucleotides using assembly techniques described herein.

The term "oligonucleotide", as used herein, refers to DNA and RNA, and to any other type of nucleic acid molecule that is an N-glycoside of a purine or pyrimidine base but will typically be DNA. Oligonucleotides are thus a subset of nucleic acid molecules and may be single-stranded or double-stranded. Oligonucleotides (including primers as described below) may be referred to as "forward" or "reverse" to indicate the direction in relation to a given nucleic acid sequence. For example, a forward oligonucleotide may represent a portion of a sequence of the first strand of a nucleic acid molecule (*e.g.,* the "sense" strand), whereas a reverse oligonucleotide may represent a portion of a sequence of the second strand (*e.g.,* "antisense" strand) of said nucleic acid molecule or *vice versa.* In many instances, a set of oligonucleotides used to assemble longer nucleic acid molecules (e.g. a target nucleic acid having a predefined sequence) will comprise both forward and reverse oligonucleotides capable of hybridizing to each other via complementary regions.

In many instances oligonucleotides used to assemble a larger target nucleic acid comprise an "assembly region" that comprises or represents a portion of the assembly product. Within a population of oligonucleotides used for the assembly reaction, the assembly region of an individual oligonucleotide may comprise a sequence region that is complementary to at least one other oligonucleotide in the population and therefore capable of hybridizing to that other oligonucleotide. Whereas the assembly region of a sense oligonucleotide designed to hybridize with two adjacent antisense oligonucleotides within a target nucleic acid typically have two sequence regions with complementarity to the two adjacent antisense oligonucleotides, the assembly region of a sense oligonucleotide designed to represent the 5'- or 3'- end of the target nucleic acid typically has one sequence region with complementarity to an adjacent antisense oligonucleotides. The assembly region of such "terminal" oligonucleotide may further comprise a sequence region with complementarity to another nucleic acid molecule, such as a linear double-stranded polynucleotide (i.e. a polynucleotide with two free ends) that is to be joined to or assembled with a population of oligonucleotides.

Oligonucleotides are typically less than 300 nucleotides, more typically less than 200 nucleotides, yet more typically less than 100 nucleotides in length. Thus, "primers" will generally fall into the category of oligonucleotide. Oligonucleotides can be prepared by any suitable method, including direct chemical synthesis by a method such as the phosphotriester method of Narang et al., Meth. Enzymol. 68:90-99 (1979); the phosphodiester method of Brown et al., Meth. Enzymol. 68:109-151 (1979); the diethylphosphoramidite method of Beaucage et al., Tetrahedron Letters 22:1859-1862 (1981); and the solid support method of U.S. Pat. No. 4,458,066. A review of synthesis methods of conjugates of oligonucleotides and modified nucleotides is provided in Goodchild, Bioconjugate Chemistry 1:165-187 (1990).1. Where appropriate, the term oligonucleotide may refer to a primer or probe and these terms may be exchangeably used herein. In some instances probes or "capture" oligonucleotides may be designed to specifically hybridize with a region of a target nucleic acid molecule in a mixed pool of different nucleic acid molecules such as to identify, label, select, concentrate or segregate the target nucleic acid molecule in or from the mixed pool. In some instances "capture" oligonucleotides may comprise tags or hybridized with tags contained in other nucleic acid molecules as described below.

Whereas probes may be typically used to detect at least partially complementary nucleic acid molecules, primers are often referred to as starter nucleic acid molecules for enzymatic assembly reactions. Thus, the term "primer", as used herein, refers to a short nucleic acid molecule capable of acting as a point of initiation of nucleic acid synthesis under suitable conditions. Such conditions include those in which synthesis of a primer extension product complementary to a nucleic acid strand is induced in the presence of different nucleoside triphosphates (*e.g.,* A, C, G, T and/or U) and an agent for extension (for example, a DNA polymerase or reverse transcriptase) in an appropriate buffer and at a suitable temperature. A primer is generally composed of single-stranded DNA but can be provided as a double-stranded molecule for specific applications (*e.g.,* blunt end ligation). Optionally, a primer can be naturally occurring or synthesized using chemical synthesis of recombinant procedures. The appropriate length of a primer depends on the intended use of the primer but typically ranges from about 6 to about 200 nucleotides, including intermediate ranges, such as from about 10 to about 50 nucleotides, from about 15 to about 35 nucleotides, from about 18 to about 75 nucleotides and from about 25 to about 150 nucleotides. The design of suitable primers for the amplification of a given target sequence is well known in the art and described in the literature (see for example OLIGOPERFECT^{™} Designer, Thermo Fisher Scientific). Primers can incorporate additional features which allow for the detection or immobilization of the primer but do not alter the basic property of the primer, that of acting as a point of initiation of DNA synthesis.

In some examples, an oligonucleotide (including probe, primer or tag) includes a detectable moiety or label. The label can generate, or cause to generate, a detectable signal. In some examples, the detectable signal can be generated from a chemical or physical change (*e.g*., heat, light, electrical, pH, salt concentration, enzymatic activity, or proximity events). In some examples, the label can include compounds that are luminescent, photoluminescent, electroluminescent, bioluminescent, chemiluminescent, fluorescent, phosphorescent or electrochemical. In some examples, the label can include compounds that are fluorophores, chromophores, radioisotopes, haptens, affinity tags, atoms or enzymes. In some examples, the label comprises a moiety not typically present in naturally occurring nucleotides. For example, the label can include fluorescent, luminescent or radioactive moieties. Primers may contain an additional nucleic acid sequence at the 5' end which does not hybridize to the target nucleic acid, but which facilitates cloning or detection of the amplified product, or which enables transcription of RNA (for example, by inclusion of a promoter) or translation of protein (for example, by inclusion of a 5'-UTR, such as an Internal Ribosome Entry Site (IRES) or a 3'-UTR element, such as a poly(A)n sequence, where n is in the range from about 20 to about 200). A primer can include an extendible 3' end or a non-extendible 3' end, where the terminal nucleotide at the non-extendible end carries a blocking moiety at the 2' or 3' sugar position. The region of the primer that is sufficiently complementary to the template or target nucleic acid molecule to hybridize is typically located in the 3' region of a primer and referred to herein as the hybridizing or complementary region, or target-specific region. The primer can also include a region that is designed to exhibit minimal hybridization to a portion of the template nucleic acid molecule (*e.g.,* a non-target specific sequence in the 5' region of the primer). For example, the primer can include at least one tag in the 5' non-target specific region.

A set of primers or tags used in the same amplification reaction may have melting temperatures that are substantially the same, where the melting temperatures are within about 10-5 °C of each other, or within about 5-2 °C of each other, or within about 2-0.5 °C of each other, or less than about 0.5 °C of each other.

The term "tag", as used herein, refers to a nucleic acid segment that may be used as a component of a nucleic acid molecule to identify or select or sort this nucleic acid molecule (e.g. by hybridization thereto or connection therewith). For example, a single-stranded nucleic acid molecule of 50 nucleotides in length may have at its 5' terminus a 10 nucleotide region that identifies it as coming from a particular sample. Such tags may be added to nucleic acid molecules from different samples, where nucleic acid molecules in each sample share a common tag. These tagged samples may then be mixed, with each nucleic acid molecule in the new mixture being traceable back to the sample that it came from (sample origin tracing).

Tags may also be used to identify individual nucleic acid molecules that result in the generation of specific amplification products after, for example, multiple rounds of amplification (molecule origin tracing). In such instances, tags may be used, for example, to determine the effects of "PCR heterogeneity". Typically, individual nucleic acid molecules to be analyzed are connected (*e.g.,* via ligation or PCR-based amplification) to a library of diverse (*e.g.,* degenerate) tags at an early step in a workflow. This connection is typically done in a manner such that each nucleic acid molecule in a sample (or in a diluted portion thereof) is statistically connected to a different tag that remains associated with it during amplification processes. The origin of amplified nucleic acid molecules may then be traced back to starting nucleic acid molecules. This is typically done by sequencing of nucleic acid molecules present after amplification.

Tags of the disclosure may be of any number of lengths but they will typically be long enough such that they can be readily identified (e.g., by hybridization, by sequencing, etc.) but short enough so that they do not interfere with processes related to the workflows they are used in. For example, a two hundred nucleotide tag would typically not be used to identify nucleic acid molecules when identification is done by DNA sequencing. This is so because of the read lengths of sequencing platforms. Tags will typically be from about 5 nucleotides (or base pairs) to about 50 nucleotides (or base pairs) (e.g., from about 7 to about 50, from about 10 to about 50, from about 10 to about 30, from about 7 to about 45, from about 7 to about 40, from about 5 to about 35, from about 5 to about 30, from about 5 to about 25, from about 5 to about 15, from about 7 to about 15, from about 12 to about 25 etc. nucleotides) but may also be about 100-200 nucleotides or longer depending on the required function or purpose. In some instances the assembly region of an oligonucleotide may be from about 40 to about 80 nt in length and the tag of the oligonucleotide may be from about 12 to about 24 nt in length.

In many examples, a tag or segment thereof may be single-stranded. In some examples, a tag or segment thereof may be double-stranded. In some examples, at least one end of a double-stranded tag is a blunt end or an overhang end, including a 5' or 3' overhang end. In some examples, a tag includes a blocking group which inhibits nucleotide incorporation as described elsewhere herein. Such blocking group may also be referred to as "spacer" where it separates a first portion of a nucleic acid molecule (*e.g.* a tag) from a second portion of the same nucleic acid molecule (*e.g*. an assembly region of an oligonucleotide).

Tags used in methods described herein may be designed to have a melting temperature (Tₘ) within a certain range to allow for similar or equal annealing efficiency in a hybridization or PCR amplification reaction. In some examples, the Tₘ of tags of a library may fall within a range from about 50 to about 55°C.

Where tags or tag libraries as described elsewhere herein are used to select, concentrate, segregate or specifically hybridize to desired nucleic acid molecules (*e.g.*, nucleic acid molecules that are to be selectively immobilized or amplified), the nucleotide composition of each tag within a tag library may be designed to allow for sufficient distinction between the tags such that no cross-hybridization with other target nucleic acid molecules present in a mixture occurs. In some instances, sufficiently distinct tags may be designed using "edit distance" (also referred to as "Levenshtein distance") or "Hamming distance" parameters. The Levenshtein distance is a string metric for measuring the difference between two sequences. For example, the Levenshtein distance between two sequences is the minimum number of single nucleotide changes (insertions, deletions or substitutions) required to change one sequence into another sequence.

Tags may be designed individually to have specific sequences. In many such instances, each sequence will be produced in a separate nucleic acid synthesis run. Like oligonucleotides, tags may be synthesized on a microchip or array in microscale amounts using standard chemistry or electrochemistry as disclosed elsewhere herein.

In some examples, a tag or a segment thereof can include a nucleotide sequence that is identical or complementary to any portion of a nucleic acid molecule, a "capture" oligonucleotide, capture primer, fusion primer, amplification primer, etc. In some instances a "capture" oligonucleotide comprises a single-stranded or partially single-stranded nucleic acid molecule with a sequence region that is capable of hybridizing with a tag region contained in another nucleic acid molecule (*e.g.* an oligonucleotide). Thus, in some aspects, capture oligonucleotides may be designed based on similar criteria selected for the design of respective tag regions to which they hybridize.

Capture oligonucleotides used in methods described herein may be designed to have a hybridizable portion with a melting temperature (Tₘ) within a certain range such that all capture oligonucleotides in a library allow for similar or equal annealing efficiency with their respective targets in a hybridization reaction. In some instances, capture oligonucleotides may be designed with a minimal "edit distance" or "Levenshtein distance" or "Hamming" distance as described elsewhere herein to avoid unwanted cross-hybridization between nucleic acids (*e.g.* capture oligonucleotides and tags of assembly oligonucleotides) belonging to different sorted pools. For purpose of illustration, capture oligonucleotide sequences of 15 nt used for sorting of a mixed population of assembly oligonucleotides may be designed to have a minimal Hamming distance of 3 substitutions, or capture oligonucleotide sequences of common Tₘ of 52°C to 55°C may be designed to have a minimal Hamming distance of 3 substitutions.

Capture oligonucleotides useful in methods described herein may have lengths of between about 8 and about 20 nucleotides, between about 10 and about 50 nucleotides, between about 20 and 100 nucleotides or even longer depending on the hybridization and functional requirements. ). A capture oligonucleotide may be provided in immobilized form, e.g. bound to or connected with a support as described elsewhere herein. In some instances, capture oligonucleotides may be designed to allow for sorting of defined subgroups of oligonucleotides within a mixed pool. For example one or more libraries of capture oligonucleotides may be used for combinatorial oligonucleotide assembly.

Nucleic acid molecules of the disclosure (such as *e.g.* capture oligonucleotides) may consist of or comprise one or more segments of different functionality including one or more adaptors. For example, a capture oligonucleotide may have a portion capable of hybridizing with a target nucleic acid and a portion having an adaptor.

Adaptors may be used for example, to graft a nucleic acid molecule (*e.g.* a capture oligonucleotide) to a support (*e.g.,* bead, flowcell, bottom of a well or array of reaction sites) or to connect or conjugate a first nucleic acid molecule with a second nucleic acid molecule. In some examples, an adaptor region can have any length, including fewer than 10 bases in length, or about 10-20 bases in length, or about 20-50 bases in length, or about 50-100 bases in length, or longer. An adaptor can have any combination of blunt end(s) and/or sticky end(s). In some examples, at least one end of the adaptor can be compatible with at least one end of a nucleic acid molecule. In some examples, a compatible end of the adaptor can be joined to a compatible end of a nucleic acid molecule. In some examples, the adaptor can have a 5' or 3' overhang end. In some examples, the adaptor can include an internal nick. In some examples, the adaptor can have at least one strand that lacks a terminal 5' phosphate residue. In some examples, the adaptor lacking a terminal 5' phosphate residue can be joined to a nucleic acid molecule to introduce a nick at the junction between the adaptor and the nucleic acid fragment. In some examples, the adaptor can include an oligo-dA, oligo-dT, oligo-dC, oligo-dG or oligo-U sequences. In some examples, the adaptor can include one or more inosine residues. In some examples, the adaptor can include at least one scissile linkage. In some examples, the scissile linkage can be susceptible to cleavage or degradation by an enzyme or chemical compound. In some examples, the adaptor can include at least one phosphorothiolate, phosphorothioate, and/or phosphoramidite linkage. In some examples, adaptors can include any type of restriction enzyme recognition sequence, including type I, type II, type IIS, type IIB, type III, type IV restriction enzyme recognition sequences, or recognition sequences having palindromic or non-palindromic recognition sequences.

Adaptors are often used for conducting a sequencing reaction and may therefore be included in sequencing primers for sequencing assembled target nucleic acids. In some instances, adaptors may be included in capture oligonucleotides or oligonucleotides used for assembly of larger nucleic acid molecules according to methods disclosed herein. In some instances adaptors may be included in terminal primers used for the amplification of assembled nucleic acids.

The terms "complementary" or "complementarity", as used herein, refer to the natural binding of nucleic acid molecules (primers, oligonucleotides or polynucleotides etc.) under permissive salt and temperature conditions by base pairing. For example, the sequence "A-G-T" binds to the complementary sequence "T-C-A." Complementarity between two single-stranded molecules may be "partial," such that only some of the nucleic acids bind, or it may be "complete," such that total complementarity exists between the single-stranded molecules. The degree of complementarity between nucleic acid strands has significant effects on the efficiency and strength of the hybridization between the nucleic acid strands. This is of particular importance in amplification reactions, which depend upon binding between nucleic acids strands. Complementary regions between nucleic acid molecules such as oligonucleotides may also be referred to as "overlaps" or "overlapping" regions as defined below.

The term "hybridization", as used herein, refers to any process by which a strand of nucleic acid binds with a complementary strand through base pairing. Hybridization and the strength of hybridization (for example, the strength of the association between the nucleic acids) is impacted by such factors as the degree of complementary between the nucleic acids, stringency of the conditions involved, the Tₘ of the formed hybrid, and the G:C ratio within the nucleic acids.

The term "homologous", as used herein, refers to a degree of complementarity. Nucleic acid sequences may be partially or completely homologous (identical). A partially complementary sequence is one that at least partially inhibits a completely complementary sequence from hybridizing to a target nucleic acid and is referred to using the functional term "substantially homologous".

The term "overlap" or "overlapping", as used herein often refers to a sequence homology or sequence identity shared by a portion of two or more oligonucleotides. In some instances oligonucleotides are described as overlapping if they share complementary sequence regions with capable of hybridizing to each other.

The term "gene" or "gene sequence", as used herein generally refers to a nucleic acid sequence that encodes a discrete cellular product. In many instances, a gene or gene sequence includes a DNA sequence that comprises an open reading frame (ORF) and can be transcribed into mRNA which can be translated into polypeptide chains, transcribed into rRNA or tRNA or serve as recognition sites for enzymes and other proteins involved in DNA replication, transcription and regulation. These genes include, but are not limited to, structural genes, immunity genes, regulatory genes and secretory (transport) genes etc. However, as used herein, "gene" refers not only to the nucleotide sequence encoding a specific protein, but also to any adjacent 5' and 3' non-coding nucleotide sequence involved in the regulation of expression of the protein encoded by the gene of interest. These non-coding sequences include terminator sequences, promoter sequences, upstream activator sequences, regulatory protein binding sequences, and the like. In many instances, a gene is assembled from shorter oligonucleotides or nucleic acid fragments.

The terms "fragment", "subfragment", "segment", or "component" or similar terms as used herein in connection with a nucleic acid molecule or sequence either refer to a product or intermediate product obtained from one or more process steps (*e.g.,* synthesis, assembly, amplification etc.), or refer to a portion, part or template of a longer or modified nucleic acid product to be obtained by one or more process steps (*e.g.,* assembly, amplification, construction, cloning etc.). In some instances, a nucleic acid fragment or subfragment may represent both, an assembly product (*e.g.,* assembled from multiple oligonucleotides) and a starting compound for higher order assembly (*e.g.,* a gene assembled from multiple fragments or a fragment assembled from multiple subfragments etc.).

The term "full-length" as used herein in connection with a nucleic acid molecule or sequence refers to the "desired" length of a product nucleic acid molecule or sequence to be obtained by a given process step. The term full-length is therefore not limited to the length of the final or last nucleic acid product generated in a workflow or by a series of method steps, but refers, in some instances, to the desired length of an intermediate product of a particular process step. For example, a full-length oligonucleotide may be one that does not have truncations as a result of chemical synthesis.

The term polynucleotide as used herein generally refers to any nucleic acid molecule having a certain length. Where used in the same context, polynucleotides are typically longer than oligonucleotides and may have lengths of between about 150 - 2,000 nucleotides, 200 to 5,000 nucleotides, 500 to 10,000 nucleotides or more than 1 kb, more than 2 kb, more than 5 kb, more than 10 kb or more than 20 kb. In many instances polynucleotides are obtained by assembling shorter oligonucleotides, *e.g.* via enzymatic reactions as described elsewhere herein. Polynucleotides may be single-stranded or double-stranded and may be circular or linear. An end of a linear polynucleotide is often referred to as "terminus". A terminus may be single-stranded or double-stranded and may have a sequence region that is complementary to a sequence region in another nucleic acid molecule such as an oligonucleotide to allow hybridization therewith. In some instances only the sense strand termini of a polynucleotide may have such complementary sequence region, whereas in other instances only the antisense strand termini of a polynucleotide may have such complementary sequence region. In yet other instances, both the sense and antisense strands may have such complementary sequence regions at one or both termini. In some instances a polynucleotide may be a vector or plasmid having certain functional elements.

The term "vector" or "plasmid", as used herein refers to any nucleic acid molecule capable of transferring genetic material into a host organism or able to be genetically modified to insert one or more nucleic acid molecules (*e.g.,* assembly products). A vector or plasmid may be linear (i.e. having blunt ends) or circular in topology and includes but is not limited to viruses, bacteriophages, synthetic or composed vectors. The vector or plasmid may include amplification genes, enhancers or selection markers and may or may not be integrated into the genome of the host organism. Vectors or plasmids will typically contain one or more region that renders it capable of replication in at least one cell type.

The term "amplification", as used herein, relates to the production of additional copies of a nucleic acid molecule. Amplification as used herein is often carried out using polymerase chain reaction (PCR) technologies well known in the art (*see, e.g.,* Dieffenbach, C. W. and G. S. Dveksler (1995) PCR Primer, a Laboratory Manual, Cold Spring Harbor Press, Plainview, N.Y.) but may also be carried out by other means including isothermal amplification methods such as, *e.g.*, transcription mediated amplification, strand displacement amplification, rolling circle amplification, loop-mediated isothermal amplification, helicase-dependent amplification, single primer isothermal amplification or recombinase polymerase amplification (*see, e.g.,* Fakruddin et al., "Nucleic acid amplification: Alternative methods of polymerase chain reaction", J. Pharm Bioallied Sci, 2013, v.5(4), 245-252; or Gill and Ghaemi, "Nucleic acid isothermal amplification technologies: a review", Nucleosides Nucleotides Nucleic Acids. 2008 27(3), 224-43).

The term "adjacent", as used herein, refers to a position in a nucleic acid molecule immediately 5' or 3' to a reference region.

The term "transition", when used in reference to the nucleotide sequence of a nucleic acid molecule, refers to a point mutation that changes a purine nucleotide to another purine (A ↔ G) or a pyrimidine nucleotide to another pyrimidine (C ↔ T).

The term "transversion", when used in reference to the nucleotide sequence of a nucleic acid molecule, refers to a point mutation involving the substitution of a (two ring) purine for a (one ring) pyrimidine or a (one ring) pyrimidine for a (two ring) purine.

The term "indel", as used herein, refers to the insertion or deletion of one or more bases in a nucleic acid molecule.

The term "sequence fidelity", as used herein refers to the level of sequence identity of a nucleic acid molecule as compared to a reference sequence. Full identity being 100% identical over the full-length of the nucleic acid molecules being scored for sequence identity. Sequence fidelity can be measure in a number of ways, for example, by the comparison of the actual nucleotide sequence of a nucleic acid molecule to a desired nucleotide sequence (*e.g.,* a nucleotide sequence that one wishes to be used to generate a nucleic acid molecule). Another way sequence fidelity can be measured is by comparison of sequences of two nucleic acid molecules in a reaction mixture. In many instances, the difference on a per base basis will be, on average, the same.

The term "error correction", as used herein refers to changes in the nucleotide sequence of a nucleic acid molecule to alter a defect. These defects can be mismatches, insertions, deletions and/or substitutions. Defects can occur when a nucleic acid molecule that is being generated (*e.g.,* by chemical or enzymatic synthesis) is intended to contain a particular base at a location but a different base is present at that location.

The term "transformation", as used herein, describes a process by which an exogenous nucleic acid molecule enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such "transformed" cells include stably transformed cells in which the inserted nucleic acid is capable of replication either as an autonomously replicating plasmid or as part of the host chromosome. They also include cells that transiently express the inserted DNA or RNA for limited periods of time.

The terms "microchip", "chip", "synthesis chip", or similar variations thereof as used herein will refer to an electronic computer chip on which oligonucleotide synthesis can occur.

The term "microarray" or "array", as used herein refers to an array of distinct polynucleotides or oligonucleotides arrayed on a substrate, such as paper, nylon or any other type of membrane, filter, chip, glass slide, or any other suitable solid support. In certain instances a microchip may represent a specific example of a microarray.

The terms "multiwell plate", "microplate", "microwell plate", "plate" or similar variations thereof refers to a two-dimensional array of multiple wells located on a substantially flat surface. Multiwell plates can comprise any number of wells of any width or depth. In certain instances, a multiwell plate may be configured as a microchip. For example, when a material with well-like structures is overlaid onto a microchip.

The term "solid support", as used herein refers to a porous or non-porous material on which polymers such as oligonucleotides or nucleic acid molecules can be synthesized and/or immobilized. As used herein "porous" means that the material contains pores which may be of non-uniform or uniform diameters (for example in the nm range). Porous materials include paper, synthetic filters, etc. In such porous materials, the reaction may take place within the pores. The solid support can have any one of a number of shapes, such as pin, strip, plate, disk, rod, fiber, bends, cylindrical structure, planar surface, concave or convex surface or a capillary or column. The solid support can be a particle, including bead, microparticles, nanoparticles and the like. The solid support can be a non-bead type particle (*e.g.,* a filament) of similar size. The support can have variable widths and sizes. For example, sizes of a bead (*e.g.,* a magnetic bead) which may be used in the practice of aspects of the disclosure may vary widely but include beads with diameters between 0.01 µm and 100 µm, 0.005 µm and 100 µm, 0.005 µm and 10 µm, 0.01 µm and 100 µm, 0.01 µm and 1,000 µm, between 1.0 µm and 2.0 µm, between 1.0 µm and 100 µm, 15 between 2.0 µm and 100 µm, between 3.0 µm and 100 µm, between 0.5 µm and 50 µm, between 0.5 µm and 20 µm, between 1.0 µm and 10 µm, between 1.0 µm and 20 µm, between 1.0 µm and 30 µm, between 10 µm and 40 µm, between 10 µm and 60 µm, between 10 µm and 80 µm, or between 0.5 µm and 10 µm.

The support can be hydrophobic or capable of binding a molecule via hydrophobic interaction. The support can be hydrophilic or capable of being rendered hydrophilic and includes inorganic powders such as silica, magnesium sulfate, and alumina; natural polymeric materials, particularly cellulosic materials and materials derived from cellulose, such as fiber containing papers such as filter paper, chromatographic paper or the like. The support can be immobilized or located at an addressable position of a carrier such as, *e.g.,* a multiwell plate or a microchip. The support can be loose or particulate (such as, *e.g.,* a resin material or a bead in a well) or can be reversibly immobilized or linked to the carrier (*e.g.,* by cleavable chemical bonds or magnetic forces etc.). For example, a plurality of nucleic acids having a desired sequence (such as a specific type of oligonucleotide) may be synthesized or provided on individual supports in an array device or carrier where each support is located at a uniquely addressable position such that one or more individual supports and specific nucleic acids bound thereto can be selectively retrieved from defined positions of the array or carrier. As used herein an "addressable position" generally refers to a location within an array or carrier that may be readily identified so that a nucleic acid molecule located at a specific position may be individually processed at or retrieved from the one or more specific position of said array or carrier. In some instances an array or carrier may comprise multiple individual supports (such as beads) located at distinct addressable positions (such as individual wells of a multiwell plate or a microchip) with each support carrying a specific type of oligonucleotide (an oligonucleotide having a desired base composition and/or length and/or sequence). In some instances the carrier is a microchip with multiple wells, each well configured to receive a single support. Single supports may be retrieved as disclosed for example in PCT Publication WO 2016/094512.

In some examples, solid support may be fragmentable. Solid supports may be synthetic or modified naturally occurring polymers, such as nitrocellulose, carbon, cellulose acetate, polyvinyl chloride, polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, magnetic or non-magnetic beads, ceramics, metals, and the like; either used by themselves or in conjunction with other materials.

Supports used in methods, compositions or kits described herein may have various functions. In some instances a support may be used for synthesizing nucleic acid molecules thereon. In other instances a support may be used for immobilizing one or more nucleic acid molecules thereon and may be further used for pooling, sorting selecting or identifying the immobilized nucleic acid molecules or other nucleic acids hybridized or connected thereto. In some examples, the support can be in a chip, array, microarray or microwell plate format. In many instances, a support used in methods or compositions of the disclosure will be one where individual nucleic acid molecules are synthesized on separate or discrete areas to generate features (*i.e*., locations containing individual nucleic acid molecules) on the support. In some examples, the size of the defined feature is chosen to allow formation of a microvolume droplet or reaction volume on the feature, each droplet or reaction volume being kept separate from each other. As described herein, features are typically, but need not be, separated by interfeature spaces to ensure that droplets or reaction volumes or between two adjacent features do not merge. Interfeatures will typically not carry any nucleic acid molecules on their surface and will correspond to inert space. In some examples, features and interfeatures may differ in their hydrophilicity or hydrophobicity properties. In some examples, features and interfeatures may comprise a modifier. In one example of the disclosure the feature is a well or microwell or a notch. Nucleic acid molecules may be covalently or non-covalently attached to the surface or deposited or synthesized or assembled on the surface.

"a", "an", and "the" include plural reference unless the context clearly dictates otherwise.

### Overview

The disclosure relates, in part, to compositions and methods for the preparation of nucleic acid molecules. While the disclosure has numerous aspects and variations associated with it, some of these aspects and variations of applicability of the technology may be related to one or more process steps represented in the exemplary workflow shown in FIG. 1.

FIG. 1 shows a workflow directed to compositions and methods for designing and generating a desired nucleic acid molecule (*e.g*., a gene). In this workflow, the first step is design the nucleic acid molecule and, in most instances, subfragments that will be used in the workflow to generate the nucleic acid molecule. Once the design phase is completed, the oligonucleotides that make up the desired nucleic acid molecules can then be generated and prepared for use in down-stream reactions (*e.g.,* amplification reactions).

The next step in the workflow shown in FIG. 1 is the sorting and pooling of oligonucleotides that will be used to produce the desired, product nucleic acid molecule. In many instances, oligonucleotides used to form the product nucleic acid molecule will be produced on solid supports. Where solid supports are particulate (*e.g.,* beads) these solid supports may be pooled (as illustrated e.g. in FIG. 3) and the oligonucleotides may then be released from the solid supports. Then oligonucleotides that are to be used in the generation of a desired nucleic acid molecule may be collected prior to assembly of a desired nucleic acid molecule, or oligonucleotides may not be separated but used to assembly a plurality of different desired product nucleic acid molecules in the same reaction vessel. The process chosen will typically be directed by the specifics of the assembly process, the synthesis platform used and the desired products nucleic acid molecule or molecules.

According to the workflow in FIG. 1, once the appropriate oligonucleotides are collected, these oligonucleotides are then used to generate larger nucleic acid molecules through a series of assembly reactions, which may then be used as sub-components for the assembly of larger nucleic acid molecules (*see, e.g.,* FIGs. 2 and 6). Typically, nucleic acid amplification reactions (*e.g.,* PCR) are used for the assembly process.

Also according to some examples of the workflow in FIG. 1, assembled nucleic acid molecules are cloned. Typically, assembled nucleic acid molecules are inserted into a vector, then the vector is introduced into a host cell, followed by selection to generate clonal isolates containing candidate inserts representing the desired product nucleic acid molecule. These candidates may then be sequenced to determine (1) whether the desired product nucleic acid molecule is present and, if so, (2) whether the nucleotide sequence of the desired product nucleic acid molecule is correct. Further, a sufficient number of clonal isolates will typically be generated to have a high probability that at least one of the clonal isolates is of the desired product nucleic acid molecule with the correct sequence. According to an alternative example, cloning is avoided and assembled sequences determined to have error-free sequences are retrieved from the pool of generated nucleic acid molecules for downstream processing or use in further assembly steps.

In some aspect, methods are provided for the production of nucleic acid molecules having high "sequence fidelity". This high sequence fidelity can be achieved by, for example, one two or all four of the following: accurate nucleic acid synthesis, selection of oligonucleotides and sub-assembly components having "correct" sequences, error correction, and sequence verification of product nucleic acid molecules. Components and methods for achieving high sequence fidelity will generally be used in more than one portion of workflows and each component or method may be sued more than once. For example, the exemplary workflow set out in FIG. 1 shows error correction/correct sequence selection occurring at two different locations in the workflow.

Described herein are a number of components and/or methods with applicability to workflows such as those shown in FIG. 1, as well as other workflows. Thus, components and/or methods set out herein may be useful, for example, (1) in workflows such as that set out in FIG. 1, (2) in other workflows related to nucleic acid biology, (3) as "stand alone" components and/or methods, and (4) as components and/or methods that may be practiced in combination with other components and/or methods set out herein.

### Sequence Design

As noted above, often, one of the first steps in producing a nucleic acid molecule or protein of interest, after the molecule(s) has been identified, is nucleic acid molecule design. A number of factors go into design of the nucleic acid sequence to be synthesized and the oligonucleotides used to generate the nucleic acid molecule. These factors include one or more of the following: (1) the AT/GC content of all or part of the nucleic acid molecule (*e.g.,* the coding region), (2) the presence or absence of restriction endonuclease cleavage sites (including the addition and/or removal of restriction sites), (3) preferred codon usage for the particular protein production or host expression system that is to be employed, (4) junctions of the oligonucleotides being assembled, (5) the number and lengths of the oligonucleotides used to produce the desired nucleic acid molecule, (6) minimization of undesirable regions (*e.g.,* "hairpin" sequences, regions of sequence homology to cellular nucleic acids, repetitive sequences, inhibitory cis-acting elements, restriction enzyme cleavage sites, internal splice sites etc.) and (7) coding region flanking segments that may be used for attachment of 5' and 3' components (*e.g.,* restriction endonuclease sites, primer binding sites, sequencing adaptors or tags, recombination sites, etc.).

In many instances, parameters will be input into a computer and software will generate an *in silico* nucleotide sequence that balances the input parameters. The software may place "weights" on the input parameters in that, for example, what is considered to be a nucleic acid molecule that closely matches some of the input criteria may be difficult or impossible to assemble. Exemplary nucleic acid design methods are set out in U.S. Patent No. 8,224,578.

One main aspect of nucleic acid molecule design is the probability that the nucleic acid molecule can be produced in a "single-run". Put another way, the probability that a particular synthesis and assembly cycle will result in the generation of the designed nucleic acid molecule. This may be estimated by the use of data derived from past synthesis and assembly cycles and characteristics of the designed nucleic acid molecules. One factor that results in some synthesis assembly failures is high GC content. A number of nucleic acid design parameters are set out in Fath et al., PLoS One, 6:e17596 (2011).

Further, nucleic acid molecules design factors may be considered across the length of the nucleic acid molecule or in specific regions of the molecule. For example, GC content may be limited across the length of the nucleic acid molecule to prevent synthesis "failures" resulting from specific locations within the molecule. Thus, synthesizability of the nucleic acid molecule is a characteristic of the entire nucleic acid molecule in that a regional "failure to assemble" results in the designed nucleic acid molecule not being assembled. From a regional perspective, codon may be selected for optimal translation but this may conflict with, for example, region limitation of GC content.

Assembly success often involves multiple parameters and regional characteristics of the desired nucleic acid molecule. Total and regional GC content is only one example of a parameter. For example, the total GC content of a nucleic acid molecule may be 50% but the GC content in a particular region of the same nucleic acid molecule may be 75%. Thus, in many instances, GC content will be "balanced" across the entire nucleic acid molecule and may vary regionally by less than 15%, 10%, 8%, 7%, or 5% from the total GC content.

Further, the designed nucleic acid molecule may be based upon a naturally occurring nucleic acid molecule and modifications are made to the naturally occurring to enhance synthesizability. In some instances, naturally occurring nucleic acid molecules are not considered in the production of a product nucleic acid molecule and only synthesis and expression factors are used in the design process.

A synthetic gene ought therefore to be optimized in relation to the codon usage and the GC content and, on the other hand, substantially avoid the problems associated with DNA motifs and sequence repeats and inverse complementary sequence repeats. These requirements cannot, however, ordinarily be satisfied simultaneously and in an optimal manner. When a limited set of codons is used, often the repetitive nature of a coding sequence increases. Thus, alteration of a coding sequence to what is considered preferred codon usage may lead to a highly repetitive sequence and a considerable difference from the desired GC content. Further, coding regions designed to have either low GC or high GC content will exacerbate this problem by limiting the codons used by base type.

The aim therefore is to reach a compromise which is as optimal as possible between satisfying the various requirements. In instances where the product nucleic acid molecule encodes a protein, the large number of amino acids in the protein leads to a combinatorial explosion of the number of possible DNA sequences which - in principle - are able to express the desired protein based on the degeneracy of the genetic code. For this reason, various computer-assisted methods have been proposed for ascertaining an optimal codon sequence. Further factors which may influence the result of expression are DNA motifs and repeats or inverse complementary repeats in the base sequence. Certain base sequences produce in a given organism certain functions which may not be desired within a coding sequence. Examples are *cis*-active sequence motifs such as splice sites or transcription terminators. The presence of a particular motif may reduce or entirely suppress expression or even have a toxic effect on the host organism. Sequence repeats may lead to lower genetic stability and impede the synthesis of repetitive segments owing to the risk of incorrect hybridizations. Inverse complementary repeats may lead to the formation of unwanted secondary structures at the RNA level or cruciform structures at the DNA level, which impede transcription and lead to genetic instability, or may have an adverse effect on translation efficiency.

Repetitive sequence segments may, for example, lead to low genetic stability. The synthesis of repetitive segments is also made distinctly difficult because of the risk of faulty hybridization. Therefore, the assessment of a test sequence includes whether it comprises identical or mutually similar sequence segments at various points. The presence of corresponding segments can be established for example with the aid of a variant of a dynamic programming algorithm for generating a local alignment of the mutually similar sequence segments. Such an algorithm is set out in U.S. Patent Publication No. 2007/0141557. Typically, to calculate the criterion weight relating to the repetitive elements, the individual weights of all the local alignments where the alignment weight exceeds a certain threshold value are summed. Addition of these individual weights gives the criterion weight which characterizes the repetitiveness of the test sequence.

Inverse complementary repeats present the potential formation of secondary structures and the RNA level or cruciform structures at the DNA level can be recognized on the test sequence by the presence of such inverse complementary repeats (inverse repeats). Cruciform structures at the DNA level may impede translation and lead to genetic instability. Further, the formation of secondary structures at the RNA level may have adverse effects on translation efficiency. In this connection, inverse repeats of particular importance are those which form hairpin loops or cruciform structures. Faulty hybridizations or hairpin loops may also have adverse effects in the synthesis of the former from oligonucleotides.

Typically, nucleic acid molecules will be designed to have an estimated and/or actual synthesis and assembly cycle success rate of at least 90% (e.g., from about 90% to about 99.5%, from about 92% to about 99.5%, from about 93% to about 99.5%, from about 94% to about 99.5%, from about 95% to about 99.5%, from about 90% to about 98%, from about 92% to about 98%, from about 93% to about 98.5%, from about 94% to about 98.5%, etc.).

As noted herein one important feature of the coding region is codon usage. Codon usage varies with a number of factors, including the particular organism and the type of gene being expressed within that organism. For example, it has been found in both Drosophila and Caenorhabditis that codon bias plays a major role in the selection of highly expressed genes. Further, "optimal" codons typically correspond to the tRNA molecules that are most abundant in a cell. These observations support the translation selection hypothesis which states that codon usage has been shaped by selection to improve the efficiency of translation of certain proteins. Thus, protein expression levels within cells appear to be partly controlled by the codons of the particular gene. Also, expression efficiency is also thought to increase with the use of preferred codons for a particular cell type and there is evidence that low-frequency-usage codons within a coding sequence provides for genetic instruction that regulates the rate of protein synthesis. (Reviewed in Angov, "Codon usage: Nature's roadmap to expression and folding of proteins, " Biotechnol. J., 6:650-659 (2011).)

Codon usage may be adjusted across a coding region and/or in portions of coding regions to match desired codons. In many instances, this will be done while maintaining a desired level of synthesizability.

The *in silico* design of the nucleic acid in terms of synthesizability may also include "fragmentation" of the full-length nucleic acid sequence into smaller fragments ("subfragments") that can be assembled based on single-stranded oligonucleotides. Methods and systems for automated nucleic acid synthesis design are described for example in U.S. Patent No. 7,164,992. In most instances, oligonucleotides will provide the starting point for the methods and compositions underlying the present disclosure.

### Oligonucleotide Synthesis

Oligonucleotides or nucleic acid sub fragments used for assembly of a desired nucleic acid molecule may be derived from a number of sources, for example, they may be cloned, derived from polymerase chain reactions, chemically synthesized or purchased. The oligonucleotides for an assembly reaction may be obtained according to any method disclosed or referred to herein, as, *e.g.,* by synthesizing on a microarray or microchip as disclosed in PCT Publication WO 2016/094512.

In many instances, chemically synthesized oligonucleotides tend to be of less than 100 nucleotides in length. PCR and cloning can be used to generate much longer nucleic acids. Further, the percentage of erroneous bases present in nucleic acids (*e.g.,* nucleic acid fragments) is, to some extent, tied to the method by which it is made. Typically, chemically synthesized nucleic acids have the highest error rate.

A number of methods for chemical synthesis of oligonucleotides are known. In many instances, oligonucleotide synthesis is performed by a stepwise addition of nucleotides to the 5'-end of the growing chain until oligonucleotides of desired length and sequence are obtained. Further, each nucleotide addition can be referred to as a synthesis cycle and often consists of four chemical reactions: (1) De-Blocking/De-Protection, (2) Coupling, (3) Capping, and (4) Oxidation. One example of chemical oligonucleotide synthesis chemical processes is set out below.

Nucleic acid synthesis typically starts with selection of synthesis components or "building blocks" used to produce oligonucleotides. Often 3'-O-(N,N-diisopropyl phosphoramidite) derivatives of nucleosides (nucleoside phosphoramidites) are used as building blocks in phosphite triester methodologies. To prevent undesired side reactions, all other functional groups present in the nucleosides are normally rendered unreactive (protected) by the attachment of protecting groups. Upon oligonucleotide synthesis completion, remaining protecting groups are typically removed to yield the desired oligonucleotides free of protein groups.

5'-hydroxyl groups are often protected by an acid-labile DMT (4,4'-dimethoxytrityl) group. Phosphite group are often protected by a base-labile 2-cyanoethyl group.

De-blocking/De-protection: DMT groups are often removed with a solution of one or more acids, such as trichloroacetic acid (TCA) or dichloroacetic acid (DCA), present in an inert solvent (dichloromethane or toluene), resulting in the solid support-bound oligonucleotide precursor bearing a free 5'-terminal hydroxyl group. A washing step typically follows.

Coupling: In an exemplary process, a solution of nucleoside phosphoramidite in acetonitrile is activated by a solution of an acidic azole catalyst (*e.g.,* 1 H-tetrazole, 2-ethylthiotetrazole, 2-benzylthiotetrazole, or 4,5-dicyanoimidazole) and then brought in contact with the solid support where free 5'-hydroxy group reacts with the activated phosphoramidite moiety of the incoming nucleoside phosphoramidite to form a phosphite triester linkage. The coupling reaction of 2'-deoxynucleoside phosphoramidites is rapid and goes to near completion in about 20 seconds. Upon the completion of the coupling, unbound reagents and by-products are typically removed by washing.

Capping: Typically, after the completion of the coupling reaction, a small percentage (generally in the range of 0.1 to 1%) of the solid support-bound 5'-OH groups is unreacted and must be permanently blocked from further chain elongation so as to prevent the formation of oligonucleotides with an internal base deletion commonly referred to as (n-1) shortmers. Unreacted 5'-hydroxy groups are, to a large extent, acetylated by the capping mixture. Capping steps are often performed by treating the solid support-bound material with a mixture of acetic anhydride and 1-methylimidazole.

Oxidation: The newly formed tricoordinated phosphite triester linkage typically formed by chemical synthesis is not natural and is relatively unstable. Treatment with reagents containing iodine and water in the presence of a weak base (*e.g.,* pyridine, lutidine, or collidine) oxidizes the phosphite triester linkage into a tetracoordinated phosphate triester, which is a protected precursor of naturally occurring phosphodiester internucleosidic linkages.

A number of variations to the oligonucleotide synthesis process may be made. For example, electrochemically generated acid (EGA) or photogenerated acid (PGA) may be used to remove the protecting group (*e.g.,* DMT) before the next amidite is added to the nucleic acid molecule attached to the solid support. In some examples, at least one proton carrier, such as 2-chloro-6-methylpyridine or diphenylamine, may be present in the solution with the EGA or PGA. The at least one proton carrier may act to reduce the effect of DNA degradation by accepting protons from the EGA or PGA, thereby adjusting the acidity of the solution.

EGA and PGA deprotection reagents and methods for generating such acids, as well as their use in oligonucleotide synthesis are set out for example in Maurer et al., "Electrochemically Generated Acid and Its Containment to 100 Micron Reaction Areas for the Production of DNA Microarrays ", PLoS, Issue 1, e34 (2006), or in PCT Publications WO 2013/049227 and WO 2016/094512.

While in many instances oligonucleotides may be produced using phosphoramidite synthesis chemistry, as well as variations thereof, other methods may also be used to produce oligonucleotides, including PCR, restriction enzyme digest, exonuclease treatment, or template-independent synthesis using a nucleotidyl transferase enzyme. Exemplary methods of template-independent synthesis using a nucleotidyl transferase enzyme are set out in U.S. Patent No. 8,808,989. The nucleotidyl transferase enzyme (*e.g.,* terminal deoxynucleotidyl transferase) is used to incorporate nucleotide analogs having an unmodified 3' hydroxyl and a cleavable protecting group. Because of the protecting group, synthesis pauses with the addition of each new base, whereupon the protecting group is cleaved, leaving a polynucleotide that is essentially identical to a naturally occurring nucleotide (*i.e.,* is recognized by the enzyme as a substrate for further nucleotide addition). Thus, in certain examples, the disclosure includes methods in which oligonucleotides are produced by enzymatic reaction.

Nucleotide triphosphates (*e.g.,* deoxynucleotide triphosphates) (NTPs) suitable for use with enzymatic oligonucleotide synthesis methods will have protecting groups that do not prevent the NTPs from being used by a nucleotidyl transferase as a substrate and can be efficiently removed to allow for addition to an oligonucleotide chain. Thus, in certain examples, the nucleotide addition occurs via enzymatic reaction. In some instances, EGA is generated as part of the deprotection process. Further, in certain instances, all or part of the oligonucleotide synthesis reaction may be performed in aqueous solutions. In other instances, organic solvents will be used.

In some examples of the disclosure, oligonucleotides are attached to one or more solid support (*e.g.,* a magnetic or non-magnetic bead, a matrix, a resin, a gel, the surface of an array etc.). In some examples oligonucleotides are attached to one or more support via a succinyl linker. In certain examples, a universal linker may be located between the succinyl group and the oligonucleotides. Alternatively, the linker may have a specific base attached as the starting base of the oligonucleotide. In such instances, solid supports (*e.g.,* once having a dA, dT, dC, dG, or dU) are selected based upon the starting base of the oligonucleotide being synthesized.

### Post Processing and Pooling

Once the oligonucleotide synthesis has been completed, the resulting oligonucleotides are typically subjected to a series of post processing steps that may include one or more of the following: (a) cleavage of the oligonucleotides or elution from the support, (b) concentration measurement, (c) concentration adjustment or dilution of oligonucleotide solutions, often referred to as "normalization", to obtain equally concentrated dilutions of each oligonucleotide species, and, optionally, (d) pooling or mixing aliquots of two or more normalized oligonucleotide samples to obtain equimolar mixtures of all oligonucleotides required to assemble one or more specific nucleic acid molecules, wherein the aforementioned steps may be combined in different orders.

The method used to cleave or elute the oligonucleotides from a support is often specific for the linking group through which the oligonucleotide is linked to the solid support. Typically, oligonucleotides are cleaved from solid supports using, for example, gaseous ammonia, aqueous ammonium hydroxide, aqueous methylamine, or mixtures thereof. A succinyl linker may be cleaved by the use of, for example, concentrated aqueous ammonium hydroxide. The reaction is usually carried out at temperatures between 50°C and 80°C for at least one to about eight hours. In certain examples, the succinyl linker may be cleaved by the use of ammonia gas, using increased heat and pressure, such as, for example, a temperature of about 80°C, and a pressure of about 3 bars for a time of about 2 hours.

In some instances, oligonucleotides will be released from synthesis supports and then pooled as unbound nucleic acid molecules for subsequent assembly steps. In some examples, oligonucleotides attached to individual or "loose" solid supports (such as microparticles or beads) may be pooled before they are released. Depending on the nucleic acid molecule to be assembled, the number of solid supports that may be pooled prior to release of synthesized oligonucleotides may vary widely and include from about 10 to about 50, from about 50 to about 100, from about 100 to about 1,000, from about 50 to about 10,000, from about 100 to about 10,000, or from about 500 to about 10,000 individual supports. Pooled oligonucleotides may be components of a single fragment or subfragment to be assembled or may be components of multiple fragments or subfragments to be assembled simultaneously in a single reaction ("multiplex assembly").

In standard procedures, each oligonucleotide required to assemble a fragment or subfragment is typically synthesized in excess. In instances where oligonucleotides are released from supports prior to pooling, the concentration of individual oligonucleotides is typically determined by optical density measurement. After appropriate dilution of each required oligonucleotide solution, equal amounts or volumes of each diluted solution are generally pooled, *e.g.,* by manual pipetting or by using an automated pipetting device to obtain oligonucleotide mixtures for subsequent assembly. In many instances, the pooled oligonucleotides may be assembled by PCR-based methods as described elsewhere herein.

To shorten the time associated with post-processing, normalization and pooling of oligonucleotides may occur in a single step, e.g. by using a modification on the 5' nucleotide that is added during or after the last synthesis cycle. Such modification may comprise digoxigenin, acrydite, SSH-linker, monomethoxytrityl, fluorous label, primary amines or other modifications known to the person skilled in the art that allow for selective retrieval or purification. In some examples, the modification is a trityl group, such as a dimethyltrityl (DMT) group typically attached to the 5' hydroxyl groups of phosphoramidite building blocks used for chemical oligonucleotide synthesis. Typically, the protecting groups on the oligonucleotide backbone are base labile and may be removed simultaneously with cleavage of the oligonucleotide from the solid phase (a process often referred to as cleavage and deprotection as described in more detail above). In contrast, the terminal DMT group is acid sensitive and will therefore not be removed during ammonia-based cleavage and deprotection. This allows for selective maintenance of the terminal DMT group on the cleaved and deprotected oligonucleotide. Thus, if not removed by a separate de-blocking step after the last base has been coupled, the trityl group may be used to selectively remove and/or purify one or more oligonucleotides having a desired length and/or sequence.

As indicated above, a portion of oligonucleotides synthesized on a support may be truncated (*i.e.,* have a length less than the desired length). A truncated oligonucleotide may, for example, be generated where a 5'-DMT protecting group has been removed but no base coupling has occurred resulting in an unreacted 5'-hydroxyl group which may be subject to additional coupling in a subsequent synthesis step. To avoid accumulation of deletions with each successive cycle a "capping step" is used to block any unreacted 5'-hydroxyl groups. This will, however, result in shorter oligonucleotides as no further bases can be added to capped oligonucleotides in subsequent reactions. Such truncations accumulate with increasing length of oligonucleotides being synthesized. Although the coupling efficiency may be about 99% using suitable synthesis protocols, oligonucleotides having deletions or truncations (which increase with the length of an oligonucleotide to be synthesized as discussed *supra*), may significantly affect subsequent assembly processes, even if present only at a low amount. It is therefore desired to generate a large portion or high yield of oligonucleotides having the desired full-length.

To address this problem full-length oligonucleotides may be selectively retrieved based on a terminal modification such as a trityl group that will only be present on the last base of a full-length oligonucleotide, whereas truncated oligonucleotides will not have such modification. Oligonucleotides comprising full-length sequences can then be selectively retrieved from a mixture of full-length and truncated oligonucleotides. In some aspects, members of a mixture of oligonucleotides that lack the terminal modification may be truncated oligonucleotides (*i.e.*, they may have one or more base deletions compared to the sequence of the desired full-length oligonucleotide to be synthesized).

Oligonucleotides carrying terminal modifications may be captured by various means. For example, oligonucleotides carrying a terminal trityl group may be captured by hydrophobic interaction. For example, C18 silica or porous matrix may be used to capture oligonucleotides with trityl groups in aqueous solution. Other suitable supports include C5 or C4 silica supports or polystyrene based supports available from various providers including, *e.g.*, Sigma Aldrich SUPELCO^{®}, Bioclone Inc., Thermo Fisher Scientific or MoBiTec. In some examples, particulate supports such as beads may be used to capture full-length oligonucleotides carrying a terminal modification. In aspects of the disclosure, the hydrophobic matrix used to capture molecules with trityl modifications may, *e.g.,* be magnetic material, such as magnetic beads which allow for oligonucleotide purification or selection in a high throughput setting. For example, magnetic beads may be used to capture and purify trityl-carrying molecules using an automatic handling platform like, *e.g.,* the THERMO SCIENTIFIC^{™} KINGFISHER^{™} Flex Purification Systems (Thermo Fisher Scientific). Such automatic handling platform allows for transfer of magnetic beads from well to well during the purification procedure, thereby selectively collecting full-length oligonucleotides from each well.

In an exemplary automated workflow, oligonucleotide samples each representing a portion of a desired nucleic acid molecule to be assembled can be provided as trityl-on molecules in wells of a multiwell plate at varying concentrations. A first sample of oligonucleotides comprising a mixture of full-length oligonucleotides carrying a terminal DMT group and truncated species not carrying such modification can be contacted in a first well with a defined amount of magnetic C18 beads, capable of binding less than the minimum concentration required for a standard PCR assembly reaction. For purposes of illustration, where 1.5 pmol of each oligonucleotide is required for a standard PCR assembly reaction and 1 mg of C18 support is capable of binding about 6 nmol of oligonucleotides, 0.25 ng of C18 support would capture 1.5 pmol of oligonucleotides to attain a final pool with the right quantity and concentration for one assembly reaction.

Beads capable of binding full-length oligonucleotides via hydrophobic interaction with the terminal trityl group can then collected with a magnetic pipette. Beads collected from the well can be directly released into a vessel or well of another multiwell plate, optionally comprising a PCR reaction mix. The pipette can then washed and used to collect beads from the next well and combined those with the oligonucleotides collected from the previous well etc. Efficient pooling can also be achieved where the pipette is used to subsequently collect all beads from adjacent wells for a single assembly reaction and all beads carrying full-length oligonucleotides from different samples are then deposited into one or more target wells for PCR assembly.

In some examples, manual workflows may be used to isolate matrix carrying modified oligonucleotides. In an exemplary manual protocol, a magnetic field applied by an external source may be used to capture magnetic beads against the wall of a tube, well or container. The supernatant containing unbound truncated molecules may be removed for example by aspiration. Another option would be the use of a manual magnetic pipette tool (*e.g*., QUICKPICK^{™} from Bionobile, Finland). This pipette contains a strong magnet that can be lowered into a closed pipette tip, the magnetic particles stick to the pipette tip surface and can be transferred into a new vessel.

Captured oligonucleotides collected from multiple wells may then be pooled or released into a vessel or a well of a multiwell plate for subsequent assembly. In some examples, the oligonucleotides may be eluted from the support prior to assembly. Elution may be achieved by various means depending on the type of 5' modification. For example, molecules comprising a DMT group may be eluted using acetonitrile, whereas molecules having a SSH-linker may be eluted with 10% glacial acetic acid at room temperature, respectively. In other examples, the pooled oligonucleotides may be directly used for assembly without prior elution from the support. Assembly may be conducted by PCR-based methods as described elsewhere herein.

When starting from different oligonucleotide samples wherein a first sample comprises a first oligonucleotide mixture of full-length and truncated molecules representing a first portion of a desired nucleic acid sequence to be assembled and a second sample comprises a second oligonucleotide mixture of full-length and truncated molecules representing a second portion of the desired nuclei acid sequence etc.), each oligonucleotide mixture may be provided at excess concentration (*i.e.,* at a concentration that exceeds the concentration required for subsequent assembly into the desired nucleic acid sequence). For purposes of illustration, oligonucleotides may be synthesized at concentrations of about 50 to 60 µM, whereas the concentration of individual oligonucleotides required for subsequent assembly may be as low as 0.15 µM. Thus, when pooling oligonucleotides provided at excess concentration from the different samples, those oligonucleotides comprising a terminal modification such as a trityl group may be captured at similar or substantially equal (*i.e.,* equimolar) amounts from each sample by selecting an appropriate amount of hydrophobic material (such as column or bead material) with a defined or limited binding capacity for the terminal modification. In some instances, each oligonucleotide comprising a terminal modification may be pooled from a given sample at an amount of between about 0.01 to about 0.1 µM, or between about 0.05 and about 0.2 µM. In some instances, the amount or concentration of oligonucleotides pooled from a first mixture may not deviate by more than 20%, more than 10%, more than 5%, more than 2% or more than 1% from the amount or concentration pooled from a second mixture.

To allow for pooling of equimolar amounts of full-length oligonucleotides from different samples, the initial concentration of oligonucleotides in each sample should be equal or similar. However, there may be instances where a higher amount of a first type of oligonucleotide is required and a lower amount of a second type of oligonucleotide. For example, where the first type of oligonucleotide represents a terminal region of a nucleic acid molecule to be assembled and the second type of oligonucleotide represents a central region of the nucleic acid molecule to be assembled, an assembly strategy may require a higher amount of the terminal oligonucleotide to be efficient. In such case, the first type of oligonucleotide may be provided at a higher concentration in the initial sample than the second type of oligonucleotide such that the total amount of pooled oligonucleotides reflects the difference in initial concentration between the first and second types of oligonucleotide.

By positive selection of a limited amount of oligonucleotides carrying the modification, excess oligonucleotides and truncated molecules can be removed thereby increasing the reliability of subsequent assembly workflows and the correctness of assembled molecules.

The positive selection of oligonucleotides comprising a terminal modification may be supported by a precedent step that actively eliminates truncated oligonucleotides not comprising such terminal modification to further enrich full-length molecules in the mixture for subsequent pooling. This may be achieved for example, by digesting truncated oligonucleotides by means of a nuclease activity as described in U.S. Patent No. 8,728,767. In some examples, such digestion may take place while the oligonucleotides are still bound to a solid support. In other examples the digestion may be practiced on oligonucleotides in solution. In some instances, an exonuclease having 5'-3' activity may be added to the mixture of full-length and truncated oligonucleotides to digest away truncated oligonucleotides that do not comprise a 5' terminal modification. Enzymes with 5'-3'exonuclease activity digesting single-stranded DNA substrates include, *e.g*., RecJ_{f}, or T5 exonuclease. Thus, in some examples the improved workflow comprises a step of eliminating truncated oligonucleotides by exonuclease digestion followed by active pooling of full-length oligonucleotides with a terminal modification. To allow exclusive elimination of truncated oligonucleotides it is essential that the 5' terminal modification confers exonuclease resistance to the full-length oligonucleotide. Thus, in some examples, the selected 5' terminal modification serves to (i) protect full-length oligonucleotides from exonuclease cleavage and (ii) allows for subsequent pooling via specific interaction. In other examples, the selected 5' terminal modification (iii) does not interfere with subsequent PCR assembly of pooled oligonucleotides.

### Sorting and Assembly of Nucleic Acid Molecules

Next generation gene synthesis techniques benefit from miniaturization of oligonucleotide synthesis and the ability to perform successive reaction steps. Further, reagent usage may be decreased, which currently contributes significantly to costs, by several orders of magnitude as compared to standard controlled pore glass (CPG) bead based oligonucleotide synthesis methods.

Microarray or chip synthesized oligonucleotides are attractive for nucleic acid synthesis because of low cost and high throughout. Some chip-based oligonucleotide synthesis platforms allow for the synthesis of up to one million 100mers on a single glass slide. Further, assembly reaction of a typical nucleic acid molecule (*e.g.,* a gene) generally requires a mix of about 20 to 60 (*e.g.,* about 40) oligonucleotides. Thus, assuming only one copy of each 100mer is produced, such slide based oligonucleotide synthesis can be used to generate enough different oligonucleotides for the synthesis of 25,000 nucleic acid molecules.

An issue arises with chip- or array-based oligonucleotide synthesis platforms in that, typically, the synthesized oligonucleotides are released simultaneously from the chip. This results in a highly complex mixture of oligonucleotides that are assembly components for multiple, different nucleic acid fragments or subfragments. Highly complex mixtures of oligonucleotides often lead to failed assembly reactions due to unintended hybridization of the nucleic acid molecules intended to form a single assembly product. It has been shown that pools above a certain complexity cannot be efficiently assembled without problems (Borovkov et al., Nucleic Acid Res., 38:e108 (2010)). This often limits the number of different oligonucleotides that can be synthesized on a microarray or microchip in parallel and can limit the usefulness of microarray oligonucleotide synthesis platforms.

One approach for addressing the complexity issue in nucleic acid assembly is to release and/or collect oligonucleotides that are components of a limited number of assembly products (*e.g*., from about 1 to about 20, from about 2 to about 20, from about 3 to about 20, from about 4 to about 20, from about 1 to about 3, from about 1 to about 5, from about 1 to about 10, etc. assembly products) to keep oligonucleotide complexity down in assembly mixtures.

When a large number of oligonucleotides are produced, for example, on a single surface, oligonucleotides may be released by methods such as through the cleavage of a linker. Such linkers may be cleaved, for example, photochemically with a laser, electrochemically, or micromechanically. A defined number of oligonucleotides may also be selectively released by amplification off the synthesis support, *e.g.*, by using primers that specifically bind to primer binding sites included in some or all of the oligonucleotides. In instances where oligonucleotides are synthesized on "loose" supports or on a chip-based platform on beads within wells (as described, *e.g.*, in U.S. Pat. Publ. No. 2016/0186166 A1), a selected number of beads carrying oligonucleotides comprising a pre-defined sequence may be selectively released, *e.g*., using a micropipette, by suction, magnetic attraction or by gas bubble displacement, as described in U.S. Pat. Publ. No. 2016/0186166 A1. For example, in a first step, all beads carrying oligonucleotides that belong to a first assembly product, may be simultaneously released and pooled into a first vessels (such as a well of a multiwell plate). In a second step, all beads carrying oligonucleotides that belong to a second assembly product, may be simultaneously released and pooled into a second vessel or well of a multiwell plate. Although this process works well, it requires time as all oligonucleotides belonging to a defined assembly product must be pooled. Furthermore, where bead-bound oligonucleotides are pooled from a microchip (*e.g*., by gas bubble displacement), the reliability of pooling correct sub-sets at sufficient quantity may decrease with each pooling steps.

The efficiency of this process may be improved in a number of ways that begin with the generation of oligonucleotide pools for the formation of more than one assembly product (a subfragment) in a single reaction vessel (*e.g.,* a tube, a well of a microwell plate, etc.). For example, the oligonucleotide pool may contain oligonucleotides for the formation of multiple subfragments but only a sub-set of these subfragments may be generated. One way of doing this is illustrated in FIG. 3.

The left side of FIG. 3, Tube A, shows a schematic representation of a workflow for the release of oligonucleotides from beads for the formation of a pool, where the oligonucleotides are components of a single subfragment, as well as the generation of the single subfragment by PCR. The right side of FIG. 3, Tube B, shows a schematic representation of a workflow for the release of oligonucleotides from beads for the formation of a pool, where the oligonucleotides are components of four subfragments, as well as the generation of the four subfragments by PCR. This allows for the generation of four nucleic acid molecules (having different sequences) in a single vessel and through the same PCR cycling processes. Such processes allow for the collection of oligonucleotides that are components of more than one nucleic acid molecule. This improves the efficiency of nucleic acid molecule assembly in that more than one nucleic acid molecule may be produced without sorting of oligonucleotide components. Workflows such as those shown in FIG. 3 may be combined with the terminal modification (*e.g.,* DMT)-based selective retrieval of full-length oligonucleotides described above or size exclusion purification. Such processes can significantly enhance the quality of the oligonucleotides and the reliability of subsequent assembly reactions.

In some instances, the number of nucleic acid molecules represented by oligonucleotides in a pool may be so large that assembly of all of the nucleic acid molecules may not occur efficiently. In certain examples it may be desired to reduce the complexity of an oligonucleotide pool to a level that allows for simultaneous assembly of multiple subfragments in a single reaction but is sufficiently low to guarantee efficient assembly without cross-hybridization events between oligonucleotides belonging to different subfragments.

In some instances two or more subfragments may be assembled in wells of a microwell plate. In some instances, two or more subfragments may be assembled in a reaction volume of between about 0.1 µl and about 1,000 µl or between about 0.5 µl and about 50 µl, such as for example a 1-µl reaction volume. In some instances, nucleic acids may be assembled according to methods disclosed herein in a reaction volume smaller than 1 µl or smaller than 0.1 µl.

In some instances the pooled oligonucleotides may be designed such that all subfragments assembled in the same reaction (e.g. in the same continuous liquid phase) comprise substantially identical 5'- and/or 3'-ends, which may contain functional elements such as universal primer binding sites and/or restriction enzyme cleavage sites or homologous regions, allowing for simultaneous amplification with a universal pair of primers in a subsequent PCR reaction and/or simultaneous processing in a downstream assembly or cloning step. Thus, in some instances an oligonucleotide belonging to a first subfragment may have the same or substantially the same 5'end as an oligonucleotide belonging to a different second subfragment. Likewise, in some instances an oligonucleotide belonging to a first subfragment may have the same or substantially the same 3'end as an oligonucleotide belonging to a different second subfragment.

Once a subset of oligonucleotides is released from a synthesis array or chip, the released oligonucleotides may be collected and subjected to one or more assembly reactions to generate a single assembly product or a known number of assembly products. Oligonucleotides may then be released from additional regions of the chip for assembly of additional nucleic acid molecules.

In some circumstances, a stepwise release of oligonucleotides from a microarray or chip-based platform may be limited in terms of miniaturization, or still be too time consuming to execute, and may therefore be of limited use in high throughput applications. Further, some chip synthesis platforms do not allow for selective release of oligonucleotides.

The disclosure therefore also relates to compositions and methods that address these limitations and circumvent the necessity of selective oligonucleotide release. Methods of the disclosure use collections of oligonucleotides generated by different synthesis reactions. In some aspects, the disclosure relates to the inherent feature of nucleic acid molecules for specific hybridization to concentrate and/or sort oligonucleotides intended to be combined to form an assembled target nucleic acid (*e.g.,* a desired gene). The disclosure thus allows, in part, for efficient high-throughput nucleic acid synthesis and assembly by massive parallelization.

In some aspects, the disclosure is exemplified by FIGs. 4, 5, and 6. Nucleic acid molecules used in some aspects of the disclosure are represented in FIG. 4. FIG. 4B shows a nucleic acid molecule that contains a tag region on the 5' end, a spacer, and an assembly region on the 3' end. The tag region may be a defined region of an oligonucleotide used in an assembly reaction and may be on the 5' end or the 3' end, and in some cases, tags may be present on each end. In many instances, the tag will be located on the 5' end because enzymatic based nucleic acid replication typically proceeds in a 5' to 3' direction. Tags suitable for use with methods disclosed herein are set out elsewhere herein. In various examples of the disclosure, tags may be from about 5 nucleotides to about 30 nucleotides (*e.g.,* from about 5 nucleotides to about 30 nucleotides, from about 7 nucleotides to about 30 nucleotides, from about 10 nucleotides to about 30 nucleotides, from about 7 nucleotides to about 20 nucleotides, from about 10 nucleotides to about 20 nucleotides, from about 7 nucleotides to about 16 nucleotides, etc.) in length. Tags will generally be of sufficient length to allow for sequence specific hybridization of tagged oligonucleotides to cognate complementary oligonucleotides under conditions that allow for "sorting" of the tagged oligonucleotides as shown in FIG. 5.

In many instances, a spacer will be located between the tag region and the assembly region. Further, in many instances, the spacer will be or will contain a chemical group that terminates polymerase based nucleic acid replication (*i.e.* a blocking moiety). Spacers may therefore terminate chain elongation during polymerase-based amplification such that the tag will not be replicated in an oligonucleotide assembly reaction. The blocking moiety of the spacer, however, is so chosen that it does not prevent hybridization of the adjacent assembly region of the oligonucleotide to the assembly region of another oligonucleotide. One example of a chemical group that may serve as blocking moiety is shown in FIG. 4C.

Suitable blocking moieties of spacers include among others, bulky base analogs, abasic sites, chemical modifications such as hexaethylene glycol (HEG) dideoxynucleotides, 9-O-Dimethoxytrityl-triethylene glycol, 3-(4,4'-Dimethoxytrityloxy)propanol, or 5'-O-Dimethoxytrityl-1',2'-Dideoxyribose or any non-natural or modified nucleotide which prevents read-through of or elongation of a nucleic acid strand by the polymerase including DNA analogues such as peptide nucleic acid (PNA). Spacers will typically be at least 90% (*e.g.,* at least 93%, at least 95%, at least 97%, at least 98%, at least 99%, or 100%) efficient in stopping polymerase mediated chain extension. In some examples, a spacer may comprise two or more blocking moieties of any one of the groups mentioned above or combinations thereof.

The assembly region of an oligonucleotide will typically have the nucleotide sequence of a segment of a desired nucleic acid molecule intended for assembly. Where a population of single-stranded oligonucleotides is provided to assemble a double-stranded target nucleic acid intended to have a predefined sequence, each of the oligonucleotides typically comprises an assembly region that represents a portion of said target nucleic acid, wherein each assembly region has a sequence region that is capable of hybridizing to at least one other oligonucleotide in the population. The sequence region that is capable of hybridizing to other oligonucleotides may represent 100% of the assembly region, e.g. where the entire assembly region of a sense strand oligonucleotide hybridizes to two oligonucleotides of the antisense strand in a nicked conformation (as described below and exemplified in FIG. 8A). Alternatively, the sequence region that is capable of hybridizing to one or more other oligonucleotides may represent less than 100% of the assembly region, such as e.g. 75%, 70%., 65%, 60%, 50%, 45%, 40%, 30% or 20% of the assembly region, e.g. where a first portion of the assembly region of a sense oligonucleotide hybridizes to a first antisense oligonucleotide and a second portion of the assembly region of the same sense oligonucleotide hybridizes to a second antisense oligonucleotide (as described below and exemplified in FIG. 8B and C). Thus, in some instances an assembly region may have two separate regions capable of hybridizing with different oligonucleotides. Further, assembly regions will typically be from about 15 to about 60 nucleotides (*e.g.,* from about 15 to about 60 nucleotides, from about 20 to about 60 nucleotides, from about 25 to about 60 nucleotides, from about 30 to about 60 nucleotides, from about 15 to about 50 nucleotides, from about 20 to about 50 nucleotides, from about 25 to about 50 nucleotides, from about 30 to about 50 nucleotides, etc.) in length.

As suggested above, nucleic acid molecules similar to the representation in FIG. 4B may be synthesized in a manner that results in them being in a mixed population of other nucleic acid molecules. In some instances, a large number of nucleic acid molecules may be present in a mixture. Further, this mixture may contain individual components that may be used to assemble a number of different nucleic acid molecule assembly products.

All oligonucleotides belonging to a final assembly product may contain, not only sequences necessary for the assembly of the final product molecule (i.e. an assembly region), but also a shared hybridization tag. This shared hybridization tag may be used to concentrate oligonucleotides to a location (*e.g.,* a bead, a spot, a well, etc.) containing a "capture" oligonucleotide, wherein the "capture" oligonucleotide comprises at least a portion of the reverse complement sequence of the hybridization tag (see FIG. 5).

Hybridization tag recognition is responsive enough such that thousands of different tags may be used in a single mix and separated by sequence specific hybridization to "capture" oligonucleotides, each corresponding to a different product molecule or a collection of product molecules for assembly. However, the number of tags may often be selected with regard to the number of pools of sorted oligonucleotides contributing to specific assembly reactions and/or with regard to the platform used for the sorting. For example, in some instances, 12 or 96 or 256 or 1,000 or 10,000 different tags may be used.

Capture oligonucleotides may be immobilized on a support such as a bead, spot on a chip, array, well of a multiwell plate etc. In some examples, a capture oligonucleotide may be linked to or synthesized with a biotin moiety at the 3' or 5' end and may be immobilized on a surface (*e.g.* a bead) coated with streptavidin according to standard protocols. In some examples, a capture oligonucleotide may carry an amino group and may be immobilized on a support comprising carboxylic acid groups thereon according to standard coupling procedures. In some examples, a capture oligonucleotide may be immobilized using click chemistry. In some examples, a capture oligonucleotide may be immobilized on a support via an adapter having additional functional features such as a restriction enzyme cleavage site as described elsewhere herein.

The sequence of the tag, however, will typically not share significant sequence identity/complementarity to the nucleic acid molecule to be synthesized, therefore, might possibly interfere with assembly and/or final sequence integrity of the synthetic nucleic acid molecule.

In some instances, a tag library may be designed such that each tag is sufficiently different from another tag in the library so that the library of tags can be used to specifically sort a plurality of different target sequences. One exemplary approach to design a tag library may rely on a method comprising the following steps: (a) designing, *in silico* a set of candidate tag sequences having desired sequence properties (*e.g.,* a pre-determined GC content, length, melting temperature, or other properties known to be desirable for hybridization function); (b) picking, *in silico* one candidate tag of the set of candidate tag sequences and (c) removing, *in silico* all candidate tag sequences from the set of candidate tag sequences that are "too similar" to the picked candidate tag sequence, where similarity is determined by identifying the number of deletions, insertions or mutations necessary to transform one candidate tag sequence to another candidate tag sequence, a parameter known as the "edit distance" or "Levenshtein distance" between two sequences (*see, e.g., "*Introduction to Algorithms", Third Edition (2009) by Cormen et al., The MIT Press Cambridge, Massachusetts London, England). Alternatively, "Hamming distance" (as described, *e.g.,* in Bystrykhor "Generalized DNA Tag Design Based on Hamming Codes", PLoS One, 2012, Vol. 7, issue 5, e36852) or other appropriate distances may be used to remove tag sequences of high similarity. The method may further comprise the step d) of iteratively picking further candidate tag sequences until no candidate sequences of high similarity remain. The resulting pool of picked tag sequences forms the tag library with tags sufficiently different from each other. In some examples, the candidate tag picked in step (b) may be chosen randomly or by using a "greedy" approach such that a plurality of tag libraries with varying tag numbers may be obtained from which the largest one may be chosen. A similar approach may be used to design and select suitable capture oligonucleotides as described elsewhere herein.

In some instances, each set of oligonucleotides that together represent an assembly product within a mixed population of oligonucleotides may receive an individual tag. This may be achieved by using a tag library with a diversity that exceeds the diversity of oligonucleotide assembly sets in the pool. For example, depending on the complexity of the oligonucleotide pool, the diversity of the tag library may be from about 5 to about 50, from about 10 to about 100, from about 50 to about 1,000 or from about 1,000 to about 10,000 or from about 50,000 to about 100,000. In some instances the diversity of a tag library may reflect the diversity of a library of capture oligonucleotides as described elsewhere herein.

To detach functionality of the assembly region of an oligonucleotide and its tag, a spacer may be added between both oligonucleotide parts. Oligonucleotide synthesis not only allows for the incorporation of A,C,G,T phosphoramidites but also a variety of other functional building blocks, either at the 5' or 3' end or - as in this application - internally. Thus, a spacer may be incorporated at a desired position during chemical synthesis of an oligonucleotide.

An advantageous spacer would be one that can be used to reliably separate the tag from the assembly region under desired conditions (*e.g.,* photocleavable). However, another method employs asymmetric PCR which does not require any extra step but the assembly reaction itself. By separating the tag from the assembly region of the oligonucleotide with a building block that terminates elongation during PCR assembly (*e.g.,* hexaethylene glycol (HEG)), reactions can be performed such that only assembly regions of oligonucleotides designed participate in assembly, elongation and gene synthesis reactions are amplified. Thus, asymmetric PCR may be used to generate untagged oligonucleotides from tagged oligonucleotides.

To achieve this, all oligonucleotides, belonging to a certain fragment assembly reaction, are designed to not only contain the sequences necessary for the assembly and composition of the synthetic gene, but are further provided with a shared 5' hybridization tag, to be used to concentrate them on a bead or spot or well or matrix that is coated with a "capture" oligonucleotide comprising the reverse complement sequence of the hybridization tag.

In oligonucleotide synthesis for example HEG can be internally added as a "spacer". This group acts as an elongation terminator if the oligonucleotide is used as a template for PCR or other enzymatic polymerization. Examples of asymmetric PCR methods using spacer- modified oligonucleotides are set out, *e.g.,* in Liang et al., "Comparison of the methods for generating single-stranded DNA in SELEX", Analyst. 2015, 140(10):3439-44.

As illustrated by the example in FIG. 6, the first cycle round of hybridization and elongation already creates extended oligonucleotides with termini that do not reach into the tag region of their template counterparts. This process continues until full-length fragments emerge which can then be either amplified by terminal primers (which can be universal primers allowing cloning via seamless assembly or restriction digestion/ligation) or seamlessly integrated into a linear polynucleotide such as a linearized vector with complementary ends. In both cases, the complete reactions of primer extension and full-length amplification can be accomplished in a single tube and cycle program. The Sequence Elongation and Ligation protocol discussed below may be used for integration of assembled nucleic acid molecules into a vector backbone (or other linear polynucleotide) in the same reaction or through downstream reactions, followed by, for example, direct transformation into a host cell (*e.g., E. coli*).

Specific examples of disclosed methods may involve the use of hardware and protocols that employ pipetting aids, as well as automation for spotting and/or coating substrates or slides with given sets of oligonucleotides (*e.g.* capture oligonucleotides). In some instances, the target substrate is selected to be a microwell plate or chip suitable to hold individual PCR reactions and to be fit into an apt PCR cycler. To further leverage parallel synthesis capacity, loose synthesis supports (*e.g.,* CPG resin or beads) may be coated with distinct capture oligonucleotides. Capture oligonucleotides may either be immobilized on a support or directly synthesized 3'->5' on the support and may be deprotected without cleaving the oligonucleotides from the supports. To synthesize 10,000 nucleic acid molecules in parallel, 10,000 such "capture" support syntheses are necessary. To reduce the number of separate syntheses, the supports could be subjected to a "split-and-mix" synthesis as described *e.g.,* in U.S. patent No. 5,789,577. For example, 6 splits produce 128 different combinations, thereby reducing the number of oligonucleotide syntheses to 78 to yield a variety of 78 x 128 = 9,984). Still, a single support holds a distinct sequence and the plurality of capture sequences is known. Supports may optionally be diluted and mixed with tagged assembly oligonucleotides. In some instances oligonucleotides may be mixed with supports with a - fold overrepresentation of necessary supports (e.g. 10-fold), to ensure full coverage of each capture oligonucleotide within the plurality of supports. Tagged assembly oligonucleotides belonging to the same assembly product concentrate and sort on their supports by specific hybridization with respective capture oligonucleotides. Sorted oligonucleotides may then be washed (e.g. to remove unbound oligonucleotides), and may optionally be released from the support prior to incubation with a suitable assembly PCR reaction mix.

In some instances, at least a portion of the sorted oligonucleotides may be released from the support prior to assembly by cleavage with an endonuclease or restriction enzyme (*e.g.* by cleavage of a cleavable moiety or restriction enzyme cleavage site contained in the capture and/or assembly oligonucleotides). However, in many instances cleavage may not be required to release the oligonucleotides into solution. In many instances, a heat denaturation step prior to or during a PCR assembly reaction will be sufficient to release at least a portion of the sorted oligonucleotides into solution to initiate the assembly reaction.

In one example, supports carrying assembly oligonucleotides may be dispensed in a water-in-oil emulsion to aim for an average of not more than one support per drop. Emulsion is then cycled under assembly conditions as described elsewhere herein (*e.g.,* using a Sequence Elongation and Ligation protocol) to produce a distinct linear or circular full-length assembly product, ready for cloning or direct transformation. After cycling, emulsion could, for example, be broken or sorted by FACS, then amplified, *e.g*., by PCR or rolling circle amplification and sequenced to determine which reaction holds which sequence. Emulsion could also be pooled after cycling, transformed in *E. coli* and colonies could be subjected to sequencing to allocate colony ID with a discrete tag sequence derived from the assembled oligonucleotides, as described elsewhere herein.

One way of concentrating assembly oligonucleotides in emulsions is by using a method referred to as "DropSynth" described by Plesa et al. 2018 (Science 359, 343-347). In the DropSynth method tagged beads are used to pull down PCR-amplified assembly oligonucleotides which are required to assemble a particular assembly product. The beads are then emulsified to isolate each bead with concentrated assembly oligonucleotides into a droplet. Unbound oligonucleotides are washed away and the beads are mixed with PCR reagents, a restriction enzyme and oil to form a water-in-oil emulsion, which is placed into a thermocycler where the restriction enzyme displaces the oligonucleotides carrying restriction enzyme cleavage sites from the bead and the gene assembly reaction takes place inside the droplets. Upon completion, the emulsion is broken up and the assembly products are recovered and PCR-amplified for downstream applications.

The method illustrated by FIGs. 4-6 has certain advantages over the DropSynth method described above. The DropSynth protocol requires the initial amplification of the single-stranded oligonucleotides into double-stranded nucleic acid fragments and nicking the double-stranded fragments to set free the single-stranded tag portion of the oligonucleotide. Further, the processed double-stranded fragment needs to be ligated to the immobilized capture oligonucleotide after hybridization and then needs to be cleaved enzymatically from the tag portion of the oligonucleotide prior their assembly into larger double-stranded nucleic acid molecules. These steps comprise an intrinsic efficiency and may lead to incomplete capture and loss of oligonucleotides. It may also lead to incomplete cleavage and unwanted inclusion of the tag sequence in the assembled product. Also, the overall multiplex assembly of the partially cleaved oligonucleotides can be less efficient due to wrong or hampered hybridization of uncleaved molecules. Further, the cleavage site used must be avoided in the assembly region of the oligonucleotides, limiting the design flexibility of the nucleic acid molecules to be built in the workflow. The improved protocol proposed herein, on the other hand, decouples the functional portions of the oligonucleotides by a non-template spacer molecule, securing the consistent termination of each elongation in the oligonucleotide assembly at the end of the assembly region of the oligonucleotides. No avoidance of restriction sites in the assembly regions is necessary and the restriction cleavage step can be omitted. Therefore the efficiency of such cleavage step is irrelevant and there is no risk of faulty incorporation of tag sequences into the assembled nucleic acid molecules.

In the practice of methods of the disclosure, populations of oligonucleotides may be generated where each population contains oligonucleotides designed to be sub-components of multiple final assembly products. These oligonucleotides may be sorted as described above to decrease the complexity of assembly reaction mixtures with the resulting oligonucleotide mixture to form a sorted oligonucleotide population. As examples, the number of oligonucleotides in the reaction mixture may be designed to be sub-components of from about 5 to about 500 (*e.g.,* from about 10 to about 500, from about 20 to about 500, from about 30 to about 500, from about 50 to about 500, from about 75 to about 500, from about 100 to about 500, from about 150 to about 500, from about 200 to about 500, from about 75 to about 250, etc.) assembly products. Further, the sorting of oligonucleotides may result in a reduction of the number of different sub-components present where the sorted oligonucleotide population contains oligonucleotides that are intended to form either a fixed number of final assembly products or a certain level of decrease in reaction mixture complexity as described above. By way of example, the number of assembly products represented in individual sorted oligonucleotide populations may be from about 1 to about 40 (*e.g.,* from about 1 to about 40, from about 1 to about 30, from about 1 to about 20, from about 1 to about 10, from about 1 to about 5, from about 1 to about 2, from about 2 to about 40, from about 2 to about 4, from about 5 to about 40, from about 5 to about 20, from about 5 to about 10, from about 8 to about 30, etc.).

The complexity of a population of oligonucleotides is, in part, determined by the number of different oligonucleotides present. In some instances, the number of oligonucleotides present that are designed to have different nucleotide sequences, may be from about 2,000 to about 20,000 (*e.g.,* from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, from about 2,000 to about 20,000, etc.).

Further, populations of oligonucleotides may be sorted into (i) specified numbers of sorted oligonucleotide populations (*e.g.,* from about 1 to about 300, from about 5 to about 300, from about 10 to about 100, from about 1 to about 10, from about 1 to about 100, from about 10 to about 75, from about 20 to about 300, from about 25 to about 400, etc.) (ii) sorted oligonucleotide populations that contain specific numbers of different oligonucleotides (*e.g.,* from about 100 to about 2,000, from about 200 to about 2,000, from about 500 to about 2,000, from about 750 to about 2,000, from about 750 to about 3,000, etc.), (iii) sorted oligonucleotide populations that contain oligonucleotides designed for the assembly of a specified number of product nucleic acid molecules.

To exemplify some of the above, assume that a populations of oligonucleotides contains 15,000 oligonucleotides designed to have different sequences (*e.g.,* 15,000 oligonucleotides generated separately using chemical synthesis). Further assume that these 15,000 oligonucleotides are designed to be sub-components of 75 different final product nucleic acid molecules, wherein each final product nucleic acid molecule is composed of, on average, 200 individual oligonucleotides. Also assume that three final product nucleic acid molecules are to be generated at each assembly reaction area. In such an instance, 25 different tags could be used to generate sorted oligonucleotide populations at 25 different assembly reaction areas.

In some instances, the disclosure is, in part, directed to the generation of a large population of tagged oligonucleotides, sorting of the large population of tagged oligonucleotides by use of one or more tags to obtain a reduced group of oligonucleotides designed to be sub-components or a fixed number (*e.g.,* from about one to about ten) of assembled nucleic acid molecules, and assembly of the oligonucleotides to generate assembled nucleic acid molecules that do not contain nucleic acid associated with the one or more tags.

Following sorting and/or pooling, the oligonucleotides will typically be assembled into larger nucleic acid molecules in a step-wise manner and optionally, amplified.

In some aspects, assembled nucleic acid molecule length will vary from about 20 base pairs to about 10,000 base pairs, from about 100 base pairs to about 5,000 base pairs, from about 150 base pairs to about 5,000 base pairs, from about 200 base pairs to about 5,000 base pairs, from about 250 base pairs to about 5,000 base pairs, from about 300 base pairs to about 5,000 base pairs, from about 350 base pairs to about 5,000 base pairs, from about 400 base pairs to about 5,000 base pairs, from about 500 base pairs to about 5,000 base pairs, from about 700 base pairs to about 5,000 base pairs, from about 800 base pairs to about 5,000 base pairs, from about 1,000 base pairs to about 5,000 base pairs, from about 100 base pairs to about 4,000 base pairs, from about 150 base pairs to about 4,000 base pairs, from about 200 base pairs to about 4,000 base pairs, from about 300 base pairs to about 4,000 base pairs, from about 500 base pairs to about 4,000 base pairs, from about 50 base pairs to about 3,000 base pairs, from about 100 base pairs to about 3,000 base pairs, from about 200 base pairs to about 3,000 base pairs, from about 250 base pairs to about 3,000 base pairs, from about 300 base pairs to about 3,000 base pairs, from about 400 base pairs to about 3,000 base pairs, from about 600 base pairs to about 3,000 base pairs, from about 800 base pairs to about 3,000 base pairs, from about 100 base pairs to about 2,000 base pairs, from about 200 base pairs to about 2,000 base pairs, from about 300 base pairs to about 1,500 base pairs, etc.

Any number of methods may be used for nucleic acid amplification and assembly. One exemplary method is described in Yang et al., Nucleic Acids Research 21:1889-1893 (1993) and U.S. Patent No. 5,580,759. In the process described in Yang *et al.,* a linear vector is mixed with double-stranded nucleic acid molecules which share sequence homology at the termini. An enzyme with exonuclease activity (*i.e.,* T4 DNA polymerase, T5 exonuclease, T7 exonuclease, etc.) is added which generates single-stranded overhangs of all termini present in the mixture. The nucleic acid molecules having single stranded overhangs are then annealed and incubated with a DNA polymerase and deoxynucleotide triphosphates under condition which allow for the filling in of single-stranded gaps. Nicks in the resulting nucleic acid molecules may be repaired by introduction of the molecule into a cell or by the addition of ligase. Of course, depending on the application and workflow, the vector may be omitted. Further, the resulting nucleic acid molecules, or sub-portions thereof, may be amplified by polymerase chain reaction.

Other methods of nucleic acid assembly include those described in U.S. Patent Publication Nos. 2010/0062495 A1; 2007/0292954 A1; 2003/0152984 AA; and 2006/0115850 AA and in U.S. Patents Nos. 6,083,726; 6,110,668; 5,624,827; 6,521,427; 5,869,644; and 6,495,318.

In some instances, standard ligase based joining of partially and fully assembled nucleic acid molecules may be employed. For example, assembled nucleic acid molecule may be generated with restriction enzyme sites near their termini. These nucleic acid molecules may then be treated with one of more suitably restrictions enzymes to generate, for example, either one or two "sticky ends". These sticky end molecules may then be introduced into a vector by standard restriction enzyme-ligase methods. In instances where the inert nucleic acid molecules have only one sticky end, ligases may be used for blunt end ligation of the "non-sticky" terminus.

One method for assembling nucleic acid molecules is depicted in FIG. 2 and involves starting with oligonucleotides or sub fragments that will generally contain sequences that are overlapping at their termini which are "stitched" together via these complementary sequence regions using PCR. In some examples, the overlaps are approximately 10 base pairs; in other examples, the overlaps may be 15, 25, 30, 50, 60, 70, 80 or 100 base pairs, etc. (*e.g.,* from about 10 to about 120, from about 15 to about 120, from about 20 to about 120, from about 25 to about 120, from about 30 to about 120, from about 40 to about 120, from about 10 to about 40, from about 15 to about 50, from about 40 to about 80, from about 60 to about 90, from about 20 to about 50, from about 15 to about 35,etc. base pairs). In order to avoid mis-assembly, individual overlaps will typically not be duplicated or closely matched amongst the subfragments. Since hybridization does not require 100% sequence identity between the participating nucleic acid molecules or regions, each terminus should be sufficiently different to prevent mis-assembly. Further, termini intended to undergo homologous recombination with each other should share at least 90%, 93%, 95%, or 98% sequence identity.

Further, multiple cycles of polymerase chain reactions may be used to generate successively larger nucleic acid molecules. In many instances, stitched oligonucleotides will be chemically synthesized and will be less than 100 nucleotides in length (*e.g.,* from about 40 to 100, from about 50 to 100, from about 60 to 100, from about 40 to 90, from about 40 to 80, from about 40 to 75, from about 50 to 85, etc. nucleotides). Primers may also be used which contain restriction sites for instances where insertion into a cloning vector is desired. Where desirable, assembled nucleic acid molecules may be directly inserted into vectors and host cells. PCR-based insertion into a target vector may be appropriate when the desired construct is fairly small (*e.g.,* less than 5 kilobases).

In many instances, a typical nucleic acid assembly protocol may comprise a combination of the above described techniques, such as, *e.g.,* a combination of exonuclease-mediated generation of single-stranded overhangs followed by PCR-based assembly (hereinafter referred to as "standard workflow"). In some examples, such standard workflow may comprise at least the following steps: (i) synthesizing single-stranded oligonucleotides together comprising a sequence of a desired target nucleic acid having a predefined sequence, wherein each oligonucleotide has a sequence region that is complementary to a sequence region in another oligonucleotide and capable of hybridizing thereto, (ii) hybridizing the oligonucleotides via their complementary sequence regions and elongating the oligonucleotides in an overlap extension PCR reaction ("1st PCR") to assemble one or more double-stranded nucleic acid molecules, (iii) amplifying the assembled nucleic acid molecules in the presence of terminal primers ("2nd PCR"), (iv) purifying the amplified nucleic acid molecules, (v) generating single-stranded overhangs at the terminal ends of the amplified one or more nucleic acid molecules and optionally, generating single-stranded overhangs at the terminal ends of a linearized target vector for subsequent cloning (*e.g.,* by treatment of the fragments with one or more restriction endonucleases and/or an exonuclease), (vi) inserting the one or more nucleic acid molecules into the target vector via the complementary single-stranded overhangs, optionally followed by a ligation step, and (vii) transforming host cells (such as, *e.g., E. coli*) with the assembled vector construct. In some examples, assembled nucleic acid molecules may be ligated *"in vivo"* by endogenous enzymatic activities of the transformed cell. For example, a gapped or nicked assembly product may be directly transformed into *E. coli* and gaps or nicks may be repaired by the *E. coli* endogenous repair machinery.

Such standard workflow which is represented in FIG. 7A by the basic steps of oligonucleotide synthesis, a first PCR to assemble the oligonucleotides, a second PCR to amplify the assembled product, followed by purification of the amplified product, treatment with a nuclease to generate single-stranded overlaps between the purified insert and a target vector, and insertion of the insert into the target vector followed by a transformation step., (a) requires a considerable amount of "hands-on" time, (b) requires several hours of overall incubation time, and (c) consumes large amounts of multiple reagents. Aspects described herein therefore seek to provide improved and reliable protocols that can be implemented in an automated assembly line and allow for faster and more cost-effective nucleic acid production.

In a first aspect, an improved assembly method comprises a combined Sequence Elongation and Ligation reaction, wherein steps (ii), (iii) and (vi) of the standard workflow described above are combined in a single ("one-pot") reaction, whereas other steps such as steps (iv) and (v) may be omitted. In particular, such methods comprise direct assembly of single-stranded overlapping oligonucleotides into a linear double-stranded polynucleotide target nucleic acid (such as a linearized target vector) via overlap extension PCR and amplification of the resulting fusion construct in a single step. According to some examples, no separate PCR reaction is required to generate double-stranded subfragments from the single-stranded oligonucleotides prior to vector insertion. Instead, the single-stranded oligonucleotides together comprising the sequence of the desired target nucleic acid can be directly used in the overlap extension reaction. After an initial denaturation step of the PCR reaction to separate at least the termini of the linear double-stranded polynucleotide or linearized vector, the single-stranded oligonucleotides are annealed via their complementary ends. Two of the oligonucleotides are designed to carry sequence homologies with the vector backbone allowing for hybridization with the ends of one of the denatured vector strands. The 3'ends of the annealed oligonucleotides and/or the 3' ends of the vector strands serve as primers for the synthesis of the complementary nucleic acid strand. The polymerase-mediated elongation stops when the 5' end of a hybridized oligonucleotide is encountered resulting in the production of a nicked circularized double-stranded nucleic acid molecule. The fused and amplified assembly products can be directly transformed into host cells without further purification. In some examples, no ligation step is performed prior to the transformation. The final ligation of the nicked fusion construct is achieved endogenously within the host cell.

Thus the present disclosure provides a method of assembling a double-stranded target nucleic acid intended to have a predefined sequence, the method comprising: (a) providing a population of single-stranded oligonucleotides, each of the oligonucleotides having an assembly region representing a portion of the target nucleic acid, wherein each assembly region has a sequence region that is capable of hybridizing to at least one other oligonucleotide in the population, (b) providing a linear double-stranded polynucleotide, each terminus of the linear double-stranded polynucleotide having a sequence region that is capable of hybridizing to at least one oligonucleotide of the population, (c) combining the population of single-stranded oligonucleotides with the linear double-stranded polynucleotide and a polymerase and dNTPs in a buffer to generate a reaction mixture, and (d) subjecting the reaction mixture to cycling conditions that allow for i. at least partial denaturation of the linear double-stranded polynucleotide to obtain single-stranded termini, ii. hybridization of the population of single-stranded oligonucleotides to one another and to the single-stranded termini of the polynucleotide in a pre-determined order, iii. polymerase-mediated extension of the free 3' ends of the hybridized oligonucleotides and the polynucleotide strands to generate a double-stranded circularized assembly product comprising a linear arrangement of assembly regions, and iv. amplification of the double-stranded circularized assembly product to generate an amplified double-stranded target nucleic acid intended to have a predefined sequence.

An exemplary Sequence Elongation and Ligation workflow is shown in FIG. 7B and represented by the following basic steps: oligonucleotide synthesis, a Sequence Elongation and Ligation PCR reaction combining the steps of oligonucleotide assembly/elongation and fusion into a linearized target vector, followed by a transformation step.

Based on the combination of oligonucleotide assembly and vector insertion, a Sequence Elongation and Ligation reaction can be performed in significantly shorter time and requires less hands-on time than the above standard protocol. For example, a Sequence Elongation and Ligation reaction may be performed in less than 3 hours with only 5 minutes hands-on time per 96 reactions. Furthermore, the single reaction step saves reagents, materials and lab device capacity and simplifies the automation of high-throughput polynucleotide production.

Another advantage of the Sequence Elongation and Ligation workflow is the reduction of PCR steps required to synthesize the full-length nucleic acid molecule. Whereas a standard workflow according to FIG. 7A requires at least two PCR steps for assembly of the oligonucleotides and subsequent amplification of the assembled fragment, only one PCR reaction is needed in a Sequence Elongation and Ligation reaction to accomplish simultaneous assembly, elongation and fusion of a desired polynucleotide into a target vector. Without being bound by theory it is believed that less replication cycles limit the amount of errors introduced by the activity of the polymerase, as most commercially available polymerases such as, *e.g.,* Taq polymerase introduce errors at a rate of approximately 1-20 x 10⁻⁵ mutations/base pair per template duplication. The error rate may be further reduced to about 2-3 x 10⁻⁶ mutations/base pair per template duplication by using high fidelity polymerases (*see, e.g.,* McInerney et al., "Error Rate Comparison during Polymerase Chain Reaction by DNA Polymerase", Hindawi Publishing Corporation Molecular Biology International, Vol. 2014, Article ID 287430). High fidelity polymerases with proof-reading activity suitable for methods of the disclosure are disclosed elsewhere herein.

Table 1 shows an exemplary time schedule of a standard workflow compared to a Sequence Elongation and Ligation reaction.

| Table 1 | | |
|---|---|---|
| Standard workflow steps | standard workflow approx. time | Sequence Elongation and Ligation reaction |
| 1st PCR | 75 min | ~ 170 min |
| 2nd PCR | 70 min | |
| Purification | 15 min | |
| Nuclease treatment | 50 min | |
| Vector Insertion | 60 min | |
| *Total time* | *4.5 hours* | ~*3.0 hours* |

Another advantage of the combined reaction steps is that errors that may multiply with the sequence of separate steps are eliminated or reduced if only a single reaction step is required which increases the success rate of assembling a correct full-length polynucleotide. Thus, the Sequence Elongation and Ligation protocol increases the overall process stability, significantly reduces costs and provides increased throughput due to reduced device utilization and required work time.

The principle of a Sequence Elongation and Ligation reaction is illustrated in FIG. 8. In many instances, first and second sets of oligonucleotides are synthesized for subsequent fusion into a linearized or linear polynucleotide such as a target vector. The oligonucleotides may be synthesized by any means using, *e.g.*, microarray or microchip-based chemical synthesis as described above. Thus, in aspects of the disclosure the single-stranded oligonucleotides used in a Sequence Elongation and Ligation reaction are chemically synthesized nucleic acid molecules. In some examples the chemical synthesis may comprise an electrochemical or photochemical deprotection step. The first set of oligonucleotides may comprise or represent at least a portion of the first strand (forward or sense strand) of a double-stranded assembly product, whereas the second set of oligonucleotides may comprise or represent at least a portion of the second strand (reverse or antisense strand) of the double-stranded assembly product. Each member of the first set (referred to as forward oligonucleotides or "f-oligos") is at least partially complementary to at least one member of the second set (referred to as reverse oligonucleotides or "r-oligo") such that hybridization of members of the first and second sets of oligonucleotides in a predetermined order can be achieved. The first and second sets of oligonucleotides may be designed to hybridize in a "nicked", "single gapped" or "double gapped" conformation as illustrated in FIG. 8.

In a first example shown in FIG. 8A, the first and second sets of oligonucleotides are arranged in a "nicked" conformation when hybridized, wherein both sets represent the full-length sequences of first and second strands of the assembly product without leaving single-stranded gaps of one or more nucleotides between adjacent oligonucleotides of each set. In such confirmation the first and second sets may comprise oligonucleotides of similar or equal lengths. For example, the oligonucleotides representing both strands may have an average length of between about 25 to about 100 or between about 30 to about 50 or between about 36 to about 80 nucleotides.

In a second example shown in FIG. 8B, one set of oligonucleotides may only represent a portion (*i.e.*, less than the full-length) of a first strand, whereas the other set may represent the full-length sequence of the second strand resulting in a "single gapped" conformation when both sets are hybridized. Such "single gapped" confirmation is characterized by single-stranded gaps of one or more nucleotides between adjacent oligonucleotides of the same strand which are subsequently filled up by PCR-mediated elongation. A "single gapped" confirmation is achieved when oligonucleotides belonging to one set are shorter than the oligonucleotides belonging to the other set. In a single gapped scenario, the f-oligos may have an average length of between about 25 to about 100 or between about 30 to about 60 or between about 36 to about 80 or between about 36 to about 120 nucleotides, whereas the r-oligos may have an average length of between about 25 to about 100 or between about 30 to about 50 or between about 36 to about 44 nucleotides.

In a third example shown in FIG. 8C, both sets of oligonucleotides may only represent a portion (*i.e.*, less than the full-length) of each strand resulting in a "double gapped" confirmation with gaps of one or more nucleotides between adjacent oligonucleotides of both strands. In a double gapped confirmation the first and second sets may comprise oligonucleotides of similar or equal lengths which may in certain instances be shorter than the oligonucleotides used in a nicked confirmation described above. Double gapped confirmation may have advantages in certain instances: Double gapped confirmation results in a minimal amount of oligonucleotides required to cover a desired nucleic acid sequence to be assembled, less phosphoramidite couplings limiting the amount of errors introduced via inefficient coupling events, and less hybridization events required for successful nucleic acid assembly, possibly facilitating oligonucleotide design considerations. In some examples, a single or double gapped confirmation may be preferred over a nicked conformation, *e.g.*, where the production of shorter oligonucleotides for one or both strands reduces oligonucleotide synthesis time and reagent consumption. In other examples a nicked confirmation may be preferred, *e.g.*, where PCR amplification of two sets of complementary oligonucleotides may fail due to certain sequence properties (such as, *e.g.,* GC-rich and/or highly repetitive sequence stretches). Under such circumstances, oligonucleotides provided in a nicked confirmation can be used in a LCR reaction (ligation chain reaction) to directly ligate adjacent oligonucleotides prior to PCR extension as described, *e.g.,* in U.S. Patent No. 6,472,184. This process is based on a combination of overlap extension and ligation-based assembly wherein only the oligonucleotides forming the first strand are provided in a linkable" *(i.e.,* phosphorylated) form whereas the overlapping oligonucleotides forming part of the second strand are used as primers for PCR extension. In certain examples a LCR reaction may be desirable in particular where nucleic acid molecules with highly repetitive regions (*e.g.,* containing GC stretches) are to be assembled.

Simultaneous fusion of the hybridized sets of oligonucleotides with the 5' and 3' ends of the linearized target vector may be achieved for example by adding two additional "fusion oligonucleotides" (referred to as "fusion-oligos"). As exemplified in FIG. 8, the 3' region of a first fusion-oligo may be complementary to the 3' end of a first strand of the linearized vector, whereas the 5' region of the first fusion-oligo may be complementary to the 5' region of the oligonucleotide adjacent to the 3' end of the first strand of the linearized vector. Likewise, the 5' region of a second fusion-oligo may be complementary to the 5' end of the first strand of the linearized vector, whereas the 3' region of the second fusion-oligo may be complementary to the 3' region of the oligonucleotide adjacent to the 5' end of the first strand of the linearized vector. Using for example different sets of fusion-oligos with different vector complementary regions, a given polynucleotide can be assembled into different target vectors by combining the first and second sets of oligonucleotides with the respective pair of fusion-oligos. Complementary regions allowing for partial hybridization of fusion-oligos with vector ends may be about 15 to about 40 or about 20 to about 30 bases in size but may also be shorter or longer depending on the overall size and/or melting temperature of assembled nucleic acid products. In some instances, first and second fusion oligonucleotides comprise sequence portions that belong to the same strand of the double-stranded polynucleotide to be assembled. In some instances, first and second fusion oligonucleotides comprise sequence portions that belong to different strands. For purposes of illustration, first and second fusion oligonucleotides may be about 45 nucleotides in length, each one having a region of about 25-30 bp that is complementary to one of both ends of the linearized vector.

Subfragments of various lengths and assembled from various numbers of oligonucleotides may be inserted into a target polynucleotide or vector using the method of the disclosure. For example inserted subfragments or assembly products may have sizes of between about 100 bp and about 5 kb or between about 0.5 kb and about 3 kb or may have sizes of at least 2 kb. This method may be used to assemble from about two to about forty oligonucleotides (*e.g.,* from about two to about forty, from about three to about forty, from about five to about forty, from about eight to about forty, from about two to about thirty, from about two to about twenty, from about two to about ten, etc. nucleic acid molecules).

Melting temperatures of oligonucleotides typically depend on length and sequence requirements, but may typically be within a range of about 45 to about 72°C, such as about 55°C. The complementary regions within oligonucleotides used for joining may have a minimum length to allow for efficient base pairing of overlapping oligonucleotides and/or oligonucleotides with the target vector. For example, the complementary overlaps used for joining the f- and r-oligonucleotides and the fusion-oligos with a target vector will generally be from about 8 to about 30, from about 10 to about 30, from about 15 to about 20 nucleotides in length. In some examples, the overlaps may be at least about 15 bases or at least about 18 bases in length depending on the overall length of the nucleic acid molecules to be assembled. In other examples the overlaps may be between about 18 to about 25 bases or between about 20 to about 30 bases in length. As discussed above, the GC content of nucleic acids may affect the efficiency of assembly. The GC content of oligonucleotides used in a Sequence Elongation and Ligation reaction should therefore be somewhat balanced and may be within a range of about 30 to about 75% (*e.g.,* from about 35% to about 75%, from about 35% to about 70%, from about 40% to about 75%, from about 40% to about 70%, from about 45% to about 75%, from about 45% to about 70%, from about 45% to about 65%, etc.).

Other parameters that may be varied in examples of the disclosure include the concentration of oligonucleotides and target vector present and the ratio of these nucleic acids. In many instances, the nucleic acid concentration will be adjusted in combination with the concentration of other components, such as enzymatic activities (*e.g.,* a nuclease, an enzyme with ligase activity, a polymerase etc.). Further, the ratio of oligonucleotides within the assembly mixture will often be 1:1 but ratios may vary for particular applications and depending on the length of the oligonucleotides. When hybridization termini are AT-rich (*e.g.,* greater than 50%, 55%, 60%, 65% AT), these oligonucleotides may be present in a higher ratio than nucleic acid fragments with non-AT-rich hybridization termini. In some instances, oligonucleotides that share AT rich hybridization termini may be present in a ratio of from about 1.2:1 to about 2.5:1 (*e.g.,* from about 1.5:1 to about 2.5:1, from about 1.2:1 to about 2:1, from about 1.5:1 to about 2:1, etc.) with oligonucleotides that have non-AT-rich hybridization termini. Also, longer nucleic acids (typically the target vector) may be present at a lower concentration than shorter nucleic acids (typically the oligonucleotides to be inserted). For example, the molar ratio of vector to oligonucleotides may be about 1 : 10, 1 : 50, 1 : 100, 1 : 500, or 1 : 1,000.

The linearized polynucleotide or target vector may be any vector suitable for cloning and transforming a host cell. In many instances high-copy number vectors may be used to obtain high yields of the desired polynucleotide. Common high-copy number vectors include pUC (~ 500 - 700 copies), PBLUESCRIPT^{®} or PGEM^{®} (~ 300 - 500 copies, respectively) or derivatives thereof. In some instances, low-copy number vectors may be used, for example where high expression of a given insert may be toxic for the transformed cell. Such low-copy number vectors with copy numbers of between about 5 and about 30 include for example pBR322, various pET vectors, pGEX, pColE1, pR6K, pACYC or pSC101.

An exemplary list of vectors that can be used in any of the assembly or cloning methods disclosed herein, includes the following: BACULODIRECT^{™} Linear DIMA; BACULODIRECT^{™} Linear; DNA Cloning Fragment DNA; BACULODIRECT^{™} N-term Linear DNA_verA; BACULODIRECT^{™} C-Term Baculovirus Linear DNA; BACULODIRECT^{™} N-Term Baculovirus Linear DNA; CHAMPION^{™} pET100/D-TOPO^{®}; CHAMPION^{™} pET 101/D-TOPO^{®}; CHAMPION^{™} pET 102/D-TOPO^{®}; CHAMPION^{™} pET 104/D-TOPO^{®}; CHAMPION^{™} pET104-DEST; CHAMPION^{™} pET151/D-TOPO^{®}; CHAMPION^{™} pET 160/D-TOPO^{®}; CHAMPION^{™} pET 160-DEST; CHAMPION^{™} pET 161-DEST; CHAMPION^{™} pET200/D-TOPO^{®}; pAc5.1/V5-His A, B, and C; pAd/CMVA/5 DEST; pAd/PL-DEST; pAO815; pBAD/glll A, B, and C; pBAD/His A, B, and C; pBAD/myc-His A, B, and C; pBAD/Thio-TOPO^{®}; pBAD 102/D-TOPO^{®}; pBAD20/D-TOPO^{®}; pBAD202/D-TOPO^{®}; pBAD DEST49; PBAD-TOPO; PBAD-TOPO^{®}; pBlueBac4.5; pBlueBac4.5A/5-His TOPO^{®}; pBlueBacHis2 A, B, and C; pBR322; pBudCE4.1; pcDN3.1A/5-His-TOPO; pcDNA3.1(-); pcDNA3.1(+); pcDNA3.1(+)/myc-HisA; pcDNA3.1(+)/myc-His A, B, C; pcDNA3.1(+)/myc-His B; pcDNA3.1(+)/myc-HisC; pcDNA3.1/His A; pcDNA3.1/His B; pcDNA3.1/His C; pcDNA3.1/Hygro(-); pcDNA3.1/Hygro(+); pcDNA3.1/NT-GFP-TOPO; pcDNA3.1/nV5-DEST; pcDNA3.1A/5-His A; pcDNA3.1A/5-His B; pcDNA3.1A/5-His C; pcDNA3.1/Zeo(-); pcDNA3.1/Zeo(+); pcDNA3.1/Zeo(+); pcDNA3.1DA/5-His-TOPO; pcDNA3.2/V5-DEST; pcDNA3.2A/5-GW/D-TOPO; pcDNA3.2-DEST; pcDNA4/His A; pcDNA4/His B; pcDNA4/His C; pcDNA4/HisMax-TOPO; pcDNA4/HisMax-TOPO; pcDNA4/myc-His A, B, and C; pcDNA4/TO; pcDNA4/TO; pcDNA4/TO/myc-His A; pcDNA4/TO/myc-His B; pcDNA4/TO/myc-His C; pcDNA4/V5-His A, B, and C; pcDNA5/FRT; pcDNA5/FRT; pcDNA5/FRT/TO/CAT; pcDNA5/FRT/TO-TOPO; pcDNA5/FRT/V5-His-TOPO; pcDNA5/TO; pcDNA6.2/cGeneBLAzer-DEST_verA_sz; pcDNA6 2/cGeneBLAzer-GW/D-TOPO pcDNA6; 2/cGeneBlazer-GW/D-TOPO_verA_sz pcDNA6.2/cLumio-DEST; pcDNA6 2/cLumio-DE STverAsz; pcDNA6.2/GFP-DEST_verA_sz; pcDNA-DEST40; pcDNA-DEST47; pcDNA-DEST53; pCEP4; pCEP4/CAT; pCMV/myc/cyto; pCMV/myc/ER; pCMV/myc/mito; pCMV/myc/nuc; pCMVSPORT6 Notl-Sall Cut; pCoBlasi; pCR Blunt; pCR XL TOPO; pCR^{®}T7/CT TOPO^{®}; pCR^{®}T7/NT TOPO^{®}; pCR2.1-TOPO; pCR3.1; pCR3.1-Uni; pCR4BLUNT-TOPO; pCR4-TOPO; pCR8/GW/TOPO TA; pCR8/GW-TOPO_verA_sz; pCR-Blunt II-TOPO;-pCRII-TOPO; pDEST^{™} R4-R3; pDEST^{™}10; pDEST^{™}14; pDEST^{™}15; pDEST^{™}17; pDEST^{™}20; pDEST^{™}22; pDEST^{™}24; pDEST^{™}26; pDES^{™}27; pDEST^{™}32; pDEST^{™}8; pDEST^{™}38; pDEST^{™}39; pDisplay; PDONR^{™} P2R P3; PDONR^{™} P2R-P3; PDONR^{™} P4-P1R; PDONR^{™} P4-P1R; PDONR^{™}/Zeo; PDONR^{™}/Zeo; PDONR^{™}201; PDONR^{™}207; PDONR^{™}221; PDONR^{™}222; pEF/myc/cyto; pEF/myc/mito; pEF/myc/nuc; pEFi/His A, B, and C; pEF1/myc-His A, B, and C; pEF1/V5-HisA, B,andC; pEF4/myc-His A, B, and C; pEF4/V5-His A, B, and C; pEF5/FRT V5 D-TOPO; pEF5/FRT/V5-DEST^{™}; pEF6/His A, B, and C; pEF6/myc-His A, B, and C; pEF6/V5-His A, B, and C; pEF6A/5-His-TOPO; pEF-DEST51; PENTR^{™} U6_verA_sz; PENTR^{™}/HirTO_verA_sz; PENTR^{™}-TEV/D-TOPO; PENTR^{™}/D-TOPO; PENTR^{™}/D-TOPO; pHybLex/Zeo; pHyBLex/Zeo-MS2; pIB/His A, B, and C; pIBA/5-His Topo; pIBA/5-His-DEST; plBA/5-His-TOPO; plZA/5-His; p!ZT/V5-His; p1_en!i4 BLOCK-iT-DEST; pLenti4/BLOCK-iT-DEST; pLenti4/TOA/5-DEST; pThioHis A, B, and C; pTracer-CMV/Bsd; pTracer-CMV2; pTracer-EF A, B, and C; pTracer-EF/Bsd A, B, and C; pTracer-SV40; pTrcHis A, B. and C; pTrcHis2 A, B, and C; pTrcHis2-TOPO^{®}; pTrcHis2-TOPO^{®}; pTrcHis-TOPO^{®}; pT-Rex-DEST30; pT-Rex-DEST30; pT-Rex-DEST^{™} 31; pT-REx^{™}-DEST31; pUB/BSD TOPO; pUB6A/5-His A, B, and C; pUC18; pUC19; pUni/V5 His TOPO; pVAX1; pVP22/myc-His TOPO^{®}; pVP22/myc-His2 TOPO^{®}; pYC2.1-E; pYC2/CT; pYC2/Nt A, B. C; pYC2-E; pYC6/CT; pYD1; pYES2; pYES2.1A/5-His-TOPO; pYES2/CT; pYES2/NT; pYES2/NT A, B, & C; pYES3/CT; pYES6/CT; pYES-DEST^{™} 52; pYESTrp; pYESTrp2; pYESTrp3; pZeoSV2; pZeoSV2(+); PZERO^{™}-1; and PZERO^{™}-2.

In some examples, a linear double-stranded polynucleotide or target vector may have a limited size to allow for PCR-mediated elongation of the full-length fusion construct. However, the size of the vector is not limiting the applicability of this protocol as *E. coli* is capable of repairing incompletely elongated molecules and reconstituting full-length circular plasmid nucleic acid molecules upon transformation. Thus, in some examples the target vector may have a size of between about 0.5 kb (kilo base pairs) and about 5 kb, or between about 1 kb and about 3 kb, or between about 2 kb and about 10 kb or between about 5 kb and about 10 kb or between about 10 kb and about 30 kb or between about 10 kb and about 50 kb.

Assembled nucleic acid molecules may also include functional elements which confer desirable properties. These elements may either be provided by the plurality of oligonucleotides or by the double-stranded polynucleotide (e.g. a target vector). Examples of such elements include origins of replication, long terminal repeats, resistance markers (such as antibiotic resistance genes), selectable markers and antidote coding sequences (*e.g., ccdA* coding sequences for counter-acting toxic effects of *ccd*B), promoters, enhancers, polyadenylation signal coding sequences, 5' and 3' UTRs and other components suitable for the particular use(s) of the nucleic acid molecules (*e.g.,* enhancing mRNA or protein production efficiency). In examples where nucleic acid molecules are assembled to form an operon, the assembled nucleic acid products will often contain promoter and terminator sequences. Furthermore, assembled nucleic acid molecules may contain multiple cloning sites, such as, *e.g.,* type II or type IIS cleavage sites and/or GATEWAY^{®} recombination sites, as well as other sites for the connection of nucleic acid molecules to each other.

A polynucleotide may be linearized by any means including PCR amplification of a closed circular template nucleic acid molecule. Alternatively, the polynucleotide may be linearized by restriction enzyme cleavage with one or more enzymes producing either blunt or sticky ends. Such enzymes include restriction endonucleases of type II which cleave nucleic acid at fixed positions with respect to their recognition sequence. Restriction enzymes that can be selected to produce either "blunt" or "sticky" ends upon cleavage of a double-stranded nucleic acid are known to those skilled in the art and can be selected by the skilled person depending on the vector sequence and assembly requirements. In some instances, a vector may be linearized using a type II restriction endonuclease that generates blunt ends. Such endonucleases include, *e.g., Sca*I*, Sma*I, *Hpa*I, *Hinc*II, *Hae*II and *Alu*I. One subclass of restriction enzymes are restriction endonucleases of type IIS, which cleave a nucleic acid at a fixed position outside their recognition sequence. Type IIS restriction enzyme recognition sites and type IIS restriction enzymes that are useful in the present cloning and assembly methods, compositions, nucleic acids, vectors and kits include, but are not limited to, *Aar*I, *Bsa*I, *Bbs*I*, Bbv*II*, Bsm*AI*, Bsp*MI*, Eco*31I, *Bsm*BI*, Bae*I*, Fok*I*, Hga*I, *Mly*I, *Sfa*NI and *Sth*132I. Other means to cleave or linearize a vector include the use of site-specific engineered nucleases that induce double-strand breaks in a given nucleic acid molecule, such as Transcription activator-like effector nuclease (often referred to as TALEN), CRISPR/Cas9 nuclease, Zinc-finger nuclease or Argonaute protein-nuclease.

Following cleavage, the linearized polynucleotide or vector may either be used directly in a Sequence Elongation and Ligation reaction (following heat inactivation of the sample), or purified using gel extraction, or amplified in a PCR reaction prior to use in the Sequence Elongation and Ligation protocol. Purification of a linearized vector generated by PCR amplification is not required and the PCR product can be directly used in a Sequence Elongation and Ligation reaction. Also, a nuclease treatment of the vector (*e.g.,* by using the 3' exonuclease activity of T4 DNA polymerase) as described for the above standard workflow is no longer necessary since no protruding overhangs are required.

To limit the amount of errors introduced during the Sequence Elongation and Ligation assembly reaction, the overlap extension PCR reaction may be performed using a high fidelity polymerase with 3'-5' exo proofreading activity. A non-exhaustive list of suitable polymerases includes Pfu DNA polymerase (Thermo Fisher Scientific), DEEP VENT^{®} DNA polymerase (New England Biolabs), Q5^{®} High-Fidelity DNA Polymerase (New England Biolabs), PHUSION^{®} (New England Biolabs), PHUSION^{®} Hot Start Flex (New England Biolabs), PRIMESTAR^{®} HS (TAKARA), PRIMESTAR^{®} GXL (TAKARA), PRIMESTAR^{®} Max (TAKARA), ACCUPRIME^{™} Pfx (Thermo Fisher Scientific), PLATINUM^{™} DNA Polymerase High Fidelity (Thermo Fisher Scientific), PHUSION^{®} Flash II DNA Polymerase (Thermo Fisher Scientific), PHUSION^{®} Hot Start II High-Fidelity DNA Polymerase (Thermo Fisher Scientific), ACCURA^{®} High-Fidelity polymerase (Lucigen), IPROOF^{™} High-Fidelity polymerase (Bio-Rad), PAN PowerScript DNA Polymerase (PAN Biotech) or TRUESCRIPT^{™} DNA polymerase (PAN Biotech).

Following Sequence Elongation and Ligation assembly, the reaction mix comprising the assembled circularized construct or an aliquot thereof may be directly used to transform suitable competent host cells such as, *e.g.,* a common *E. coli* strain according to standard protocols. The skilled person can select suitable host cells depending on construct size and nucleotide composition, plasmid copy number, selection criteria etc. Useful strains are available through the American Type Culture Collection (http://www.atcc.org) and the *E. coli* Genetic Stock Center at Yale (cgsc.biology.yale.edu), as well as from commercial suppliers such as Agilent, Promega, Merck, Thermo Fisher Scientific, and New England Biolabs, respectively.

In many instances, nucleic acid molecules prepared by methods of the disclosure will be replicable. Further, many of these replicable nucleic acid molecules will be circular (*e.g.,* plasmids). Replicable nucleic acid molecules, regardless of whether they are circular, will generally be formed from the assembly of two or more (*e.g.,* three, four, five, eight, ten, twelve, etc.) nucleic acid fragments. In some instances, methods of the disclosure employ selection based upon the reconstitution of one or more (*e.g.,* two, three, four, etc.) selection marker or one or more (*e.g.,* two, three, four, etc.) origin of replication resulting from the linking of different nucleic acid fragments. Further selection may result from the formation of a circular nucleic acid molecule, in instances where circularity is required for replication.

Functional elements may be used to select for correctly assembled fusion constructs. For example, the target vector may provide one or more truncated and therefore inactive versions of certain elements (such as, *e.g.,* a portion of an origin of replication or a portion of a selection marker) which need to be completed by flanking sequences provided by complementary oligonucleotides during the assembly procedure. Such positive selection approach which renders only clones comprising correctly assembled functional elements is described in Baek et al., "Positive selection improves the efficiency of DNA assembly", Analytical Biochemistry 476 (2015), p. 1-4 and compatible with the above described Sequence Elongation and Ligation assembly method. Thus, the disclosure further includes methods involving multiple selection methods for obtaining assembled nucleic acid molecules. In one example, the disclosure includes methods for selecting assembled nucleic acid molecules through a combination of the generation of replicable vectors (*e.g.,* recircularized vectors) and one or more selectable marker. For example, some of the nucleic acid molecules to be assembled into a target vector may contain one or more resistance markers, one or more selectable markers or have functionalities that are otherwise required for replication (*e.g.,* contain an origin of replication).

The disclosure further comprises but not as part of the claimed invention, a kit for seamless cloning that comprises at least the following components: a first and second set of single-stranded oligonucleotides each set representing at least a portion of first and second strands of a double-stranded polynucleotide to be assembled, a linearized target vector, a polymerase, a buffer system, dNTPs, and, optionally, a pair of fusion oligonucleotides. In some examples, the 3' region of the first fusion oligonucleotide is complementary to the 3' end of a first strand of the linearized vector, whereas the 5' region of the first fusion oligonucleotide is complementary to the 5' region of the oligonucleotide adjacent to the 3' end of the first strand of the linearized vector; and the 5' region of a second fusion oligonucleotide is complementary to the 5' end of the first strand of the linearized vector, whereas the 3' region of the second fusion oligonucleotide is complementary to the 3' region of the oligonucleotide adjacent to the 5' end of the first strand of the linearized vector. The kit may further comprise competent host cells for transformation of assembled constructs. The kit may optionally include one or more of the following: assembly and/or cloning protocols and instructions, one or more controls for evaluation of assembly and/or transformation efficiency.

In one example, a kit for assembling a double-stranded target nucleic acid intended to have a predefined sequence may comprise: a population of single-stranded oligonucleotides each of the oligonucleotides having an assembly region representing a portion of the target nucleic acid, wherein each assembly region has a sequence region that is capable of hybridizing to at least one other oligonucleotide in the population; a linear double-stranded polynucleotide, each terminus of the linear double-stranded polynucleotide having a sequence region that is capable of hybridizing to at least one oligonucleotide of the population; a polymerase, dNTPs and a buffer system, and, optionally, competent host cells for transformation.

In another example, a kit for assembling a double-stranded target nucleic acid intended to have a predefined sequence may comprise: a population of single-stranded oligonucleotides each of the oligonucleotides having an assembly region representing a portion of the target nucleic acid, wherein each assembly region has a sequence region that is capable of hybridizing to at least one other oligonucleotide in the population; a circular double-stranded polynucleotide having a first and a second sequence region capable of hybridizing to at least one oligonucleotide of the population; a polymerase, dNTPs and a buffer system, and, optionally, competent host cells for transformation.

In an alternative example, the single-stranded oligonucleotides used in a Sequence Elongation and Ligation reaction may be replaced by one or more double-stranded nucleic acid fragments with complementary ends to allow overlap extension PCR with a linearized target vector (and between fragments if two or more fragments are to be assembled into a target vector simultaneously). The complementary ends (*i.e.,* the overlap) may have a size of between about 15 bp to about 50 bp, between about 20 bp to about 40 bp, such as, *e.g.,* 40 bp. The size of the required overlap may depend on the size of the fragments to be fused and the melting temperatures thereof. The double-stranded fragment(s) is/are first assembled from single-stranded oligonucleotides and amplified in the presence of terminal primers as described above in steps (ii) and (iii) of the standard workflow, respectively. The amplified fragments may then be subjected to one or more error correction and/or error removal cycles (*e.g.,* by mismatch endonuclease treatment as described below) and subsequently used in a combined insertion, elongation reaction as described for the Sequence Elongation and Ligation reaction above. In some examples, overlaps of the interconnected adjacent fragments and/or overlaps of the terminal fragments to the linearized vector may be from about 15 to about 40 or from about 18 to about 30 nucleotides in length. In instances where hybridization is required over a longer region to guarantee successful assembly, the overlaps may be from about 30 to about 60 nucleotides in length or even more than 60 nucleotides in length. Results indicate that an average error rate of 1 error in 6,000 bp is achievable, with a dual error correction (*e.g.,* assembly PCR followed by amplification PCR, 1st error correction as described below, overlap extension PCR to re-assemble error-corrected molecules, 2nd error correction, followed by a Sequence Elongation and Ligation, reaction and transformation) average error rates of 1 error in 15,000 bp can be realized. As illustrated in Example 2 and FIG. 9, the error rate in nucleic acid molecules assembled according to a Sequence Elongation and Ligation protocol was significantly reduced as compared to a standard workflow.

Assembled constructs obtained by a Sequence Elongation and Ligation workflow may be further combined with other Sequence Elongation and Ligation assembly products or nucleic acid molecules obtained from other sources to assemble larger nucleic acid molecules (*e.g.,* genes). Constructs of larger sizes may be assembled by any means known to the person skilled in the art. For example, Type IIS restriction site mediated assembly methods may be used to assemble multiple fragments (*e.g.,* two, three, five, eight, ten, etc.) when larger constructs are desired (*e.g.,* 5 to 100 kilobases). One suitable cloning system is referred to as Golden Gate which is set out in various forms in U.S Patent Publication No. 2010/0291633 A1 and PCT Publication WO 2010/040531.

It may be desirable at a number of points during workflows of the disclosure to separate nucleic acid molecules or assembly products from reaction mixture components (*e.g.,* dNTPs, primers, truncated oligonucleotides, tRNA molecules, buffers, salts, proteins, etc.). This may be done in a number of ways, such as, *e.g.,* by enzymatically removing undesired nucleic acid side-products with an exonuclease, restriction enzyme or UNG glycosylase. Once separated from reaction components for facilitating a process (*e.g.,* pooling or multiplexing of selected oligonucleotides, nucleic acid synthesis, error correction, etc.), nucleic acid molecules may then be used in additional reactions (*e.g.,* assembly reactions, amplification, cloning etc.).

Larger nucleic acid molecules may also be assembled *in vivo.* In *in vivo* assembly methods, a mixture of all of the subfragments to be assembled is often used to transfect the host cell using standard transfection techniques. The ratio of the number of molecules of subfragments in the mixture to the number of cells in the culture to be transfected should be high enough to permit at least some of the cells to take up more molecules of subfragments than there are different subfragments in the mixture. Thus, in most instances, the higher the efficiency of transfection, the larger number of cells will be present which contain all of the nucleic acid subfragments required to form the final desired assembly product. Technical parameters along these lines are set out in U.S. Patent Publication No. 2009/0275086 A1.

Large nucleic acid molecules are relatively fragile and, thus, shear readily. One method for stabilizing such molecules is by maintaining them intracellularly. Thus, in some aspects, the disclosure involves the assembly and/or maintenance of large nucleic acid molecules in host cells. Large nucleic acid molecules will typically be 20 kb or larger (*e.g.,* larger than 25 kb, larger than 35 kb, larger than 50 kb, larger than 70 kb, larger than 85 kb, larger than 100 kb, larger than 200 kb, larger than 500 kb, larger than 700 kb, larger than 900 kb, etc.).

Methods for producing and even analyzing large nucleic acid molecules are known in the art. For example, Karas et al., "Assembly of eukaryotic algal chromosomes in yeast, Journal of Biological Engineering 7:30 (2013) shows the assembly of an algal chromosome in yeast and pulse-field gel analysis of such large nucleic acid molecules.

As suggested above, one group of organisms known to perform homologous recombination fairly efficient is yeasts. Thus, host cells used in the practice of the disclosure may be yeast cells (*e.g., Saccharomyces cerevisiae, Schizosaccharomyces pombe, Pichia, pastoris,* etc.).

Yeast hosts are particularly suitable for manipulation of donor genomic material because of their unique set of genetic manipulation tools. The natural capacities of yeast cells, and decades of research have created a rich set of tools for manipulating DNA in yeast. These advantages are well known in the art. For example, yeast, with their rich genetic systems, can assemble and re-assemble nucleotide sequences by homologous recombination, a capability not shared by many readily available organisms. Yeast cells can be used to clone larger pieces of DNA, for example, entire cellular, organelle, and viral genomes that are not able to be cloned in other organisms. Thus, in some examples, the disclosure employs the enormous capacity of yeast genetics generate large nucleic acid molecules (*e.g.,* synthetic genomics) by using yeast as host cells for assembly and maintenance.

Exemplary of the yeast host cells are yeast strain VL6-48N, developed for high transformation efficiency parent strain: VL6-48 (ATCC Number MYA-3666TM)), the W303a strain, the MaV203 strain (Thermo Fisher Scientific, cat. no. 11281-011), and recombination-deficient yeast strains, such as the RAD54 gene-deficient strain, VL6-48- Δ 54G (MAT *α* his3- Δ 200 trp1- Δ 1 ura3-52 lys2 ade2-101 met14 rad54- Δ 1::kanMX), which can decrease the occurrence of a variety of recombination events in yeast artificial chromosomes (YACs).

### Error Correction

The disclosure further includes compositions and methods for the assembly of nucleic acid molecules with high sequence fidelity. High sequence fidelity can be achieved by several means, including sequencing of nucleic acid fragments prior to assembly or partially assembled nucleic acid molecules, sequencing of fully assembled nucleic acid molecules to identify ones with correct sequences, and/or error correction.

Errors may find their way into nucleic acid molecules in a number of ways. Examples of such ways include chemical synthesis errors, amplification/polymerase mediated errors (especially when non-proof reading polymerases are used), and assembly mediated errors (usually occurring at nucleic acid fragment junctions).

The assembled nucleic acid molecule may be a composite in the sense that all the components (*e.g.,* oligonucleotides) are synthesized and assembled or only some of the components are synthesized and these components are then assembled with other nucleic acid molecules (*e.g.,* nucleic acid fragments generated by PCR or nucleic acid molecules propagated within cells). However, for purposes of illustration, consider the situation where one hundred nucleic acid molecules are to be assembled, each molecule is one hundred base pairs in length and there is one error per 200 base pairs. The net result is that there will be, on average, 50 sequence errors in each 10,000 base pair assembled nucleic acid molecule. If one intends, for example, to express one or more proteins from the assembled nucleic acid molecule, then the number of amino acid sequence errors would likely be considered to be too high. Further, a number of the protein coding region nucleotide sequence errors will result in "frame shifts" mutations yielding proteins that will generally not be desired. Also, non-frame shift coding regions may result in the formation of proteins with point mutations. All of these will "dilute the purity" of the desired protein expression product and many of the produced "contaminant" proteins will be carried over into the final expression product mixture even if affinity purification is employed.

Sequence errors in nucleic acid molecules may be referenced in a number of ways. As examples, there is the error rate associated with the synthesis nucleic acid molecules, the error rate associated with nucleic acid molecules after error correct and/or the selection, and the error rate associated with end product nucleic acid molecules (*e.g.,* error rates of (1) a synthetic nucleic acid molecules that have either been selected for the correct sequence or (2) assembled chemically synthesized nucleic acid molecules). These errors may come from the chemical synthesis process, assembly processes, and/or amplification processes. Errors may be removed or prevented by methods, such as, the selection of nucleic acid molecules having correct sequences, error correction, and/or improved chemical synthesis methods.

In some instances, methods of the disclosure will combine error removal and prevention methods to produce nucleic acid molecules with relative low numbers of errors. Thus, assembled nucleic acid molecules produced by methods of the disclosure may have error rates from about 1 base in 1,500 to about 1 base in 30,000, from about 1 base in 2,000 to about 1 base in 30,000, from about 1 base in 4,000 to about 1 base in 30,000, from about 1 base in 8,000 to about 1 base in 30,000, from about 1 base in 10,000 to about 1 base in 30,000, from about 1 base in 15,000 to about 1 base in 30,000, from about 1 base in 10,000 to about 1 base in 20,000, etc.

Two ways to lower the number of errors in assembled nucleic acid molecules is by (1) selection of nucleic acid molecules (oligonucleotides, subfragments etc.) for assembly with corrects sequences and (2) correction of errors in nucleic acid molecules, partially assembled sub-assemblies, or fully assembled nucleic acid molecules.

In many instances, errors are incorporated into nucleic acid molecules regardless of the method by which the nucleic acid molecules are generated. Even when nucleic acid molecules known to have correct sequences are used for assembly, errors can find their way into the final assembly products. Thus, in many instances, error reduction will be desirable.

In most instances, regardless of the method by which a larger nucleic acid molecule is generated from chemically synthesized oligonucleotides, errors from the chemical synthesis process will be present. While sequencing of individual nucleic acid molecules may be performed to identify and select error-free nucleic acid molecules as described in more detail below, alternative approaches may comprise one or more error correction or removal steps. Thus, in many instances, error correction will be desirable. Error correction can be achieved by any number of means. Typically, such error removal steps will be performed after a first round of assembly. Thus, in one aspect, methods of the disclosure involve the following (in this order or different orders): (i) fragment amplification and/or assembly (*e.g.,* according to the methods described herein), (ii) error correction, (iii) final assembly (*e.g.,* according to the *in vitro* or *in vivo* methods described herein, *e.g.,* using a Sequence Elongation and Ligation protocol).

One exemplary method of error correction that may be used in methods described herein is set out in FIG. 10. FIG. 10A is a flow chart of an exemplary process for synthesis of error-minimized nucleic acid molecules. In the first step (line 1), nucleic acid molecules of a length smaller than that of the full-length desired nucleotide sequence (*e.g.,* oligonucleotides) are obtained. Each oligonucleotide is intended to have a desired nucleotide sequence that comprises a part of the full-length desired nucleotide sequence. Each oligonucleotide may also be intended to have a desired nucleotide sequence that comprises an adaptor primer for PCR amplification of the nucleic acid molecule, a recognition site for a restriction enzyme, a tethering sequence for attachment to a microchip or solid support, or any other nucleotide sequence determined by any experimental purpose or other intention. The oligonucleotides may be obtained in any of one or more ways as described elsewhere herein, for example, through synthesis, purchase, etc.

In the optional second step (line 2), the oligonucleotides are amplified to obtain more of each oligonucleotide. In many instances, however, sufficient numbers of oligonucleotides will be produced so that amplification is not necessary. When employed, amplification may be accomplished by any method known in the art, for example, by PCR, Rolling Circle Amplification (RCA), Loop Mediated Isothermal Amplification (LAMP), Nucleic Acid Sequence Based Amplification (NASBA), Strand Displacement Amplification (SDA), Ligase Chain Reaction (LCR), Self Sustained Sequence Replication (3SR) or solid phase PCR reactions (SP-PCR) such as Bridge PCR etc. (*see, e.g.,* Fakruddin et al., J. Pharm. Bioallied. Sci. 5(4):245-252 (2013) for an overview of the various amplification techniques). Introduction of additional errors into the nucleotide sequences of any of the nucleic acid molecules may occur during amplification. In certain instances it may be favorable to avoid amplification following synthesis. The optional amplification step may be omitted where nucleic acid molecules have been produced at sufficient yield in step 1. This may be achieved by using *e.g.,* optimized bead formats, designed to allow synthesis of nucleic acid molecules at sufficient yield and quality as described, *e.g.,* in PCT Publication WO 2016/094512.

In the third step (line 3), the optionally amplified nucleic acid molecules are assembled into a first set of nucleic acid molecules intended to have a desired length, which may be the intended full-length of the desired nucleotide sequence or a fragment thereof. Assembly of amplified nucleic acid molecules into full-length molecules or fragments may be accomplished in any way, for example, by using a PCR-based method.

In the fourth step (line 4), the first set of assembled nucleic acid molecules is denatured. Denaturation renders single-stranded molecules from double-stranded molecules. Denaturation may be accomplished by any means. In some examples, denaturation is accomplished by heating the molecules.

In the fifth step (line 5), the denatured molecules are annealed. Annealing renders a second set of double-stranded nucleic acid molecules from single-stranded molecules. Annealing may be accomplished by any means. In some examples, annealing is accomplished by cooling the molecules. Some of the annealed molecules may contain one or more mismatches indicating sites of sequence error.

In the sixth step (line 6), the second set of molecules are reacted with one or more mismatch cleaving endonucleases to yield a third set of nucleic acid molecules intended to have lengths less than the length of the complete desired gene sequence. Exemplary mismatch binding and/or cleaving enzymes include T7 endonuclease I, endonuclease VII (encoded by the T4 gene 49), RES I endonuclease, CEL I endonuclease, and SP endonuclease or an endonuclease containing enzyme complex. The endonucleases cut one or more of the molecules in the second set into shorter molecules. The cuts may be accomplished by any means. Cuts at the sites of any nucleotide sequence errors are particularly desirable, in that assembly of pieces of one or more molecules that have been cut at error sites offers the possibility of removal of the cut errors in the final step of the process.

Variations of this process are as follows. First, two or more (*e.g.,* two, three, four, five, six, etc.) rounds of error correction may be performed. Second, more than one endonuclease may be used in one or more rounds of error correction. For example, T7 endonuclease I and Cel II may be used in each round of error correction. Third, different endonucleases may be used in different error correction rounds or may be combined with steps of error filtration using mismatch binding proteins. For example, a pool of re-annealed oligonucleotides may be subject to an error filtration step using a mismatch binding protein (such as MutS) to remove a first plurality of oligonucleotides having errors from the pool and the remaining ("unbound") oligonucleotides may then be subject to an error correction step using an endonuclease such as, *e.g.,* T7 endonuclease I to correct remaining errors. In another example, T7 endonuclease I and Cel II may be used in a first round of error correction and Cel II may be used alone in a second round of error correction. In another exemplary example, the molecules are cut only with one endonuclease (which may be a single-strand nuclease, such as Mung Bean endonuclease or a resolvase, such as T7 endonuclease I or another endonuclease of similar functionality). In yet another example the same endonuclease (*e.g.,* T7 endonuclease I) may be used in two subsequent error correction rounds. In yet another example an enzyme having mismatch cleavage activity may be combined with an enzyme having exonuclease activity to allow for removal of errors contained in single-stranded overhangs following mismatch cleavage. In specific examples, mismatch endonucleases having intrinsic exonuclease activity may be used to achieve cleavage and subsequent error removal in a single step. Enzymes having both endonuclease and exonuclease activities include, *e.g.,* Mung Bean nuclease, Cel I or SP1 endonuclease. In other examples, removal of errors may be achieved by a separate step comprising further exonuclease treatment as described, for example, in PCT Publication WO 2005/095605 A1.

In many instances, one or more ligase may be present in reaction during error correction. It is believed that some endonucleases used in error correction processes have nickase activity. The inclusion of one or more ligase is believed to seal nicks caused by such enzymes and increase the yield of error corrected nucleic acid molecules after amplification. Exemplary ligases that may be used are T4 DNA ligase, Taq ligase, and PBCV-1 DNA ligase. Ligases used in the practice of the disclosure may be thermolabile or thermostabile (*e.g.,* Taq ligase). If a thermolabile ligase is employed, it will typically need to be added to a reaction mixture for each error correction cycle. Thermostabile ligases will typically not need to be re-added during each cycle, so long as the temperature is kept below their denaturation point.

In instances where the second set of molecules represents a subfragment of the full-length molecules, two or more subfragments (*e.g.,* two or three or more subfragments) together representing the full-length molecules may be combined and reacted with the one or more mismatch cleaving endonucleases in a single reaction mix. For example, where the open reading frame that is to be assembled is longer than 1 kb, it may be broken up into two or more subfragments separately assembled in parallel reactions in step three and the resulting two or more subfragments may be combined and error-corrected in a single reaction as indicated in FIG. 10B. The amount of subfragments to be combined in a single error correction cycle may depend on the length of the individual subfragments. For example, up to three subfragments of about 1 kb in length may be efficiently combined in a single reaction mixture. Of course, more than three (*e.g.,* four, five, six, seven, eight, nine, etc.) subfragments may be combined. Assembly efficiency may decrease so long as at least one correctly assembled amplifiable and/or replicable nucleic acid molecule is obtained. Thus, numerous subfragments (*e.g.,* subfragments of about 1 kb in length) may be assembled so long as a correctly assembled product nucleic acid molecule is obtained from the assembly process.

In the seventh step (line 7) of FIG. 10A, the third set of molecules is assembled into a fourth set of molecules, whose length is intended to be the full-length of the desired nucleotide sequence. In the seventh step, which is typically based on overlap extension PCR, the 3'->5' exonuclease activity of the DNA polymerase removes the 3' overhangs generated by endonuclease cleavage in the sixth step at sites of mismatch thereby removing the error. Thus, the intrinsic exonuclease activity of a DNA polymerase can be used to remove errors during assembly that have not been removed in step 6 (*e.g.,* by using a combination of nucleases with mismatch cleavage and exonuclease activities). This principle is outlined, *e.g.,* in Saaem et al. ("Error correction of microchip synthesized genes using Surveyor nuclease", Nucl, Acids Res., 40:e23 (2012)). Such final assembly step may be performed in the presence of terminal primers thereby including functionalities required for downstream processes such as cloning or protein expression. A respective PCR reaction may be set up to first allow the error-corrected fragments to assemble by overlap extension to the full-length in about 15 cycles of denaturation, annealing and extension in the absence of the terminal primers, followed by additional 20 cycles in the presence of the terminal primers.

The process set out above and in FIG. 10A is also set out in U.S. Patent No. 7,704,690. Furthermore, the process described above may be encoded onto a computer-readable medium as processor-executable instructions.

One representative workflow that may be used in methods of the disclosure is set out in FIG. 10B. In this workflow, three nucleic acid subfragments (line 1) are pooled and subjected to error correction in a 1-cup reaction using one or more mismatch endonucleases (line 2). The resulting products are then assembled by PCR (line 3) and then subjected to a second round of error correction which may be conducted using the same, one or more different or one or more additional mismatch endonucleases. After another round of PCR (line 5), the resulting nucleic acid molecules are transformed into *E. coli* (step 6) and then screened for those that are full-length (line 7) followed by DNA preparation (line 8). These nucleic acid molecules may then be screened for remaining errors by, for example, sequencing (line 9). In a first variation of the workflow of FIG. 10B, the pooled subfragments may be treated with an exonuclease (such as, *e.g.*, Exonuclease I) before they are subjected to the error correction process. Exonuclease treatment eliminates single-stranded primer molecules left over in the PCR reaction product that may interfere with subsequent PCR reactions and generate unspecific amplification products. In a second variation of the workflow, the first error correction step may use more than one endonuclease such as, *e.g.,* T7NI combined with RES I or Cel I. Optionally, the workflow may comprise a third error correction or error removal step to eliminate remaining mismatches after fragment fusion PCR. Such third step may be conducted with a mismatch binding protein such as, *e.g.,* MutS. The skilled person will understand that various orders and combinations of first, second and/or third and possibly further error correction and/or removal cycles may be applied to further decrease the error rate of assembled nucleic acid molecules.

Another process for effectuating error correction in chemically synthesized nucleic acid molecules that may be used in methods of the disclosure is by a commercial process referred to as FRRASE^{™} (Novici Biotech).

Yet another process for reducing errors during nucleic acid synthesis that may be used in aspects of the disclosure is referred to as Circular Assembly Amplification and described in PCT Publication WO 2008/112683 A2.

Synthetically generated nucleic acid molecules typically have error rate of about 1 base in 300-500 bases. Conditions can be adjusted so that synthesis errors are substantially lower than 1 base in 300-500 bases. Further, in many instances, greater than 80% of errors are single base frame shift deletions and insertions. Also, less than 2% of errors result from the action of polymerases when high fidelity PCR amplification is employed. Therefore, error-correction processes using PCR-based assembly steps as described above may be combined with one or more error-correction methods not involving polymerase activity. In many instances, mismatch endonuclease (MME) correction will be performed using fixed protein:DNA ratio. Non-PCR-based error correction may, *e.g.,* be achieved by separating nucleic acid molecules with mismatches from those without mismatches by binding with a mismatch binding agent in a number of ways. For example, mixtures of nucleic acid molecules, some having mismatches, may be (1) passed through a column containing a bound mismatch binding protein or (2) contacted with a surface (*e.g.,* a bead (such as a magnetic bead), plate surface, etc.) to which a mismatch binding protein is bound.

Exemplary formats and associated methods involve those using surfaces or supports (*e.g.,* beads) to which a mismatch binding protein is bound. For example, a solution of nucleic acid molecules may be contacted with beads to which is bound a mismatch binding protein. One mismatch binding protein that may be used in various aspects of the disclosure is MutS from *Thermus aquaticus* the gene sequence of which is published in Biswas and Hsieh, J. Biol. Chem. 271:5040-5048 (1996) and is available in GenBank, accession number U33117. Furthermore, mismatch cleavage endonucleases such as T7 endonuclease I or Cel I from, for example, celery may be genetically engineered to inactivate the cleavage function for use in error filtration processes based on mismatch binding. Nucleic acid molecules that are bound to a mismatch binding protein may either be actively removed from a pool of nucleic acid molecules (*e.g.,* via magnetic force where magnetic beads coated with mismatch binding proteins are used) or may be immobilized or linked to a surface such that they remain in the sample whereas unbound nucleic acids are removed or transferred (*e.g.,* by pipetting, acoustic liquid handling etc.) from the sample. Such examples are set out, for example, in PCT Publication WO 2016/094512.

Error correction methods and reagents suitable for use in methods of the disclosure are set out in U.S. Patents Nos. 7,838,210 and 7,833,759, U.S. Patent Publication No. 2008/0145913 A1 (mismatch endonucleases), PCT Publication WO 2011/102802 A1, and in Ma et al., Trends in Biotechnology, 30(3):147-154 (2012). Furthermore, the skilled person will recognize that other methods of error correction and/or error filtration (*i.e.,* specifically removing error-containing molecules) may be practiced in certain examples of the disclosure such as those described, for example, in U.S. Patent Publication Nos. 2006/0127920 AA, 2007/0231805 AA, 2010/0216648 A1, or 2011/0124049 A1.

### Sequence Verification

Once nucleic acid molecules are assembled and optionally error-corrected, their sequences may be verified to confirm that "junction" sequences are correct and that no other nucleotide sequence "errors" are located within assembled nucleic acid molecules.

A number of nucleic acid sequencing methods are known in the art and include Maxam-Gilbert sequencing, chain-termination sequencing (*e.g.,* Sanger sequencing), pyrosequencing, sequencing by synthesis and sequencing by ligation. Methods of the disclosure may use any type of sequencing platform suitable for the intended purpose, including any next-generation sequencing platform such as: sequencing by oligonucleotide probe ligation and detection (*e.g.,* SOLID^{™} from Thermo Fisher Scientific, (see *e.g.,* PCT Publication No. WO 2006/084131), probe-anchor ligation sequencing (*e.g.,* COMPLETE GENOMICS^{™} or POLONATOR^{™}), sequencing-by-synthesis (*e.g.,* GENOME ANALYZER^{™} and HISEQ^{™}, from Illumina), pyrophosphate sequencing (*e.g.,* Genome Sequencer FLX from 454 Life Sciences), single molecule sequencing platforms (*e.g.,* HELISCOPE^{™} from HELICOS^{™}), and ion-sensitive sequencing (*e.g.,* Personal Genome Machine, Proton and Ion S5 from ION TORRENT^{™} Systems, Inc.) as set out, *e.g.,* in PCT Publication WO 2012/044847 or U.S. Patent No. 7,948,015. For an overview, *see, e.g.,* Mardis E.R., "Next-Generation Sequencing Platforms", Annu. Rev. Anal. Chem. 6:287-303 (2013).

Gene assembly workflows are typically completed by identifying a clone comprising the desired polynucleotide. For this purpose assembled polynucleotides are often transformed into *E. coli* followed by several hours (often overnight) of incubation to allow for growth of transformed bacteria to colonies of visible size. Colony PCR may then be performed to identify clones comprising correctly assembled polynucleotides having the desired length and isolated clones may further be sequenced (*e.g.,* by Sanger sequencing techniques) to identify error-free polynucleotides. Such cloning-based workflows are tedious and slow and present a bottleneck for high-throughput, automated and inexpensive genome construction. New technologies have therefore been developed based on a combination of next generation sequencing and *"in vitro* cloning" to allow for massive multiplexing of gene or genome synthesis.

Apart from using sequence analysis solely for the (final) verification of isolated clones, sequencing may also be used as an alternative to error correction to identify individual error-free molecules within a pool of nucleic acids and selectively retrieve the error-free nucleic acid molecules. Sequence verification prior to (full-length) nucleic acid assembly may be useful to avoid the carry-over of errors from chemically synthesized nucleic acid molecules into amplification reactions thereby multiplying the errors in assembled polynucleotides. This may be done for example by sequencing a pool of amplified nucleic acid molecules to determine if any errors are present and retrieving one or more error-free molecules for downstream processing. Thus, sequencing techniques may be applied to identify and select error-free nucleic acid molecules for amplification and subsequent assembly.

Sequence-verified nucleic acid molecules may be retrieved by various means. One method of selective retrieval of desired nucleic acids is referred to as "laser catapulting" which relies on the use of high-speed laser pulses to eject selected clonal nucleic acid populations from a sequencing plate. This method is described, for example, in U.S. Patent Publication No. 2014/0155297.

Another method often referred to as "dial-out PCR" is based on massive parallel sequencing of a complex mixture of nucleic acid fragments modified with unique flanking tags followed by retrieval of error-free nucleic acid molecules from the library by performing a PCR reaction with tag-directed primers for subsequent gene assembly. Dial-out PCR is described *e.g.*, in U.S. Patent Publication No. 2012/0283110, 2012/0322681 or 2014/0141982.

Other methods for retrieving sequence-verified nucleic acid molecules are set out in U.S. Patent No. 8,173,368. The described method comprises the steps of monoclonizing nucleic acids from a mixture of different nucleic acid molecules, parallel sequencing of the monoclonized nucleic acids, identifying and localizing an individual nucleic acid with a desired sequence, and isolating the individual nucleic acid with the desired sequence for further processing. Localization of the desired sequence is effected by immobilization of the support during sequencing and molecules having the desired sequence are then removed directly from the sequencing reaction support. Retrieval of the selected nucleic acids may be accomplished by isolating of beads comprising the selected nucleic acid, cleaving off the nucleic acids from the respective support on which they are immobilized, selective amplification by spatially-resolved addition of PCR reagents or elution by a laser capture method.

Once, error-corrected or sequence-verified nucleic acid molecules have been retrieved, they may be further processed and cloned into a target vector and/or assembled into larger nucleic acid molecules according to any of the assembly methods disclosed or referenced herein. In some instances, the sequence-verified nucleic acid molecules may be purified by standard methods (such as by bead-based extraction, gel extraction, column purification and/or alcohol precipitation) before they are used for downstream assembly or cloning procedures.

In some instances, optionally error-corrected or sequence-verified sub fragments may be further cloned or assembled using a Golden Gate assembly method as disclosed above.

Golden Gate assembly is a one-tube efficient cloning method based on type IIS restriction enzymes (such as *Bsm*BI*, Bbs*I*, Bsa*I, *Aar*I, *Sap*I*, Btg*ZI*,* etc.) that cleave outside their recognition sites and typically leave 4-base overhangs. For this purpose, one or more (*e.g.,* 2, 3, 4, 5, 6, 7, 8, 9 or 10 etc.) linear fragments comprising parts A, B, etc., may be combined with a target vector and cleaved with one or more type IIS restriction enzymes to generate compatible overhangs between part A and a first end of the vector, between part A and part B, and between part B and a second end of the vector (or, if applicable between part B and further parts) to allow for insertion of all parts into the vector in a seamless and oriented manner. The reaction mixture may also comprise a ligase (*e.g.,* a T4 DNA ligase or a Taq ligase) for ligation of the assembled parts. Optionally, correctly assembled parts ligated together in a closed circle may further be treated with an exonuclease (*e.g.,* T5 exonuclease) to degrade all noncircular nucleic acid fragments, leaving intact only the complete circular ligation products for subsequent transformation.

It has been demonstrated that an exonuclease treatment prior to transformation increases transformation efficiency by removing unwanted noncircular nucleic acid molecules from the sample (such as cleaved vector backbone, partial assembly products, cleaved parts etc.) which may compete with the circular ligation products in transformation. Without an exonuclease treatment the correct ligation product would need to be gel purified before proceeding. The combination of Golden Gate digestion-ligation and exonuclease treatment therefore reduces the overall hands-on time required for fragment assembly. The reaction mixture comprising the assembled construct may then optionally be heated (*e.g.,* 10 min. at 65°C) to inactivate any residual enzyme activity and may then be transformed into *E. coli.* Positive clones of the assembled nucleic acid can be selected according to standard procedures. Alternatively, the assembled construct may be combined with another assembled construct in a further Golden Gate assembly reaction and optionally an additional target vector without purification or clone selection after the first assembly step.

The assembly methods disclosed above can be applied to efficiently assemble between about 2 and about 20 or between about 4 and about 10 parts depending on the overall size of the assembled parts. In some instances type IIS-based assembly methods may be used to assemble up to 10 parts having sizes of between about 200 and about 600 base pairs in directed order. In some instances the assembly methods may be used to assemble desired nucleic acid molecules of between about 3,000 and about 6,000 base pairs in length or between about 5,000 and about 15,000 base pairs in length.

Methods described herein that combine high throughput oligonucleotide synthesis and pooling, followed by highly efficient multiplexed nucleic acid assembly, including error correction or retrieval of sequence-verified nucleic acid molecules provide optimal tools for library production and parallel testing of multiple genetic variants. This can include the evaluation of several natural homologs, the systematic screening of protein variants with site saturation libraries (comprising one amino acid substitution at a time), or the selection of best candidates from complex nucleic acid libraries with ambiguities at several nucleotide positions. All these approaches have in common, that it requires specimen of genetic material (*e.g.,* an ORF) which are mostly similar to each other but comprise defined differences. The disclosed methods allow for exact synthesis of a gene as designed *in silico* including bundles of distinct nucleic acid variants and also synthetic DNA libraries for directed evolution.

FIG. 11 provides two examples, of how the disclosed assembly strategies can be used to produce distinct libraries or DNA variants using synthetic nucleic acid building blocks. In a first example shown in FIG. 11A, an oligonucleotide set for PCR-based assembly of a desired nucleic acid molecule may be obtained from an array or microchip. The set of oligonucleotides may comprise one or more oligonucleotides designed with some sequence variability or carrying known mutations, such that nucleic acid fragments having defined base variations (shown as DEF, DFE, FDF etc. at a first position and ABA, AAC, ABC etc. at a second position) will be assembled from the same oligonucleotide pool in a multiplex PCR reaction.

In a second example shown in FIG. 11B a pool of overlapping oligonucleotides is used for multiple parallel assembly reactions and only one or a few distinct oligonucleotides are replaced in each reaction by a specific variant (*e.g.,* using an oligonucleotide introducing mutation A at a defined position in a first reaction and using a second oligonucleotide introducing mutation B at the defined position in a second reaction etc.). Although the principle of oligonucleotide-based library production has been widely used, the efficiency was limited by the economically feasible number of oligonucleotides. With high throughput chip-based oligonucleotide synthesis followed by efficient multiplexed assembly in microscale and retrieval of sequence-corrected or sequence-verified nucleic acid molecules as disclosed herein, the applicability for library production can be expanded by orders of magnitude.

To achieve an even higher degree of correctness final nucleic acid products may be assembled from mixed pools of PCR products and/or cloned fragments representing the "constant regions" (*i.e.,* the sequence region in a library that is not subject to variation) with oligonucleotides covering and introducing the variation. Variants may also be synthesized by first generating linear fragments from chip oligonucleotides (*e.g.,* in a PCR reaction), selecting 100% correct fragments (*e.g.,* through sequencing and retrieval methods disclosed herein) and assembling the fragments in a combinatorial way. For purpose of illustration, a nucleic acid product having 24 x 3 x 1 x 5 = 360 combinations may be assembled from combining 24 variants of fragment A plus three variants of fragment B plus one variant of fragment C plus five variants of fragment D. For combinatorial assembly, any of the assembly strategies disclosed above may be used, such as exonuclease-based seamless cloning, fusion PCR, homologous recombination, Golden Gate cloning etc.

### Example: Cloning efficiency and sequence correctness of polynucleotides assembled according to a Sequence Elongation and Ligation protocol

Sequence Elongation and Ligation reactions were carried out to assemble 30 polynucleotide fragments with sizes ranging from 260 - 970 base pairs and a GC content of between 25% and 71%. A minimal cloning vector (a pUC derivative) was linearized by restriction endonuclease cleavage with *Kpn*I and *Sac*I (New England Biolabs) and purified by gel extraction. Alternatively, the vector was linearized via amplification in a standard PCR reaction. For the subsequent Sequence Elongation and Ligation reaction the components indicated in Table 2A were mixed and subjected to a PCR reaction using the cycling conditions specified in Table 2B:

| Table 2A: Reaction Components | |
|---|---|
| mix of f- and r-oligonucleotides (0.15 µM each) | 2.5 µl |
| linearized vector | 50 ng |
| dNTPs (10 mM) | 0.5 µl |
| 10 x PAN buffer | 0.5 µl |
| Truescript DNA polymerase (2.5 U/µl) | 1 µl |
| H₂O | ad 25 µl |

| Table 2B: Cycling Conditions | | |
|---|---|---|
| 95°C | 4 min | |
| 95°C | 30 sec | 35x |
| 70°C - 38.5°C* | 30 sec | |
| 72°C | 3 min | |
| 72°C | 6 min | |
| * -0.9°C/cycle | | |

The 30 fragments were likewise assembled using a standard workflow by mixing f- and r-oligonucleotides at equimolar amounts and subjecting the mixture to a 1st PCR reaction for oligonucleotide assembly, and using an aliquot of the resulting PCR product in a 2nd PCR reaction in the presence of terminal amplification primers to amplify the assembled fragments. The amplified reaction products were purified and inserted into a target vector using a standard seamless cloning protocol (Aslanidis and de Jong, "Ligation-independent cloning of PCR products (LIC-PCR)", Nucleic Acids Res. 18(20):6069-74 (1990)).

To compare the "clone-based cloning efficiency" (*i.e.,* the number of clones with expected insert size per total analyzed clones) of the two protocols used, four colony forming units ("cfu") per polynucleotide fragment were analyzed by colony PCR. For this purpose the picked *E. coli* colonies were added to a PCR reaction comprising primers designed to specifically target the insert polynucleotide and the PCR products were analyzed by gel electrophoresis. The average results obtained for all 30 fragments are shown in FIG. 9. As shown in FIG. 9A, the fraction of all constructs with at least one size-and-sequence-correct clone, out of four analyzed clones (fragment-based cloning efficiency, black bars) reached 90% or 97% (depending on whether the vector was linearized by PCR or digestion) when the Sequence Elongation and Ligation protocol was used as compared to 63% achieved with the standard protocol. Also, the percentage of overall correct clones (white bars) as determined by Sanger sequencing was significantly higher when the Sequence Elongation and Ligation protocol was used (77% and 90%, respectively as compared to 54% in FIG. 9A).

Furthermore, the clone-based sequence correctness was determined, wherein the sequence correctness is defined by the number of clones with 100% correct sequence per total clones analyzed. While the average clone-based sequence correctness (defined by the number of clones with 100% correct sequence per total clones analyzed) proved to be similar for both protocols as shown in FIG. 9B (white bars), the percentage of fragments with at least one clone having the right size and correct sequence (fragment-based sequence correctness, black bars) was significantly higher in the Sequence Elongation and Ligation reaction (78% with PCR-generated vector, 83% with vector produced via digestion) and exceeded significantly the corresponding fraction of correct fragments of the standard workflow (37%). The correct clone-rate achieved for the Sequence Elongation and Ligation protocols results from a combination of the high cloning efficiency and the improved correctness of the sequence.

These results demonstrate that the Sequence Elongation and Ligation protocol is not only superior to the above described standard workflow in terms of saving of materials and time and throughput increase, but also in terms of the significantly higher stability (clone based cloning efficiency and sequence correctness).

While specific embodiments of the present invention have been shown and described herein, it will be obvious to those skilled in the art that such embodiments are provided by way of example only. Numerous variations, changes, and substitutions will now occur to those skilled in the art. It should be understood that various alternatives to the embodiments of the invention described herein may be employed. It is intended that the following claims define the scope of the invention.

All publications, patents, and patent applications mentioned in this specification are U.S. Patent Publication Nos. 2003/0152984; 2006/0115850; 2006/0127920; 2007/0141557; 2007/0231805; 2007/0292954; 2009/0275086; 2010/0062495; 2010/0216648; 2010/0291633; 2011/0124049; 2012/0283110, 2012/0322681; 2014/0141982; and 2014/0155297. U.S. Patents Nos. 5,580,759; 5,624,827; 5,789,577; 5,869,644; 6,110,668; 6,472,184; 6,495,318; 6,521,427; 7,164,992; 7,704,690; 7,833,759; 7,838,210; 7,948,015; 8,173,368; 8,224,578; 8,728,767; and 8,808,989. PCT Publications WO 2005/095605; WO 2006/084131; WO 2008/112683; WO 2010/040531; WO 2011/102802; WO 2012/044847; WO 2013/049227; and WO 2016/094512.

## Claims

1. A method of assembling a double-stranded target nucleic acid intended to have a predefined sequence, the method comprising:
a) providing a population of single-stranded oligonucleotides having less than 200 nucleotides in length, wherein the plurality of single-stranded oligonucleotides are obtained by chemical synthesis,
wherein the population of single-stranded oligonucleotides comprise
a first set of oligonucleotides, together comprising at least a portion of a first strand of the double-stranded target nucleic acid, and
a second set of oligonucleotides together comprising at least a portion of a second strand of the double-stranded target nucleic acid,
wherein each oligonucleotide within the first set of oligonucleotides has a sequence region that is capable of hybridizing to at least one other oligonucleotide in the second set of oligonucleotides
each of the oligonucleotides having an assembly region representing a portion of the target nucleic acid, wherein each assembly region has a sequence region that is capable of hybridizing to at least one other oligonucleotide in the population,
b) providing a linear double-stranded polynucleotide, each terminus of the linear double-stranded polynucleotide having a sequence region that is capable of hybridizing to at least one oligonucleotide of the population,
c) combining the population of single-stranded oligonucleotides with the linear double-stranded polynucleotide and a polymerase and dNTPs in a buffer to generate a reaction mixture, and
d) subjecting the reaction mixture to cycling conditions that allow for
(i) at least partial denaturation of the linear double-stranded polynucleotide to obtain single-stranded termini,
(ii) hybridization of the population of single-stranded oligonucleotides to one another and to the single-stranded termini of the polynucleotide in a pre-determined order,
(iii) polymerase-mediated extension of the free 3' ends of the hybridized oligonucleotides and the polynucleotide strands to generate a double-stranded non-covalently closed circularized assembly product comprising a linear arrangement of assembly regions, and
(iv) amplification of the double-stranded circularized assembly product to generate an amplified double-stranded target nucleic acid intended to have a predefined sequence.

2. The method of claim 1, wherein the first and second sets of oligonucleotides are designed to hybridize with nicks or one or more nucleotide gaps between adjacent oligonucleotides within the same strand.

3. The method of any of any one of claims 1 or 2, wherein the first set of oligonucleotides is designed to hybridize with nicks between adjacent oligonucleotides, whereas the second set of oligonucleotides is designed to hybridize with one or more nucleotide gaps between adjacent oligonucleotides, optionally the oligonucleotides of the first set are between about 36 to about 120 nucleotides in length, and wherein the oligonucleotides of the second set are between about 36 and about 44 nucleotides in length.

4. The method of any previous claim, wherein the sequence region of the oligonucleotide assembly region that is capable of hybridizing to at least one other oligonucleotide in the population is at least 8 nucleotides, preferably at least 12 nucleotides in length.

5. The method of any previous claim, wherein the sequence region of the oligonucleotide assembly region that is capable of hybridizing to a terminus of the linear polynucleotide is between about 20 and about 45 nucleotides in length.

6. The method of any previous claim, wherein each single-stranded oligonucleotide further comprises a tag and a spacer,
wherein the tag is located at the 5'-end of the assembly region of each oligonucleotide and the spacer is located between the tag and the assembly region, and
wherein the spacer is either cleavable or capable of terminating enzymatic mediated nucleic acid replication reactions.

7. The method of claim 6, wherein each oligonucleotide in the population comprises a tag with an identical nucleotide sequence, optionally wherein the tag is between about 12 and about 24 nucleotides in length.

8. The method of claim 7, wherein the spacer comprises a hexaethylene glycol group.

9. The method of any previous claim, wherein the chemical synthesis comprises a photo-chemical or electrochemical deprotection step.

10. The method of any previous claim, wherein the plurality of single-stranded oligonucleotides are obtained by microarray- or chip-based synthesis.

11. The method of any previous claim, wherein the plurality of single-stranded oligonucleotides are synthesized on beads in micro-cavities.

12. The method of any previous claim, further comprising: subjecting the double-stranded target nucleic acid to one or more error correction and/or error filtration steps to obtain an error-corrected and/or error-free double-stranded target nucleic acid.

13. The method of any previous claim, further comprising: transforming a host cell with the double-stranded target nucleic acid.

14. The method of any previous claim, wherein the polymerase of step (c) is a polymerase with proof-reading activity, optionally wherein the polymerase is selected from the group consisting of Pfu DNA polymerase, Deep Vent^{®} DNA polymerase, Q5^{®} High-Fidelity DNA Polymerase, Phusion^{®}, Phusion^{®} HotStartFlex, PrimeSTAR^{®} HS, PrimeSTAR^{®} GXL, PrimeSTAR^{®} Max, AccuPrime^{™} Pfx, Platinum^{™} DNA Polymerase High Fidelity, Phusion^{®} Flash II DNA Polymerase, Phusion^{®} Hot Start II High-Fidelity DNA Polymerase, Accura^{®} High-Fidelity polymerase or iProof^{™} High-Fidelity polymerase.

15. The method of any previous claim, wherein the linear double-stranded polynucleotide has a size of between about 0.5 kb and about 5 kb or between about 10 kb and about 30 kb.

16. The method of any previous claim, wherein the length of the target nucleic acid assembled from the population of single-stranded oligonucleotides is at least about 1 kb or at least about 2 kb in length or is between about 0.2 kb and about 1 kb in length.

17. The method of any previous claim, wherein the linear double-stranded polynucleotide is derived from cleavage of a circular double-stranded polynucleotide with one or more restriction enzymes or from amplification of a circular double-stranded polynucleotide.

18. The method of any previous claim, wherein the linear double-stranded polynucleotide is a high copy number vector and/or comprises a resistance and/or selectable marker.

19. Use of a kit for assembling a double-stranded target nucleic acid intended to have a predefined sequence according to a method of any one of claims 1 - 18, wherein the kit comprises:
a) a population of single-stranded oligonucleotides having less than 200 nucleotides in length, wherein the plurality of single-stranded oligonucleotides are obtained by chemical synthesis, wherein the population of single-stranded oligonucleotides comprise:
a first set of oligonucleotides, together comprising at least a portion of a first strand of the double-stranded target nucleic acid, and
a second set of oligonucleotides together comprising at least a portion of a second strand of the double-stranded target nucleic acid,
wherein each oligonucleotide within the first set of oligonucleotides has a sequence region that is capable of hybridizing to at least one other oligonucleotide in the second set of oligonucleotides,
b) each of the oligonucleotides having an assembly region representing a portion of the target nucleic acid, wherein each assembly region has a sequence region that is capable of hybridizing to at least one other oligonucleotide in the population,
c) a linear double-stranded polynucleotide, each terminus of the linear double-stranded polynucleotide having a sequence region that is capable of hybridizing to at least one oligonucleotide of the population,
d) a polymerase, dNTPs and a buffer system, and
e) optionally, competent host cells for transformation.

## Patentansprüche

1. Verfahren zum Aufbauen einer doppelsträngigen Zielnukleinsäure, die eine vordefinierte Sequenz aufweisen soll, das Verfahren umfassend:
a) Bereitstellen einer Population einzelsträngiger Oligonukleotide, die weniger als 200 Nukleotide in Länge aufweisen, wobei die Vielzahl von einzelsträngigen Oligonukleotiden durch chemische Synthese erhalten wird,
wobei die Population einzelsträngiger Oligonukleotide einen ersten Satz von Oligonukleotiden umfasst, zusammen umfassend mindestens einen Abschnitt eines ersten Strangs der doppelsträngigen Zielnukleinsäure, und
einen zweiten Satz von Oligonukleotiden, zusammen umfassend mindestens einen Abschnitt eines zweiten Strangs der doppelsträngigen Zielnukleinsäure,
wobei jedes Oligonukleotid innerhalb des ersten Satzes von Oligonukleotiden einen Sequenzbereich aufweist, der in der Lage ist, an mindestens einem anderen Oligonukleotid in dem zweiten Satz von Oligonukleotiden zu hybridisieren,
wobei jedes der Oligonukleotide einen Aufbaubereich aufweist, der einen Abschnitt der Zielnukleinsäure darstellt, wobei jeder Aufbaubereich einen Sequenzbereich aufweist, der in der Lage ist, an mindestens einem anderen Oligonukleotid in der Population zu hybridisieren,
b) Bereitstellen eines linearen doppelsträngigen Polynukleotids, wobei jeder Terminus des linearen doppelsträngigen Polynukleotids einen Sequenzbereich aufweist, der in der Lage ist, an mindestens einem Oligonukleotid der Population zu hybridisieren,
c) Kombinieren der Population einzelsträngiger Oligonukleotide mit dem linearen doppelsträngigen Polynukleotid und einer Polymerase und dNTPs in einem Puffer, um eine Reaktionsmischung zu erzeugen, und
d) Unterziehen der Reaktionsmischung Zyklusbedingungen, die ermöglichen
(i) mindestens eine teilweise Denaturierung des linearen doppelsträngigen Polynukleotids, um einzelsträngige Termini zu erhalten,
(ii) Hybridisierung der Population einzelsträngiger Oligonukleotide aneinander und an die einzelsträngigen Termini des Polynukleotids in einer vorbestimmten Reihenfolge,
(iii) Polymerase-vermittelte Verlängerung der freien 3'-Enden der hybridisierten Oligonukleotide und der Polynukleotidstränge, um ein doppelsträngiges nichtkovalent geschlossenes, zirkularisiertes Assemblierungsprodukt zu erzeugen, umfassend eine lineare Anordnung von Assemblierungsbereichen, und
(iv) Amplifikation des doppelsträngigen zirkularisierten Assemblierungsprodukts, um eine amplifizierte doppelsträngige Zielnukleinsäure zu erzeugen, die eine vordefinierte Sequenz aufweisen soll.

2. Verfahren nach Anspruch 1, wobei der erste und der zweite Satz von Oligonukleotiden so gestaltet sind, dass sie mit Strangbrüchen oder einer oder mehreren Nukleotidlücken zwischen angrenzenden Oligonukleotiden innerhalb desselben Strangs hybridisieren.

3. Verfahren nach Anspruch 1, wobei der erste Satz von Oligonukleotiden so gestaltet ist, um mit Strangbrüchen zwischen angrenzenden Oligonukleotiden zu hybridisieren, während der zweite Satz von Oligonukleotiden so gestaltet ist, um mit einem oder mehreren Nukleotidlücken zwischen angrenzenden Oligonukleotiden zu hybridisieren, wobei optional die Oligonukleotide des ersten Satzes zwischen etwa 36 bis etwa 120 Nukleotide in Länge sind, und wobei die Oligonukleotide des zweiten Satzes zwischen etwa 36 und etwa 44 Nukleotide in Länge sind.

4. Verfahren nach einem der vorstehenden Ansprüche, wobei der Sequenzbereich des Oligonukleotidassemblierungsbereichs, der in der Lage ist, an mindestens einem anderen Oligonukleotid in der Population zu hybridisieren, mindestens 8 Nukleotide, vorzugsweise mindestens 12 Nukleotide in Länge ist.

5. Verfahren nach einem der vorstehenden Ansprüche, wobei der Sequenzbereich des Oligonukleotidassemblierungsbereichs, der in der Lage ist, an einen Terminus des linearen Polynukleotids zu hybridisieren, zwischen etwa 20 und etwa 45 Nukleotiden in Länge ist.

6. Verfahren nach einem der vorstehenden Ansprüche, wobei jedes einzelsträngige Oligonukleotid ferner eine Markierung und einen Spacer umfasst,
wobei sich die Markierung an dem 5'-Ende des Assemblierungsbereichs jedes Oligonukleotids befindet und sich der Spacer zwischen der Markierung und dem Assemblierungsbereich befindet, und
wobei der Spacer entweder spaltbar oder in der Lage ist, enzymatische vermittelte Nukleinsäurereplikationsreaktionen zu beenden.

7. Verfahren nach Anspruch 6, wobei jedes Oligonukleotid in der Population eine Markierung mit einer identischen Nukleotidsequenz umfasst, optional wobei die Markierung zwischen etwa 12 und etwa 24 Nukleotide in Länge ist.

8. Verfahren nach Anspruch 7, wobei der Spacer eine Hexaethylenglycolgruppe umfasst.

9. Verfahren nach einem der vorstehenden Ansprüche, wobei die chemische Synthese einen photochemischen oder elektrochemischen Entschützungsschritt umfasst.

10. Verfahren nach einem der vorstehenden Ansprüche, wobei die Vielzahl von einzelsträngigen Oligonukleotiden durch Mikroarray- oder Chip-basierte Synthese erhalten wird.

11. Verfahren nach einem der vorstehenden Ansprüche, wobei die Vielzahl von einzelsträngigen Oligonukleotiden auf Kügelchen in Mikrohohlräumen synthetisiert wird.

12. Verfahren nach einem der vorstehenden Ansprüche, ferner umfassend:
Aussetzen der doppelsträngigen Zielnukleinsäure einem oder mehrerer Fehlerkorrektur- und/oder Fehlerfiltrationsschritte, um eine fehlerkorrigierte und/oder fehlerfreie doppelsträngige Zielnukleinsäure zu erhalten.

13. Verfahren nach einem der vorstehenden Ansprüche, ferner umfassend:
Transformieren einer Wirtszelle mit der doppelsträngigen Zielnukleinsäure.

14. Verfahren nach einem der vorstehenden Ansprüche, wobei die Polymerase von Schritt (c) eine Polymerase mit einer Proof-Reading-Aktivität ist, optional wobei die Polymerase aus der Gruppe ausgewählt ist, bestehend aus Pfu DNA Polymerase, Deep Vent^{®} DNA Polymerase, Q5^{®} High-Fidelity DNA Polymerase, Phusion^{®}, Phusion^{®} HotStartFlex, PrimeSTAR@ HS, PrimeSTAR@ GXL, PrimeSTAR@ Max, AccuPrime^{™} Pfx, Platinum^{™} DNA Polymerase High Fidelity, Phusion^{®} Flash II DNA Polymerase, Phusion^{®} Hot Start II High-Fidelity DNA Polymerase, Accura^{®} High-Fidelity Polymerase oder iProof^{™} High-Fidelity Polymerase.

15. Verfahren nach einem der vorstehenden Ansprüche, wobei das lineare doppelsträngige Polynukleotid eine Größe zwischen etwa 0,5 kb und etwa 5 kb oder zwischen etwa 10 kb und etwa 30 kb aufweist.

16. Verfahren nach einem der vorstehenden Ansprüche, wobei die Länge der Zielnukleinsäure, die aus der Population einzelsträngiger Oligonukleotide aufgebaut ist, mindestens etwa 1 kb oder mindestens etwa 2 kb in Länge ist oder zwischen etwa 0,2 kb und etwa 1 kb in Länge ist.

17. Verfahren nach einem der vorstehenden Ansprüche, wobei das lineare doppelsträngige Polynukleotid aus einer Spaltung eines kreisförmigen doppelsträngigen Polynukleotids mit einem oder mehreren Restriktionsenzymen oder aus einer Amplifikation eines kreisförmigen doppelsträngigen Polynukleotids abgeleitet wird.

18. Verfahren nach einem der vorstehenden Ansprüche, wobei das lineare doppelsträngige Polynukleotid ein Vektor hoher Kopienzahl ist und/oder einen Widerstand und/oder einen selektierbaren Marker umfasst.

19. Verwendung eines Kits zum Aufbauen einer doppelsträngigen Zielnukleinsäure, die eine vordefinierte Sequenz gemäß einem Verfahren nach einem der Ansprüche 1 bis 18 aufweisen soll,
wobei das Kit umfasst:
a) eine Population einzelsträngiger Oligonukleotide, die weniger als 200 Nukleotide in Länge aufweist, wobei die Vielzahl von einzelsträngigen
Oligonukleotiden durch chemische Synthese erhalten werden, wobei die Population einzelsträngiger Oligonukleotide umfasst:
einen ersten Satz von Oligonukleotiden, zusammen umfassend mindestens einen Abschnitt eines ersten Strangs der doppelsträngigen Zielnukleinsäure, und
einen zweiten Satz von Oligonukleotiden, zusammen umfassend mindestens einen Abschnitt eines zweiten Strangs der doppelsträngigen Zielnukleinsäure, wobei jedes Oligonukleotid innerhalb des ersten Satzes von Oligonukleotiden einen Sequenzbereich aufweist, der in der Lage ist, an mindestens einem anderen Oligonukleotid in dem zweiten Satz von Oligonukleotiden zu hybridisieren,
b) wobei jedes der Oligonukleotide einen Aufbaubereich aufweist, der einen Abschnitt der Zielnukleinsäure darstellt, wobei jeder Aufbaubereich einen Sequenzbereich aufweist, der in der Lage ist, an mindestens einem anderen Oligonukleotid in der Population zu hybridisieren,
c) ein lineares doppelsträngiges Polynukleotid, wobei jeder Terminus des linearen doppelsträngigen Polynukleotids einen Sequenzbereich aufweist, der in der Lage ist, an mindestens einem Oligonukleotid der Population zu hybridisieren,
d) eine Polymerase, dNTPs und ein Puffersystem, und
e) optional kompetente Wirtszellen für eine Transformation.

## Revendications

1. Procédé d'assemblage d'un acide nucléique cible double brin destiné à avoir une séquence prédéfinie, le procédé comprenant :
a) la fourniture d'une population d'oligonucléotides simple brin ayant une longueur inférieure à 200 nucléotides, la pluralité d'oligonucléotides simple brin étant obtenue par synthèse chimique,
la population d'oligonucléotides simple brin comprenant un premier ensemble d'oligonucléotides, comprenant ensemble au moins une partie d'un premier brin de l'acide nucléique cible double brin, et
un second ensemble d'oligonucléotides comprenant ensemble au moins une partie d'un second brin de l'acide nucléique cible double brin,
chaque oligonucléotide au sein du premier ensemble d'oligonucléotides ayant une région de séquence qui est capable de s'hybrider à au moins un autre oligonucléotide dans le second ensemble d'oligonucléotides
chacun des oligonucléotides ayant une région d'assemblage représentant une partie de l'acide nucléique cible, chaque région d'assemblage ayant une région de séquence qui est capable de s'hybrider à au moins un autre oligonucléotide dans la population,
b) la fourniture d'un polynucléotide linéaire double brin, chaque terminaison du polynucléotide linéaire double brin ayant une région de séquence qui est capable de s'hybrider à au moins un oligonucléotide de la population,
c) la combinaison de la population d'oligonucléotides simple brin avec le polynucléotide linéaire double brin et une polymérase et des dNTP dans un tampon pour générer un mélange réactionnel, et
d) la soumission du mélange réactionnel à des conditions de cyclage qui permettent
(i) la dénaturation au moins partielle du polynucléotide linéaire à double brin pour obtenir des extrémités monocaténaires,
(ii) l'hybridation de la population d'oligonucléotides simple brin entre eux et aux extrémités monocaténaires du polynucléotide dans un ordre prédéterminé,
(iii) une extension médiée par polymérase des extrémités 3' libres des oligonucléotides hybridés et des brins polynucléotidiques pour générer un produit d'assemblage circularisé non fermé de manière non covalente à double brin comprenant une disposition linéaire de régions d'assemblage, et
(iv) l'amplification du produit d'assemblage circularisé double brin pour générer un acide nucléique cible double brin amplifié destiné à avoir une séquence prédéfinie.

2. Procédé selon la revendication 1, dans lequel les premier et second ensembles d'oligonucléotides sont conçus pour s'hybrider avec des coupures ou un ou plusieurs intervalles de nucléotide entre des oligonucléotides adjacents au sein du même brin.

3. Procédé selon l'une quelconque des revendications 1 ou 2, dans lequel le premier ensemble d'oligonucléotides est conçu pour s'hybrider avec des coupures entre des oligonucléotides adjacents, tandis que le second ensemble d'oligonucléotides est conçu pour s'hybrider avec un ou plusieurs intervalles de nucléotide entre des oligonucléotides adjacents, éventuellement les oligonucléotides du premier ensemble sont compris entre environ 36 et environ 120 nucléotides de longueur, et les oligonucléotides du second ensemble étant de longueur d'environ 36 à environ 44 nucléotides.

4. Procédé selon l'une quelconque revendication précédente, dans lequel la région de séquence de la région d'assemblage d'oligonucléotide qui est capable de s'hybrider à au moins un autre oligonucléotide dans la population est d'au moins 8 nucléotides, de préférence d'au moins 12 nucléotides de longueur.

5. Procédé selon l'une quelconque des revendications précédentes, dans lequel la région de séquence de la région d'assemblage d'oligonucléotide qui est capable de s'hybrider à une extrémité du polynucléotide linéaire est comprise entre environ 20 et environ 45 nucléotides de longueur.

6. Procédé selon l'une quelconque revendication précédente, dans lequel chaque oligonucléotide simple brin comprend en outre un marqueur et un espaceur,
l'étiquette étant située à l'extrémité 5' de la région d'assemblage de chaque oligonucléotide et l'entretoise étant située entre l'étiquette et la région d'assemblage, et
l'espaceur étant soit clivable soit capable de terminer des réactions de réplication d'acide nucléique à médiation enzymatique.

7. Procédé selon la revendication 6, dans lequel chaque oligonucléotide dans la population comprend un marqueur avec une séquence nucléotidique identique, éventuellement le marqueur étant compris entre environ 12 et environ 24 nucléotides de longueur.

8. Procédé selon la revendication 7, dans lequel l'espaceur comprend un groupe hexaéthylène glycol.

9. Procédé selon l'une quelconque revendication précédente, dans lequel la synthèse chimique comprend une étape de déprotection photo-chimique ou électrochimique.

10. Procédé selon l'une quelconque revendication précédente, dans lequel la pluralité d'oligonucléotides simple brin sont obtenus par synthèse à micro-matrice ou à puce.

11. Procédé selon l'une quelconque revendication précédente, dans lequel la pluralité d'oligonucléotides simple brin sont synthétisés sur des perles dans des micro-cavités.

12. Procédé selon l'une quelconque revendication précédente, comprenant en outre : la soumission de l'acide nucléique cible double brin à une ou plusieurs étapes de correction d'erreur et/ou de filtration d'erreur pour obtenir un acide nucléique cible à double brin corrigé par erreur et/ou sans erreur.

13. Procédé selon l'une quelconque revendication précédente, comprenant en outre : la transformation d'une cellule hôte avec l'acide nucléique cible double brin.

14. Procédé selon l'une quelconque revendication précédente, dans lequel la polymérase de l'étape (c) est une polymérase avec une activité de lecture de preuve, éventuellement la polymérase étant choisie dans le groupe constitué de la polymérase d'ADN Pfu, de la polymérase d'ADN Deep Vent^{®},de la polymérase d'ADN à fidélité élevée Q5^{®}, de Phusion^{®}, de Phusion^{®} HotStartFlex, de PrimeSTAR@ HS, de PrimeSTAR@ GXL, de PrimeSTAR@ Max, de l'AccuPrime^{™} Pfx, de la polymérase d'ADN à fidélité élevée Platinum^{™}, de la polymérase d'ADN Phusion^{®} Flash II, de la polymérase d'ADN à fidélité élevée Phusion^{®} Hot Start II, de la polymérase à fidélité élevée Accura^{®} ou de la polymérase à fidélité élevée iProof^{™}.

15. Procédé selon l'une quelconque revendication précédente, dans lequel le polynucléotide linéaire à double brin a une taille comprise entre environ 0,5 kb et environ 5 kb ou entre environ 10 kb et environ 30 kb.

16. Procédé selon l'une quelconque revendication précédente, dans lequel la longueur de l'acide nucléique cible assemblé à partir de la population d'oligonucléotides simple brin est d'au moins environ 1 kb ou d'au moins environ 2 kb de longueur ou est comprise entre environ 0,2 kb et environ 1 kb de longueur.

17. Procédé selon l'une quelconque revendication précédente, dans lequel le polynucléotide linéaire double brin est dérivé d'un clivage d'un polynucléotide circulaire double brin avec une ou plusieurs enzymes de restriction ou de l'amplification d'un polynucléotide circulaire double brin.

18. Procédé selon l'une quelconque revendication précédente, dans lequel le polynucléotide linéaire à double brin est un vecteur à nombre de copies élevé et/ou comprend un marqueur de résistance et/ou de sélection.

19. Utilisation d'un kit pour assembler un acide nucléique cible double brin destiné à avoir une séquence prédéfinie selon un procédé selon l'une quelconque des revendications 1 à 18,
dans lequel le kit comprend :
a) une population d'oligonucléotides simple brin ayant une longueur inférieure à 200 nucléotides, la pluralité d'oligonucléotides simple brin
étant obtenus par synthèse chimique, la population d'oligonucléotides simple brin comprenant :
un premier ensemble d'oligonucléotides, comprenant ensemble au moins une partie d'un premier brin de l'acide nucléique cible double brin, et
un second ensemble d'oligonucléotides comprenant ensemble au moins une partie d'un second brin de l'acide nucléique cible double brin, chaque oligonucléotide au sein du premier ensemble d'oligonucléotides ayant une région de séquence qui est capable de s'hybrider à au moins un autre oligonucléotide dans le second ensemble d'oligonucléotides,
b) chacun des oligonucléotides ayant une région d'assemblage représentant une partie de l'acide nucléique cible, chaque région d'assemblage ayant une région de séquence qui est capable de s'hybrider à au moins un autre oligonucléotide dans la population,
c) un polynucléotide linéaire double brin, chaque terminaison du polynucléotide linéaire double brin ayant une région de séquence qui est capable de s'hybrider à au moins un oligonucléotide de la population,
d) une polymérase, des dNTP et un système de tampon, et
e) éventuellement, des cellules hôtes compétentes pour la transformation.
